(19) Europäisches Patentamt
European Patent Office
Office européen des brevets

(11) EP 2 507 308 B1

(12) # EUROPEAN PATENT SPECIFICATION

(45) Date of publication and mention of the grant of the patent:
**12.02.2014 Bulletin 2014/07**

(21) Application number: **10787238.4**

(22) Date of filing: **01.12.2010**

(51) Int Cl.:
***C08K 5/00*** $^{(2006.01)}$ ***C08L 33/14*** $^{(2006.01)}$

(86) International application number:
**PCT/US2010/058478**

(87) International publication number:
**WO 2011/068820 (09.06.2011 Gazette 2011/23)**

(54) **HYDROLYTICALLY STABLE MULTI-PURPOSE POLYMERS**

HYDROLYSESTABILE MEHRZWECKPOLYMERE

POLYMÈRES POLYVALENTS STABLES À L'HYDROLYSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.12.2009 US 265437 P**

(43) Date of publication of application:
**10.10.2012 Bulletin 2012/41**

(73) Proprietor: **Lubrizol Advanced Materials, Inc.**
**Cleveland, OH 44141-3247 (US)**

(72) Inventors:
- **TAMARESELVY, Krishnan**
**Brecksville**
**OH 44141 (US)**
- **FILLA, Deborah S.**
**Twinsburg**
**OH 44087 (US)**
- **PUROHIT, Pinky G.**
**Beachwood**
**OH 44122 (US)**
- **LI, WIng K.**
**East Brunswick**
**NJ 08816 (US)**

(74) Representative: **von Kreisler Selting Werner**
**Deichmannhaus am Dom**
**Bahnhofsvorplatz 1**
**50667 Köln (DE)**

(56) References cited:
**WO-A1-00/39176**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 507 308 B1

## Description

### FIELD OF THE INVENTION

The present invention relates to a composition comprising multi-purpose polymers that are the polymerization product of a monomer mixture comprising: at least one amino-substituted meth(acrylate) (ASMA) monomer or salt thereof; and at least one nonionic vinyl (NIV) monomer, wherein the monomer mixture further comprises one or more of at least one vinyl associative (VA) monomer; at least one vinyl surfactant (VS) monomer; and/or at least one polymerizable silicone macromer (PSM) and wherein the monomer mixture further optionally comprises one or more of at least one crosslinking (XL) monomer; at least one chain transfer agent (CTA); and/or at least one polymeric stabilizer. The multi-purpose polymers can also be prepared from monomer mixtures containing chain transfer agents or other functional components commonly utilized in polymerization processes. In one embodiment, the multi-purpose polymers are hydrolytically stable over long periods of time (e.g., periods of time in excess of six months or more), provide surprisingly beneficial rheological properties in acidic aqueous compositions, and are compatible with cationic materials. The multi-purpose polymers are useful in a variety of products including, but not limited to, personal care products, health care products, household care products, institutional and industrial care products, and industrial applications.

### BACKGROUND OF THE INVENTION

Multi-purpose copolymers derived from, for example, the dimethylaminoethyl methacrylate (DMAEMA) monomer are known in the art. Such copolymers when produced from the afore-mentioned monomer can, in certain applications, have stability issue. As a result, such polymers have a poor shelf life and can start to "destabilize" after about six months. This in turn causes various logistic problems and undesirable performance degradation. The poor shelf life of these polymers is due to the amine backbone contained in the DMAEMA monomer which readily undergoes hydrolysis in an aqueous medium through "back-biting". The lone-pair electron on the nitrogen atom attacks the electron deficient carbonyl carbon to yield a five member ring Zwitterionic intermediate. Such a structure can then be easily hydrolyzed in the presence of water. The reaction scheme shown below summarizes the problem associated with copolymers that are derived from a DMAEMA monomer. As a result of the hydrolysis -COOH functional sites are produced on the backbone of a copolymer, as well as a dimethylaminoethanol by-product.

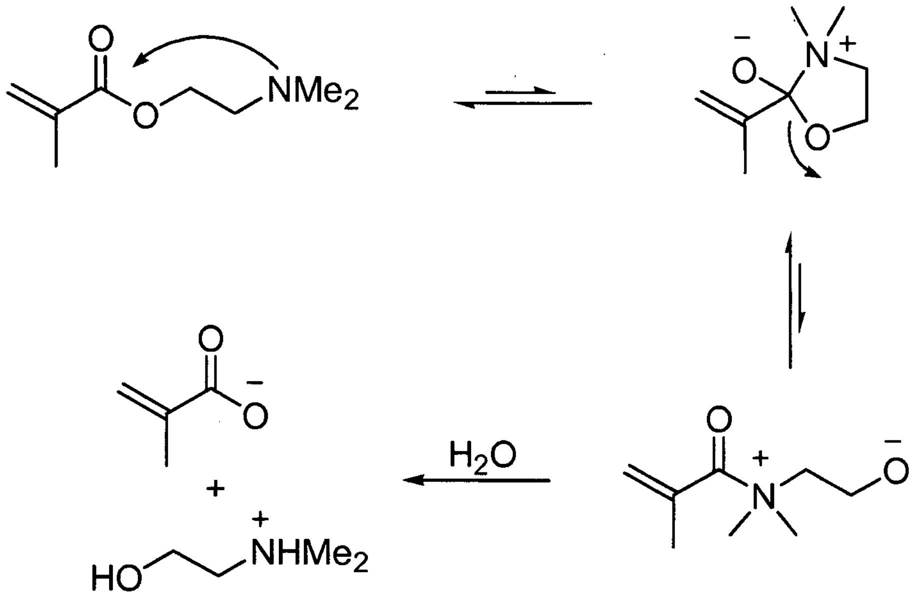


Given the above, there is a need in the art for hydrolytically stable multi-purpose copolymers that possess a suitable shelf life (e.g., in excess of six months or more).

### SUMMARY OF THE INVENTION

The present invention relates to a composition comprising multi-purpose polymers that are the polymerization product of a monomer mixture comprising: at least one amino-substituted meth(acrylate) (ASMA) monomer or salt thereof; and at least one nonionic vinyl (NIV) monomer, wherein the monomer mixture further comprises one or more

of at least one vinyl associative (VA) monomer; at least one vinyl surfactant (VS) monomer; and/or at least one polymerizable silicone macromer (PSM) and wherein the monomer mixture further optionally comprises one or more of at least one crosslinking (XL) monomer; at least one chain transfer agent (CTA); and/or at least one polymeric stabilizer. The multi-purpose polymers can also be prepared from monomer mixtures containing chain transfer agents or other functional components commonly utilized in polymerization processes. In one embodiment, the multi-purpose polymers are hydrolytically stable over long periods of time (e.g., periods of time in excess of six months or more), provide surprisingly beneficial rheological properties in acidic aqueous compositions, and are compatible with cationic materials. In addition, they provide long term suspension stability, to formulations containing insoluble materials that require suspension. The multi-purpose polymers are useful in a variety of products including, but not limited to, personal care products, health care products, household care products, institutional and industrial care products, and industrial applications.

The multi-purpose polymers and personal care compositions of the present invention may suitably comprise, consist of, or consist essentially of the components, elements, and process delineations described herein. The invention illustratively disclosed herein suitably may be practiced in the absence of any element which is not specifically disclosed herein.

Unless otherwise stated, all percentages, parts, and ratios expressed herein are based upon weight of the total compositions of the present invention, and all weights are expressed on the basis of 100 percent active ingredients.

The present invention relates to a composition comprising a hydrolytically stable polymer that is the reaction product of the polymerization of a monomer mixture comprising monomers:

(i) (a), (b) and (c);
(ii) (a), (b) and (d);
(iii) (a), (b) and (e);
(iv) (a), (b), (c) and (d);
(v) (a), (b), (c) and (e);
(vi) (a), (b), (d) and (e); or
(vii) (a), (b), (c), (d) and (e),

where (a) is at least one amino-substituted meth(acrylate) (ASMA) monomer or salt thereof; (b) is at least one nonionic vinyl (NIV) monomer; (c) is at least one vinyl associative (VA) monomer; (d) is at least one vinyl surfactant (VS) monomer; (e) is at least one polymerizable silicone macromer (PSM), and wherein any of the above mixtures further optionally contain: (f) at least one crosslinking (XL) monomer; (g) at least one chain transfer agent (CTA); (h) at least one polymeric stabilizer; or any suitable combination of two or more of components (f), (g) and/or (h).

In still another embodiment, the present invention relates to a hydrolytically stable polymer that is the reaction product of the polymerization of a monomer mixture comprising monomers:

(i) (a), (b) and (c);
(ii) (a), (b) and (d);
(iii) (a), (b) and (e);
(iv) (a), (b), (c) and (d);
(v) (a), (b), (c) and (e);
(vi) (a), (b), (d) and (e); or
(vii) (a), (b), (c), (d) and (e),

where (a) is at least one amino-substituted meth(acrylate) (ASMA) monomer or salt thereof; (b) is at least one nonionic vinyl (NIV) monomer; (c) is at least one vinyl associative (VA) monomer; (d) is at least one vinyl surfactant (VS) monomer; (e) is at least one polymerizable silicone macromer (PSM), and wherein any of the above mixtures further optionally contain: (f) at least one crosslinking (XL) monomer; (g) at least one chain transfer agent (CTA); (h) at least one polymeric stabilizer; or any suitable combination of two or more of components (f), (g) and/or (h), wherein the monomer mixture comprises, on a total monomer mixture weight basis about 1 weight percent to about 90 weight percent of component (a); about 20 weight percent to about 80 weight percent of component (b); about 0.01 weight percent to about 25 weight percent of component (c); about 0.01 weight percent to about 25 weight percent of component (d); about 0.01 weight percent to about 10 weight percent of component (e); up to about 5 weight percent of component (f); up to about 10 weight percent of component (g); and up to about 2 weight percent of component (h).

In still yet another embodiment, the present invention relates to a hydrolytically stable polymer that is the reaction product of the polymerization of a monomer mixture comprising monomers:

(i) (a), (b) and (c);

(ii) (a), (b) and (d);
(iii) (a), (b) and (e);
(iv) (a), (b), (c) and (d);
(v) (a), (b), (c) and (e);
(vi) (a), (b), (d) and (e); or
(vii) (a), (b), (c), (d) and (e),

where (a) is at least one amino-substituted meth(acrylate) (ASMA) monomer or salt thereof; (b) is at least one nonionic vinyl (NIV) monomer; (c) is at least one vinyl associative (VA) monomer; (d) is at least one vinyl surfactant (VS) monomer; (e) is at least one polymerizable silicone macromer (PSM), and wherein any of the above mixtures further optionally contain: (f) at least one crosslinking (XL) monomer; (g) at least one chain transfer agent (CTA); (h) at least one polymeric stabilizer; or any suitable combination of two or more of components (f), (g) and/or (h), wherein the monomer mixture comprises, on a total monomer mixture weight basis about 10 weight percent to about 80 weight percent of component (a); about 20 weight percent to about 70 weight percent of component (b); about 0.1 weight percent to about 15 weight percent of component (c); about 0.1 weight percent to about 10 weight percent of component (d); about 0.1 weight percent to about 7.5 weight percent of component (e); about 0.01 up to about 5 weight percent of component (f); about 0.1 to about 10 weight percent of component (g); and 0.001 to about 2 weight percent of component (h).

**BRIEF DESCRIPTION OF THE DRAWINGS**

Figure 1 is a plot of acid number versus time for various monomers when subjected to hydrolysis conditions;
Figure 2 is a plot of percent hydrolysis versus time for DMAEMA and DMADMPMA as determined by $^1H$ NMR at 500 Hz;
Figure 3 is a is a plot of acid number versus number of weeks the latex is stored 45°C for polymer Example 1 (DMAPMA) and polymer Example 2 (DMAPMA/ DMADMPMA);
Figure 4 is a plot of 2 percent TS viscosity versus number of weeks the latex is stored at 45°C for Example 1 (DMAPMA) and polymer Example 2 (DMAPMA/ DMADMPMA);
Figure 5 is a plot of acid number versus number of weeks the latex is stored at 50°C for a DMAEMA based commercial polymer (Control 3, INCI Name: Polyacrylate-1 Crosspolymer), polymer Example 4 (DMADMPMA), and polymer Example 7 (DMADMPMA);
Figure 6 is a plot of 2 percent TS gel viscosity versus number of weeks the latex is stored at 50°C for a DMAEMA based commercial polymer (Control 3, INCI Name: Polyacrylate-1 Crosspolymer), polymer Example 4 (DMADMPMA), and polymer Example 7 (DMADMPMA);
Figure 7 is a plot of acid number versus number of months the latex is stored at room temperature for a DMAEMA based commercial polymer (Control 3, INCI Name: Polyacrylate-1 Crosspolymer), polymer Example 1, polymer Example 2, polymer Example 4, polymer Example 6, polymer Example 7, and polymer Example 9; and
Figure 8 is a plot of 2 percent gel viscosity versus number of months the latex is stored at room temperature a DMAEMA based commercial polymer (Control 3, INCI Name: Polyacrylate-1 Crosspolymer), polymer Example 4, polymer Example 6, polymer Example 7, and polymer Example 9.

**DETAILED DESCRIPTION OF THE INVENTION**

The present invention relates to a composition comprising a multi-purpose polymer that is the polymerization product of a monomer mixture comprising: at least one amino-substituted meth(acrylate) (ASMA) monomer or salt thereof; and at least one nonionic vinyl (NIV) monomer, wherein the monomer mixture further comprises one or more of at least one vinyl associative (VA) monomer; at least one vinyl surfactant (VS) monomer; and/or at least one polymerizable silicone macromer (PSM) and wherein the monomer mixture further optionally comprises one or more of at least one crosslinking (XL) monomer; at least one chain transfer agent (CTA); and/or at least one polymeric stabilizer. The multi-purpose polymers can also be prepared from monomer mixtures containing chain transfer agents or other functional components commonly utilized in polymerization processes. In one embodiment, the multi-purpose polymers are hydrolytically stable over long periods of time (e.g., periods of time in excess of six months or more), provide surprisingly beneficial rheological properties in acidic aqueous compositions, and are compatible with cationic materials. The multi-purpose polymers are useful in a variety of products including, but not limited to, personal care products, health care products, household care products, institutional and industrial care products, and industrial applications.

The polymers are generally basic, aqueous acid-swellable, or aqueous acid-soluble, polymers, and salts thereof, which contain at least one basic amino substituent that is cationic at low pH and at least one hydrophobically modified

polyoxyalkylene substituent derived from a vinyl associative (VA) monomer, and optionally at least one polyoxyalkylene substituent derived from a vinyl surfactant (VS) monomer. Furthermore, the polymer can also optionally contain substituent groups derived from other monomer units, such as crosslinking monomer units, hydroxy-substituted nonionic vinyl monomer units, chain transfer agent units, polymeric stabilizers, and the like. The polymers generally exhibit associative properties in aqueous solution. For convenience, the polymers are generally referred to herein as "multi-purpose polymers." The term "low pH formulation" refers to formulations having an acidic pH in the range of about 0.5 to not more than about 7, or even to not more than about 6.5.

As used herein, the term "(meth)acrylic" acid is meant to include both acrylic acid and methacrylic acid. Similarly, the term "(meth)acrylate" as used herein is meant to include acrylates and methacrylates. The term (meth)acrylamide" is used to include both acrylamide and methacrylamide.

The term "aqueous" as applied to formulations or media means that water is present in an amount sufficient to at least swell or dissolve the multi-purpose polymer in the composition into which it is included.

It has been surprisingly discovered that the multi-purpose polymers provide improved shelf life of about six months or more. Additionally, the multi-purpose polymers provide desirable rheological properties to low pH aqueous personal care, health care, household care, and industrial and institutional care products. The multi-purpose polymers are cationic compatible making them particularly useful as thickeners in products containing quaternary ammonium salts or amines. Surprisingly, the multi-purpose polymers are useful in compositions containing one or more surfactants (e.g., anionic, cationic, amphoteric, non-ionic, and/or combinations of any two or more thereof), and also provide hair setting efficacy. The multi-purpose polymers are useful thickeners in products containing active acid components and are useful thickeners and emulsifiers for emulsions (creams, lotions). In addition to thickening, the multi-purpose polymers are useful film formers, spreading aids and deposition aids for products containing surfactants, colorants, hair and skin conditioners, silicones, monoquaternium compounds, polyquaternium compounds, anti-dandruff agents, anti-aging, anti-wrinkle, anti-pigment anti-cellulite, anti-acne, vitamins, analgesics, anti-inflammatory compounds, self-tanning agents, hair growth promoting agents, UV protecting agents, skin lighteners, vegetable, plant and botanical extracts, antiperspirants, antioxidants, deodorants, hair fixative polymers, emollient oils, and combinations thereof.

The term "personal care products" as used herein includes, without being limited thereto, cosmetics, toiletries, cosmeceuticals and beauty aids, personal hygiene and cleansing products applied to the skin, hair, scalp, and nails of humans and animals. The term "health care products" as used herein includes, without being limited thereto, pharmaceuticals, pharmacosmetics, oral care products (mouth, teeth), eye care products, ear care products and over-the-counter products and appliances, such as patches, plasters, dressings and the like, and medical devices externally applied to or into the body of humans and animals for ameliorating a health-related or medical condition, for generally maintaining hygiene or well-being, and the like. The term "body" includes the keratinous (hair, nails) and non-keratinous skin areas of the entire body (face, trunk, limbs, hands and feet), the tissues of body openings and eyes, and the term "skin" includes the scalp and mucous membranes. The term "home care products" as used herein includes, without being limited thereto, products employed in a domestic household for surface cleaning or biocidal cleaning products for maintaining sanitary conditions, such as in the kitchen and bathroom, and laundry products for fabric care and cleaning, and the like. The term "institutional and industrial care" and "I&I," as used herein includes, without being limited thereto, products employed for cleaning or maintaining sanitary conditions in industrial and institutional environments, including hospital and health care facilities, and the like.

The multi-purpose polymers are hydrolytically stable multi-purpose polymers, which are, in one embodiment, prepared by polymerizing a mixture of monomers selected from one of the following compositions:

(i) (a), (b) and (c);
(ii) (a), (b) and (d);
(iii) (a), (b) and (e);
(iv) (a), (b), (c) and (d);
(v) (a), (b), (c) and (e);
(vi) (a), (b), (d) and (e); or
(vii) (a), (b), (c), (d) and (e),

where (a) is at least one amino-substituted meth(acrylate) (ASMA) monomer or salt thereof; (b) is at least one nonionic vinyl (NIV) monomer; (c) is at least one vinyl associative (VA) monomer; (d) is at least one vinyl surfactant (VS) monomer; (e) is at least one polymerizable silicone macromer (PSM), and wherein any of the above mixtures further optionally contain: (f) at least one crosslinking (XL) monomer; (g) at least one chain transfer agent (CTA); (h) at least one polymeric stabilizer; or any suitable combination of two or more of components (f), (g) and/or (h). The multi-purpose polymers can also be prepared from monomer mixtures containing chain transfer agents or other functional components commonly utilized in polymerization processes.

In one embodiment, the inventive hydrolytically stable multi-purpose polymer is the polymerization product of

a monomer mixture comprising, on a total monomer mixture weight basis: (a) about 1 weight percent to about 90 weight percent of at least one ASMA monomer or a salt thereof; (b) about 20 weight percent to about 80 weight percent of at least one NIV monomer; (c) about 0.01 weight percent to about 25 weight percent of at least one V monomer; (d) up to about 25 weight percent of at least one VS monomer; (e) about 0.01 weight percent to about 10 weight percent of at least one polymerizable silicone macromer (PSM); (f) up to about 5 weight percent of a XL monomer; (g) up to about 10 weight percent of a CTA; and (h) up to about 2 weight percent of a polymeric stabilizer.

In another embodiment, the multi-purpose polymer is the polymerization product of a monomer mixture comprising, on a total monomer mixture weight basis: (a) about 15 weight percent to about 80 weight percent of at least one ASMA monomer or a salt thereof; (b) about 20 weight percent to about 70 weight percent of at least one NIV monomer; (c) about 0.1 weight percent to about 15 weight percent of at least one V monomer; (d) about 0.1 weight percent to about 10 weight percent of at least one VS monomer; (e) about 0.1 weight percent to about 7.5 weight percent of at least one polymerizable silicone macromer (PSM); (f) about 0.001 weight percent to about 5 weight percent of a XL monomer; and (g) about 0.1 weight percent to about 5 weight percent of a CTA. Here, as well as elsewhere in the specification and claims, individual numerical values, or limits, can be combined to form additional non-disclosed and/or non-stated ranges.

In still another embodiment, the multi-purpose polymers are polymers that are the product of polymerization of a monomer mixture comprising, on a total monomer mixture weight basis: (a) about 20 weight percent to about 70 weight percent of at least one amino-substituted meth(acrylate) (ASMA) monomer selected from one or more monomers represented by Formulas (I), I(a), and/or (II) below:

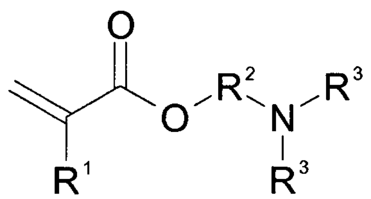

(I)

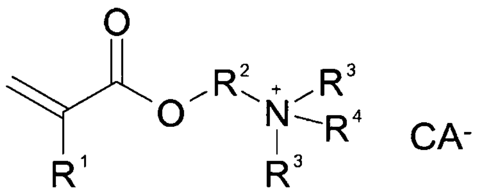

(II)

where $R^1$, $R^2$, $R^3$ and $R^4$ are as defined below, and $CA^-$ is a counter-anion suitable to balance the charge on the quaternary ammonium moiety; (b) about 50 weight percent to about 65 weight percent of at least one nonionic vinyl (NIV) monomer selected from $C_1$ to $C_{30}$ alkyl ester of acrylic acid, a $C_1$ to $C_{30}$ alkyl ester of methacrylic acid, or any suitable mixture of two or more thereof; (c) about 0.1 weight percent to about 10 weight percent of at least one vinyl associative (VA) monomer selected from cetyl polyethoxylated methacrylate (CEM), cetearyl polyethoxylated methacrylate (CSEM), stearyl polyethoxylated (meth)acrylate, arachidyl polyethoxylated (meth)arcylate, behenyl polyethoxylated methacrylate (BEM), lauryl polyethoxylated methacrylate (LEM), cerotyl polyethoxylated (meth)acrylate, monthanyl polyethoxylated (meth)acrylate, melissyl polyethoxylated (meth)acrylate, lacceryl polyethoxylated (meth)acrylate, tristyryl phenolpolyethoxylated methacrylate (TEM), hydrogenated castor oil polyethoxylated methacrylate (HCOEM), canola polyethoxylated (meth)acrylate, and cholesterol polyethoxylated methacrylate (CHEM); (d) about 0.1 weight percent to about 10 weight percent of at least one vinyl surfactant (VS) monomer represented by Formula (VI):

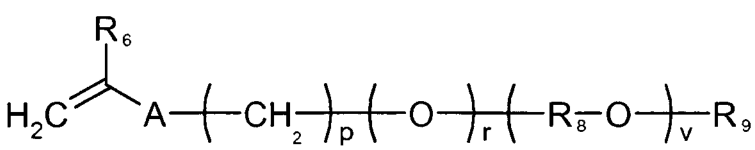

(VI)

where each $R_6$ is independently hydrogen or methyl, -C(O)OH, or $-C(O)OR_7$; $R_7$ is $C_1$ to $C_{30}$ alkyl; A is $-CH_2C(O)O-$, -C(O)O-, -O-, or $-CH_2O-$; p is an integer in the range of 0 to about 30, and r is 0 or 1, with the proviso that when p is 0, r is 0, and when p is in the range of 1 to about 30, r is 1; $(R_8-O)_v$ is a polyoxyalkylene, which is a homopolymer, a random copolymer or a block copolymer of $C_2$ to $C_4$ oxyalkylene units, wherein each $R_8$ is independently $C_2H_4$, $C_3H_6$, $C_4H_8$, or

a mixture thereof, and v is an integer in the range of about 1 to about 250, or from about 5 to about 100, or from about 10 to about 80, or even from about 15 to about 60; and $R_9$ is hydrogen or $C_1$ to $C_4$ alkyl; (e) about 1 weight percent to about 7.5 weight percent of at least one polymerizable silicone macromer (PSM); (f) up to about 5 weight percent of a crosslinking (XL) monomer; (g) up to about 10 weight percent of a chain transfer agent (CTA); and (h) up to about 2 weight percent of a polymeric stabilizer. Here, as well as elsewhere in the specification and claims, individual numerical values, or limits, from various separate embodiments can be combined to form additional non-disclosed and/or non-stated ranges.

As used herein the term "alkyl" means a substituted or unsubstituted aliphatic hydrocarbon moiety including linear, branched and carbocyclic alkyl moieties. The term "carbocyclic alkyl" means an alkyl group comprising one or more carbocyclic rings of from 3 to about 12 carbon atoms in size and optionally including alkyl substituents on the carbocyclic ring. The term "aryl" includes substituted and unsubstituted phenyl and naphthyl moieties. Modifiers of the form "$C_x$-$C_y$" designate that the alkyl or carbocyclic alkyl groups have molecular formulas containing a total of x to y carbon atoms, where x and y are specified integers. As used herein and in the appended claims, the term "complex ester" means a di-, tri-, or poly-ester of a polyol such as a sugar, having at least one hydroxyl group capable of being alkylated with a $C_2$ to $C_7$ alkylene oxide. The term "complex ester" includes, in one instance the complex hydrophobes described in United States Patent No. 5,639,841, the relevant disclosure of which is incorporated herein by reference.

The terms "halogen-substituted," "hydroxy-substituted," "carboxy-substituted," "polyoxyalkylene-substituted," "alkyl-substituted," and "aryl-substituted" as used herein in reference to alkyl or aryl groups, and the like, mean that at least one hydrogen atom on an alkyl, aryl, or like group has been replaced by at least one halogen atom, hydroxyl group, carboxyl group, polyoxyalkylene group, alkyl group, or aryl group, respectively. The terms "poly(meth)acrylate" and "poly(meth)acrylamide" as used herein refer in the alternative to polyacrylate or polymethacrylate, and to polyacrylamide or polymethacrylamide, respectively.

Suitable monomers useful in the preparation of the multi-purpose polymers are described below. Regarding the monomers, the monomer mixtures, and the amount of each component of the "charges" utilized to form the multi-purpose polymers of the present invention, the following should be noted. Although the total amount of each monomer or component of a various "charge" may individually total more then 100 weight percent when each component is taken individually and totaled using the broadest amounts disclosed herein, one of skill in the art will realize that this is not the case. Rather, each individual component (i.e., components (a) through (e) inclusive and, if present, components (f), and (g)) of a various reaction "charge" of the present invention can be varied within any stated range as desired in order to achieve a total weight percent of 100.

**ASMA Monomer:**

Amino-substituted meth(acrylate) (ASMA) monomers suitable for the preparation of the multi-purpose polymers are basic, polymerizable, ethylenically unsaturated monomers that contain at least one amino functional group. These basic amino groups can be derived from mono-, di- or poly-amino alkyl groups or nitrogen containing heteroaromatic groups. The amino group can comprise primary, secondary or tertiary amines. The monomers can be used in the amino form or in the salt form, as desired.

The polymers include, in one embodiment, one or more ASMA monomers selected from the monomers represented by Formulas (I) and (II) shown below:

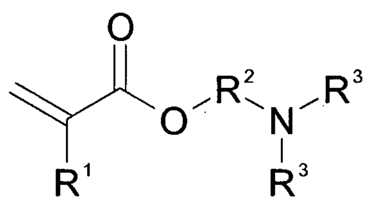

(I)

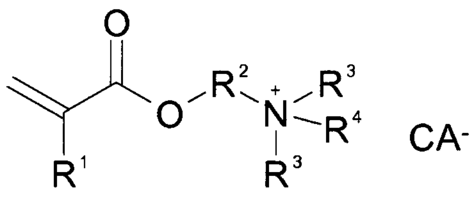

(II)

where $R^1$ is methyl; where $R^2$ is a substituted or unsubstituted, linear or branched $C_2$ to $C_8$ alkanediyl group (i.e., an

alkane group having at least two free valencies), with the proviso that when $R^2$ has two carbons at least one of the two carbon atoms of the $R^2$ group is substituted (e.g., mono-substituted or disubstituted) with a linear or branched $C_1$ to $C_{30}$ alkyl group; where each $R^3$ is independently selected from hydrogen, linear or branched $C_1$ to $C_{30}$ alkyl groups, linear or branched $C_1$ to $C_{30}$ alkyl groups that contain one or more heteroatoms, linear or branched $C_2$ to $C_{30}$ alkenyl groups, linear or branched $C_2$ to $C_{30}$ alkenyl groups that contain one or more heteroatoms, linear or branched $C_2$ to $C_{30}$ alkynyl groups, linear or branched $C_2$ to $C_{30}$ alkynyl groups that contain one or more heteroatoms, $C_4$ to $C_{20}$ aryl groups, $C_4$ to $C_{20}$ aryl groups that contain one or more heteroatoms, $C_4$ to $C_{20}$ cycloalkyl groups, $C_4$ to $C_{20}$ cycloalkyl groups that contain one or more heteroatoms, $C_4$ to $C_{20}$ heterocyclic groups, or where both $R^3$ substituents and the nitrogen atom to which they are attached can form a saturated or unsaturated $C_2$ to $C_{20}$ heterocyclic group or a saturated or unsaturated $C_2$ to $C_{20}$ heterocyclic group having two or more heteroatoms, where the heteroatoms, if present, are selected from a carbonyl group, N, S, P or O; $R^4$ is a linear or branched $C_1$ to $C_{30}$ alkyl group; and where CA- is a counter-anion suitable to balance the charge on the quaternary ammonium moiety. In another embodiment, $R^1$ is methyl; where $R^2$ is a substituted or unsubstituted, linear or branched $C_3$ to $C_7$ alkanediyl group (i.e., an alkane group having at least two free valencies); where each $R^3$ is independently selected from hydrogen, linear or branched $C_3$ to $C_{15}$ alkyl groups, linear or branched $C_3$ to $C_{15}$ alkyl groups that contain one or more heteroatoms, linear or branched $C_4$ to $C_{20}$ alkenyl groups, linear or branched $C_4$ to $C_{20}$ alkenyl groups that contain one or more heteroatoms, linear or branched $C_4$ to $C_{20}$ alkynyl groups, linear or branched $C_4$ to $C_{20}$ alkynyl groups that contain one or more heteroatoms, $C_5$ to $C_{10}$ aryl groups, $C_5$ to $C_{10}$ aryl groups that contain one or more heteroatoms, $C_5$ to $C_{10}$ cycloalkyl groups, $C_5$ to $C_{10}$ cycloalkyl groups that contain one or more heteroatoms, $C_5$ to $C_{10}$ heterocyclic groups, or where both $R^3$ substituents and the nitrogen atom to which they are attached can form a saturated or unsaturated $C_3$ to $C_{10}$ heterocyclic group or a saturated or unsaturated $C_3$ to $C_{10}$ heterocyclic group having two or more heteroatoms, where the heteroatoms, if present, are selected from a carbonyl group, N, S, P or O; $R^4$ is a linear or branched $C_2$ to $C_{20}$ alkyl group; and where $CA^-$ is a counter-anion suitable to balance the charge on the quaternary ammonium moiety. Here, as well as elsewhere in the specification and claims, individual numerical values (including carbon atom numerical values), or limits, can be combined to form additional non-disclosed and/or non-stated ranges.

In still another embodiment, $R^1$ is methyl; $R^2$ is a substituted or unsubstituted, linear or branched $C_4$ to $C_6$ alkanediyl group (i.e., an alkane group having at least two free valencies); each $R^3$ is independently selected from hydrogen, linear or branched $C_5$ to $C_8$ alkyl groups, linear or branched $C_5$ to $C_8$ alkyl groups that contain one or more heteroatoms, linear or branched $C_6$ to $C_{10}$ alkenyl groups, linear or branched $C_6$ to $C_{10}$ alkenyl groups that contain one or more heteroatoms, linear or branched $C_6$ to $C_{10}$ alkynyl groups, linear or branched $C_6$ to $C_{10}$ alkynyl groups that contain one or more heteroatoms, $C_5$ to $C_8$ aryl groups, $C_5$ to $C_8$ aryl groups that contain one or more heteroatoms, $C_5$ to $C_8$ cycloalkyl groups, $C_5$ to $C_8$ cycloalkyl groups that contain one or more heteroatoms, $C_5$ to $C_8$ heterocyclic groups, or where both $R^3$ substituents and the nitrogen atom to which they are attached can form a saturated or unsaturated $C_4$ to $C_8$ heterocyclic group or a saturated or unsaturated $C_4$ to $C_8$ heterocyclic group having two or more heteroatoms, where the heteroatoms, if present, are selected from a carbonyl group, N, S, P or O; and where $R^4$ is a linear or branched $C_3$ to $C_{15}$ alkyl group; and where $CA^-$ is a counter-anion suitable to balance the charge on the quaternary ammonium moiety. Here, as well as elsewhere in the specification and claims, individual numerical values (including carbon atom numerical values), or limits, can be combined to form additional non-disclosed and/or non-stated ranges.

Regarding the compounds of Formula (II), these compounds are the corresponding quaternary ammonium compounds of the compounds represented by Formula (I). As would be apparent to those of skill in the art, the monomer compounds of Formula (II) are produced by subjecting a compound represented by Formula (I) to quaternization reaction with a alkyl halide compound of the formula CA-$R^4$ where $R^4$ is a linear or branch alkyl group as defined above and where CA is any suitable species and/or moiety that yields a counter-anion when reacted to form the quaternary ammonium compounds of the compounds represented by Formula (II). In one embodiment, suitable species and/or moieties for CA and/or CA- include, but are not limited to, halogens (e.g., bromine, chlorine, fluorine or iodine), sulfate, sulfonate, phosphate, and phosphonate. In another embodiment, suitable species and/or moieties for CA and/or CA- include, but are not limited to, chloride, bromide, methosulfate (i.e., methylsulfate), acetate, formate, sulfate, nitrate, and the like. In still another embodiment CA and/or CA- is a halogen such as chlorine, bromine or fluorine.

In still another embodiment, the monomers of Formula (I) can be reacted with hydrogen peroxide ($H2O_2$) to yield an amine oxide compound represented by the following Formula:

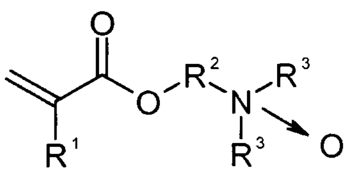

(Ia)

where $R^1$, $R^2$ and $R^3$ are as defined above. Additionally, either of the above-mentioned the conversion processes from Formula (I) to Formula (II) or the above amine oxide can be done after the multi-purpose polymers have been made.

In another embodiment, the ASMA monomer charge is a mixture of compounds according to Formula (I) and (II). In one instance, the weight ratio of the amount of monomer according to Formula (I) to quaternary monomer according to Formula (II) is in the range of from about 5:95 to about 95:5, or from about 10:90 to 90:10, or from about 15:85 to 85:15, or from about 20:80 to about 80:20, or from about 25:75 to about 75:25, or from about 30:70 to 70:30, or from about 35:65 to 65:35, or from about 40:60 to about 60:40, or from about 45:55 to about 55:45, or even about 50:50. Here, as well as elsewhere in the specification and claims, individual numerical values, or limits, can be combined to form additional non-disclosed and/or non-stated ranges.

As used throughout the specification and claims, the term "alkanediyl" is defined to mean an alkane group having at least two free valencies. The free valencies can be located at a terminal position(s) on the radical and/or situated on any carbon atom in the backbone of the radical. For illustrative purposes, non-limiting examples of linear and branched alkanediyl moieties are (the lines attached to only a single carbon atom represent a free valence):

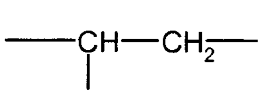 , 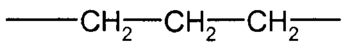 , 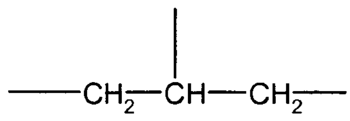 ,

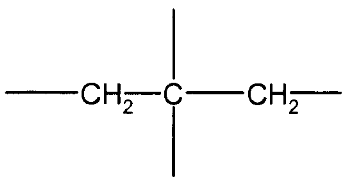 , 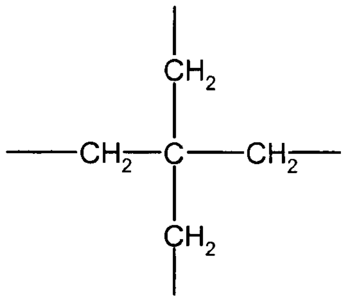

Examples of particular ASMA monomers include, but are not limited to, those shown below:

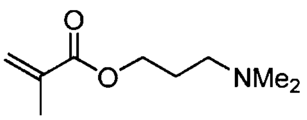

3-(dimethylamino)propyl methacrylate

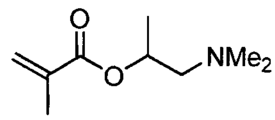

1-(dimethylamino)propan-2-yl methacrylate

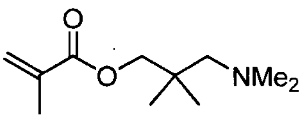

3-(dimethylamino)-2,2-dimethylpropyl methacrylate

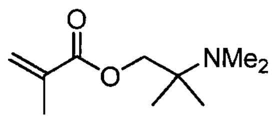

2-(dimethylamino)-2-methylpropyl methacrylate

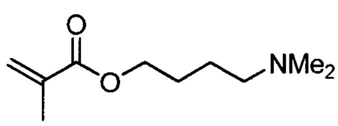

4-(dimethylamino)butyl methacrylate

where the above compounds can be represented by the following acronyms 3-(dimethylamino)propyl methacrylate (DMAPMA); 2-(dimethylamino)propan-2-yl methacrylate (DMAIPMA); 3-(dimethylamino)-2,2-dimethylpropyl methacrylate (DMADMPMA); 2-(dimethylamino)-2-methylpropyl methacrylate (DMAMPMA); and 4-(dimethylamino)butyl methacrylate (DMABMA).

Suitable salt forms of the above monomers include, but are not limited to, mineral acid salts such as the hydrochloride, sulfate, and phosphate salts; and organic acid salts such as the acetate, citrate, glycolate, lactate, maleate, and fumarate salts; and the like.

The foregoing monomers or salts thereof can be used as the amino-substituted vinyl monomer component of the inventive multi-purpose polymers, individually, or in mixtures of two or more.

The ASMA monomer comprises about 5 weight percent to about 90 weight percent, or from about 10 weight percent to about 80, or from about 15 weight percent to about 70 weight percent, or from about 20 weight percent to about 60 weight percent, or from about 25 weight percent to about 50 weight percent, or even from about 30 weight percent to about 40 weight percent, based on a total monomer mixture weight basis. Here, as well as elsewhere in the specification and claims, individual numerical values (including carbon atom numerical values), or limits, can be combined to form additional non-disclosed and/or non-stated ranges.

Due to the use of an ASMA monomer selected from Formula (I), I(a), and/or (II) shown above, the polymers utilize one or more hydrolytically stable amine monomer for producing multi-purpose copolymers with improved shelf lives. As mentioned above, the multi-purpose copolymers have a shelf life of at least about six months, at least about nine months, at least about 12 months, at least about 18 months, or even a shelf life of at least about 24 months or longer. Here, as well as elsewhere in the specification and claims, individual numerical values (including carbon atom numerical values), or limits, can be combined to form additional non-disclosed and/or non-stated ranges.

By "shelf life" it is meant that the multi-purpose copolymers remain stable even when in an aqueous solution, or environment, and do not undergo excess degradation due to the hydrolysis reaction discussed above.

While not wishing to be bound to any one theory, it is believed that the multi-purpose copolymers remain stable even when in an aqueous solution, or environment, and do not undergo excess degradation due to the hydrolysis reaction discussed above due to a number of factors including, but not limited to, an extended chain length (linear and branched) between the amino functionality, or functionalities, and the acrylate functionality. In still another embodiment, surprisingly it has been found that hydrolysis can be mitigated and/or eliminated in the ester moiety of the amino-ester portion of a multi-purpose polymer formed in accordance with the various embodiments of the present invention by utilizing an $R^2$ group containing branching and/or an extended $R^2$ in accordance with the embodiments stated above. It is believed that this reduces the tendency for the formation of the five member ring intermediate detailed above in the hydrolysis reaction. Concurrently, this approach also increases the hydrophobicity of the monomer and decreases its solubility in water. Surprisingly, the combination of both these approaches (increasing the chain length and hydrophobicity of the ASMA monomer molecule) offer unexpected stability performance in multi-purpose copolymers formed therefrom in accordance with the present invention. Additionally, "protection" of the terminal vinyl ester group by substitution of the interior hydrogen atom with a linear or branch alkyl group as discussed above with regard to Formulas (I) and (II) also offers improved and unexpected stability in an multi-purpose copolymer formed therefrom in accordance with the present invention.

The ASMA monomers can be utilized to mitigate the hydrolytic instability inherent in polymers that comprise repeating units polymerized from dimethylaminoethyl methacrylate (DMAEMA). The ASMA monomers can be incorporated into the polymerizable monomer mixture that comprises the DMAEMA monomer to mitigate the hydrolytic instability of polymers containing DMAEMA derived repeating units. In one aspect of the invention, the amount of ASMA monomer that can be included in the polymerizable DMAEMA containing monomer mixture ranges from about 1 to 99 parts by weight of the ASMA monomer(s) to about 99 to 1 parts by weight of the DMAEMA monomer based on 100 parts by weight of the combined weight of the ASMA monomer(s) and the DMAEMA monomer. In another aspect, the monomer mixture contains from about 10 to 90 parts by weight of the ASMA monomer(s) and about 90 to 10 parts by weight of the DMAEMA monomer. In a further aspect, the monomer mixture contains from about 20 to 80 parts by weight of the ASMA monomer(s) and about 80 to 20 parts by weight of the DMAEMA monomer. In yet another aspect, the monomer mixture contains from about 30 to 70 parts by weight of the ASMA monomer(s) and about 70 to 30 parts by weight of the DMAEMA monomer. In a still further aspect, the monomer mixture contains from about 40 to 60 parts by weight of the ASMA monomer(s) and about 60 to 40 parts by weight of the DMAEMA monomer. In yet a further aspect, the monomer mixture contains from about 50 parts by weight of the ASMA monomer(s) and about 50 parts by weight of the DMAEMA monomer. All of the foregoing aspects are based on 100 parts by weight of the combined weight of the ASMA monomer(s) and the DMAEMA monomer. Other polymerizable monomers can be incorporated into the polymerizable monomer mixture, such as, for example, one or more of the NIV, V, VS, PSM, and XL monomers described hereinabove.

Suitable ASMA monomers useful in mitigating the adverse hydrolytic susceptibility of DMAEMA derived polymers include one or more ASMA monomers conforming to formulae I, I(a), and II described above. Representative ASMA monomers are selected from 3-(dimethylamino)propyl methacrylate (DMAPMA); 2-(dimethylamino)propan-2-yl methacrylate (DMAIPMA); 3-(dimethylamino)-2,2-dimethylpropyl methacrylate (DMADMPMA); 2-(dimethylamino)-2-methylpropyl methacrylate (DMAMPMA); and 4-(dimethylamino)butyl methacrylate (DMABMA). The monomers conforming to formulae I, I(a), and II and the particular representative monomers disclosed herein can be utilized in the

amounts and ranges set forth immediately above.

**NIV Monomer:**

Nonionic vinyl (NIV) monomers suitable for use in the preparation of the inventive multi-purpose polymers are co-polymerizable, nonionic, ethylenically unsaturated monomers represented by Formulas (III) and/or (IV):

$$C(X)_2=C(X)Z \qquad (III)$$

$$CH_2=CH\text{-}OC(O)R_1 \qquad (IV)$$

wherein, in each of Formulas (III) and (IV), each X is independently hydrogen, methyl, $-CH_2C(O)OR_1$, $-C(O)OR_1$; and Z is $-C(O)OR_1$, $-C_6H_4R_1$, $-C_6H_4OR_1$, -CN, $-C(O)N(R_1)_2$, $-NHC(O)CH_3$, -NHC(O)H, $-C(O)OA'OR_{15}$, N-(2-pyrrolidonyl), N-caprolactamyl, $-C(O)NHCH_2CH_2$-N-ethyleneurea, or $-C(O)NHC(CH_3)_3$; A' is a divalent radical selected from $-CH_2CH(OH)CH_2-$ and $-CH_2CH(CH_2OH)-$, each $R_1$ is independently linear and branched $C_1$ to $C_{30}$ alkyl, hydroxy-substituted $C_2$ to $C_{30}$ alkyl, $C_5$ to $C_{30}$ cycloalkyl, and $C_1$ to $C_5$ alkyl-substituted $C_5$ to $C_{30}$ cycloalkyl; $R_{15}$ is an acyl residue of a linear or branched, saturated or unsaturated $C_6$ to $C_{22}$ fatty acid.
Non-limiting examples of suitable nonionic vinyl monomers include $C_1$ to $C_{30}$ alkyl (meth)acrylates; $C_1$ to $C_{30}$ alkyl (meth)acrylamides; styrene; substituted styrenes such as vinyl toluene, (e.g., 2-methyl styrene), butyl styrene, isopropyl styrene, and the like; vinyl esters such as vinyl acetate, vinyl butyrate, vinyl pivalate, vinyl hexanoate,. vinyl caprolate, vinyl nonanoate, vinyl decanoate, vinyl neodecanoate, vinyl undecanoate, vinyl laurate, and the like; unsaturated nitriles such as methacrylonitrile, acrylonitrile, and monomers that are available under the trade names of ACE™ and (M)ACE™ monomers from Hexion Specialty Chemicals, Inc., Columbus, OH.

The ACE monomer (CAS No. 94624-09-06) is the reaction product of glycidyl t-decanoate (CAS No. 71206-09-2) and acrylic acid. The (M)ACE Monomer is synthesized by reacting glycidyl t-decanoate and methacrylic acid. Other monomers set forth under Formula (III) wherein Z is $-C(O)OA'OR_{15}$ can be synthesized via esterification by reacting glycidol with a $C_6$ to $C_{22}$ fatty acid to obtain the glycidyl ester of the respective fatty acid(s). The so-formed glycidyl ester can in turn be reacted through its epoxy functionality with the carboxyl moiety of (meth)acrylic acid to obtain a preformed monomer.

In one aspect of the invention, suitable glycidyl esters for forming the preformed monomer components (ACE and (M)ACE)) described above are disclosed in U.S. Patent No. 5,179,157 (column 13), the relevant disclosure of which is herein incorporated by reference. A glycidyl ester of neodecanoic acid and isomers thereof are commercially available under the trade name Cardura™ E10P from Hexion Specialty Chemicals, Inc.

In another embodiment, nonionic vinyl (NIV) monomers include $C_1$ to $C_{30}$ alkyl esters of acrylic acid and of methacrylic acid and mixtures thereof, such as methyl acrylate (MA), ethyl acrylate (EA), methyl methacrylate (MMA), n-butyl acrylate (nBA), 2-ethylhexyl acrylate (2-EHA), 3,3,5-trimethylcyclohexyl methacrylate (TMCHMA), stearyl methacrylate (SMA), and suitable mixtures of any two or more thereof.

In still another embodiment, suitable nonionic vinyl (NIV) monomers for use in conjunction with the present invention include, but are not limited to, vinyl monomers that include $C_1$ to $C_{30}$ alkyl esters of acrylic acid and of methacrylic acid, hydroxy $C_2$ to $C_{30}$ alkyl esters of acrylic acid and methacrylic acid, and mixtures of any two or more thereof. Examples of which include, but are not limited to, methyl acrylate (MA), ethyl acrylate (EA), methyl methacrylate (MMA), n-butyl acrylate (nBA), 2-ethylhexyl acrylate (2EHA), and stearyl methacrylate (SMA); unsaturated $C_1$ to $C_{30}$ dialkyl dicarboxylates such as diethyl itaconate; vinyl esters such as vinyl neodecanoate, vinyl nonoate, and vinyl undecanoate; N-vinyl pyrrolidone; $C_1$ to $C_{30}$ alkyl (meth)acrylamides such as methacrylamide and t-butyl methacrylamide; styrene; carbocyclic $C_1$ to $C_{30}$ alkyl substituted methacrylates such as cyclohexyl methacrylates, and 3,3,5-trimethylcyclohexyl methacrylate (TMCHMA), and suitable mixtures of any two or more thereof.

In one embodiment, the NIV monomer comprises about 0.01 weight percent to about 90 weight percent, or from about 0.1 weight percent to about 85 weight percent, or from about 1 weight percent to about 80 weight percent, or from about 5 weight percent to about 75 weight percent, or from about 10 weight percent to about 70 weight percent, or from about 15 weight percent to about 65 weight percent, or from about 20 weight percent to about 60 weight percent, or from about 25 weight percent to about 55 weight percent, or from about 30 weight percent to about 50 weight percent, or even from about 35 weight percent to about 45 weight percent, based on a total monomer mixture weight basis. Here, as well as elsewhere in the specification and claims, individual numerical values (including carbon atom numerical values), or limits, can be combined to form additional non-disclosed and/or non-stated ranges.

In still another embodiment, the inventive multi-purpose polymers can be prepared from monomer mixtures containing NIV monomers that are ethylenically unsaturated monomers comprising one or more hydroxyl substituents. Examples of suitable NIV monomers of this type include, but are not limited to, a hydroxy-substituted ($C_1$ to $C_4$)alkyl (meth)acrylate such as 2-hydroxyethyl methacrylate (HEMA), 2-hydroxyethyl acrylate (2-HEA), 3-hydroxypropyl acrylate,

and the like; a hydroxy-substituted ($C_1$ to $C_4$)alkyl (meth)acrylamide such as N-(2-hydroxyethyl) methacrylamide, N-(2-hydroxyethyl) acrylamide, N-(3-hydroxypropyl) acrylamide, N-(2,3-dihydroxypropyl) acrylamide, and the like. Other useful NIV monomers include allyl alcohol, glycerol monoallyl ether, 3-methyl-3-buten-1-ol, and vinyl alcohol precursors and equivalents, such as vinyl acetate.

In one embodiment, the monomer reaction mixture of the present invention contains, in one embodiment, one or more of any of the NIV monomers disclosed above in amounts up to about 15 weight percent based on the total monomer mixture weight. In a another embodiment, the amount of NIV monomer in the mixture is in the range of about 0.01 weight percent to about 15 weight percent, or from about 0.1 weight percent to about 10 weight percent, or from about 1 weight percent to about 8 weight percent, or even from about 1 weight percent to about 5 weight percent, based on a total monomer mixture weight basis. Here, as well as elsewhere in the specification and claims, individual numerical values (including carbon atom numerical values), or limits, can be combined to form additional non-disclosed and/or non-stated ranges.

**VA Monomer:**

Vinyl associative monomers (VA) suitable for use in the production of the multi-purpose polymers are compounds that in one embodiment have: (A) an ethylenically unsaturated end group portion for addition polymerization with the other monomers of the system; (B) a polyoxyalkylene midsection portion for imparting selective hydrophilic properties to the product polymer; and (C) a hydrophobic end group portion for providing selective hydrophobic properties to the polymer.

The portion (A) supplying the ethylenically unsaturated end group is, in one embodiment, derived from an $\alpha,\beta$-ethylenically unsaturated mono or dicarboxylic acid or the anhydride thereof, or even a $C_3$ or $C_4$ mono- or dicarboxylic acid or the anhydride thereof. Alternatively, portion (A) of the vinyl associative (VA) monomer can be derived from an allyl ether or vinyl ether; a nonionic vinyl-substituted urethane monomer, such as disclosed in United States Reissue Patent No. 33,156 or United States Patent No. 5,294,692; or a vinyl-substituted urea reaction product such as disclosed in United States Patent No. 5,011,978; the relevant disclosures of which are incorporated herein by reference.

The midsection portion (B) is, in one embodiment, a polyoxyalkylene segment of about 5 to about 250, or from about 10 to about 120, or even from about 15 to about 60 repeating $C_2$ to $C_7$ alkylene oxide units. In one embodiment, midsection portions (B) include polyoxyethylene, polyoxypropylene, and polyoxybutylene segments comprising about 5 to about 150, or from about 10 to about 100, or even from about 15 to about 60 ethylene, propylene or butylene oxide units, and random or non-random sequences of ethylene oxide, propylene oxide and or butylene oxide units. Here, as well as elsewhere in the specification and claims, individual numerical values (including carbon atom numerical values), or limits, can be combined to form additional non-disclosed and/or non-stated ranges.

The hydrophobic end group portion (C) of the vinyl associative (VA) monomers is, in one embodiment, a hydrocarbon moiety belonging to one of the following hydrocarbon classes: a $C_8$ to $C_{40}$ linear alkyl, an aryl-substituted $C_2$ to $C_{40}$ alkyl, a $C_2$ to $C_{40}$ alkyl-substituted phenyl, a $C_8$ to $C_{40}$ branched alkyl, a $C_8$ to $C_{40}$ carbocyclic alkyl; and a $C_8$ to $C_{80}$ complex ester.

Non-limiting examples of suitable hydrophobic end group portions (C) of the vinyl associative (VA) monomers are linear or branched alkyl groups having about 8 to about 40 carbon atoms such as capryl ($C_8$), isooctyl (branched $C_8$), decyl ($C_{10}$), lauryl ($C_{12}$), myristyl ($C_{14}$), cetyl ($C_{16}$), cetearyl ($C_{16}$ to $C_{18}$), stearyl ($C_{18}$), isostearyl (branched $C_{18}$), arachidyl ($C_{20}$), behenyl ($C_{22}$), lignoceryl ($C_{24}$), cerotyl ($C_{26}$), montanyl ($C_{28}$), melissyl ($C_{30}$), lacceryl ($C_{32}$), and the like.

Examples of linear and branched alkyl groups having about 8 to about 40 carbon atoms that are derived from a natural source include, but are not limited to, alkyl groups derived from hydrogenated peanut oil, soybean oil and canola oil (all predominately $C_{18}$), hydrogenated tallow oil ($C_{16}$ to $C_{18}$), and the like; and hydrogenated $C_{10}$ to $C_{30}$ terpenols, such as hydrogenated geraniol (branched $C_{10}$), hydrogenated farnesol (branched $C_{15}$), hydrogenated phytol (branched $C_{20}$), and the like.

Non-limiting examples of suitable $C_2$ to $C_{40}$ alkyl-substituted phenyl groups include octylphenyl, nonylphenyl, decylphenyl, dodecylphenyl, hexadecylphenyl, octadecylphenyl, isooctylphenyl, sec-butylphenyl, and the like.

Suitable $C_8$ to $C_{40}$ carbocylic alkyl groups include, without being limited thereto, groups derived from sterols from animal sources, such as cholesterol, lanosterol, 7-dehydrocholesterol, and the like; from vegetable sources, such as phytosterol, stigmasterol, campesterol, and the like; and from yeast sources, such as ergosterol, mycosterol, and the like. Other carbocyclic alkyl hydrophobic end groups useful in the present invention include, without being limited thereto, cyclooctyl, cyclododecyl, adamantyl, decahydronaphthyl, and groups derived from natural carbocyclic materials such as pinene, hydrogenated retinol, camphor, isobornyl alcohol, and the like.

Exemplary aryl-substituted $C_2$ to $C_{40}$ alkyl groups include, but are not limited to, styryl (e.g., 2-phenylethyl), distyryl (e.g., 2,4-diphenylbutyl), tristyryl

(e.g., 2,4,6-triphenylhexyl), 4-phenylbutyl, 2-methyl-2-phenylethyl, tristyrylphenolyl, and the like.

Non-limiting examples of suitable $C_8$ to $C_{80}$ complex esters include, but are not limited to, hydrogenated castor

oil (predominately the triglyceride of 12-hydroxystearic acid); 1,2-diacyl glycerols such as 1,2-distearyl glycerol, 1,2-dipalmityl glycerol, 1,2-dimyristyl glycerol, and the like; di-, tri-, or poly-esters of sugars such as 3,4,6-tristearyl glucose, 2,3-dilauryl fructose, and the like; and sorbitan esters such as those disclosed in United States Patent No. 4,600,761, the relevant disclosures of which are incorporated herein by reference.

Useful monomers can be prepared by any method known in the art. See, for example, United States Patent Nos. 4,421,902; 4,384,096; 4,514,552; 4,600,761; 4,616,074; 5,294,692; 5,292,843; 5,770,760; and 5,412,142; the relevant disclosures of which are incorporated herein by reference.

Examples of vinyl associative (VA) monomers useful for use in connection with the present invention include, in one embodiment, those represented by Formula (V):

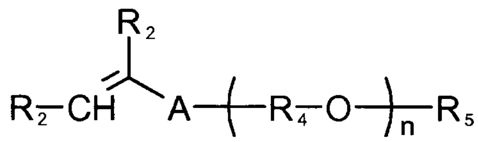
(V)

wherein, each $R_2$ is independently H, methyl, -C(O)OH, or -C(O)OR$_3$; $R_3$ is $C_1$ to $C_{30}$ alkyl; A is -CH$_2$C(O)O-, -C(O)O-, -O-, or -CH$_2$O-; $(R_4\text{-}O)_n$ is a polyoxyalkylene, which is a homopolymer, a random copolymer, or a block copolymer of $C_2$ to $C_4$ oxyalkylene units, wherein each $R_4$ is independently $C_2H_4$, $C_3H_6$, $C_4H_8$, or a mixture thereof, and n is an integer in the range of about 5 to about 250, or from about 5 to about 100, or from about 10 to about 80, or even from about 15 to about 60; and $R_5$ is a substituted or unsubstituted alkyl selected linear or branched $C_8$ to $C_{40}$ alkyls, $C_8$ to $C_{40}$ carbocyclic alkyls, $C_2$ to $C_{40}$ alkyl-substituted phenyls, aryl-substituted $C_2$ to $C_{40}$ alkyls, and $C_8$ to $C_{80}$ complex esters, wherein the $R_5$ alkyl group optionally comprises one or more substituents selected a hydroxyl group, an alkoxyl group, and/or a halogen group.

In one embodiment, suitable vinyl associative (VA) monomers of Formula (V) include, but are not limited to, cetyl polyethoxylated methacrylate (CEM), cetearyl polyethoxylated methacrylate (CSEM), stearyl polyethoxylated (meth)acrylate, arachidyl polyethoxylated (meth)acrylate, behenyl polyethoxylated methacrylate (BEM), lauryl polyethoxylated methacrylate (LEM), cerotyl polyethoxylated (meth)acrylate, montanyl polyethoxylated (meth)acrylate, melissyl polyethoxylated (meth)acrylate, lacceryl polyethoxylated (meth)acrylate, tristyryl phenolpolyethoxylated methacrylate (TEM), hydrogenated castor oil polyethoxylated methacrylate (HCOEM), canola polyethoxylated (meth)acrylate, and cholesterol polyethoxylated methacrylate (CHEM), where the polyethoxylated portion of the monomer comprises about 5 to about 100, or from about 10 to about 80, or even from about 15 to about 60 ethylene oxide repeating units.

In one embodiment, the V monomer component in the monomer mixture comprises about 0.001 weight percent to about 25 weight percent of the monomer mixture, or from about 0.01 weight percent to about 20 weight percent, or from about 0.1 weight percent to about 15 weight percent, or even from about 1 weight percent to about 10 weight percent, on a total monomer mixture weight basis. Here, as well as elsewhere in the specification and claims, individual numerical values (including carbon atom numerical values), or limits, can be combined to form additional non-disclosed and/or non-stated ranges.

**VS Monomer:**

It has been found, surprisingly, that a vinyl surfactant (VS) monomer, which contains a polyoxyalkylene chain, can moderate the associative properties of multi-purpose polymers containing them, thus producing aqueous gels with highly desirable texture and rheological properties. While not wishing to be bound to any one theory, it is thought that the polyoxyalkylene group of the VS monomer interrupts or shields against non-specific associations between the hydrophobic groups of the associative monomers in the polymer and thus attenuates the associative properties of the polymers. Such VS monomers can tailor the thickening efficiency of the resulting polymers to customize the rheological properties of the polymer as desired for a selected application. Most surprisingly, the VS monomers are found to impart desirable rheological and aesthetic properties to aqueous gels, providing softer, smoother and more spreadable gels than multi-purpose polymers containing no VS monomer.

Surprisingly, incorporation of a VS monomer into a multi-purpose polymer can minimize or diminish viscosity reduction under low shear stress and can provide a shear thinning profile that is smooth flowing.

The polyoxyalkylene portion $(R_8\text{-}O)_v$ specifically comprises a long-chain polyoxyalkylene segment, which is substantially similar to the hydrophilic portion of the associative monomers. In one embodiment, polyoxyalkylene portion $(R_8\text{-}O)_v$ includes polyoxyethylene, polyoxypropylene, polyoxybutylene units, and combinations thereof comprising about 1 to about 250, or even about 5 to about 100 oxyalkylene units. When the VS monomer comprises more than one type of oxyalkylene unit, the units can be arranged in random, non-random, or block sequences.

Suitable VS monomers include, but are not limited to, those represented by Formula (VI):

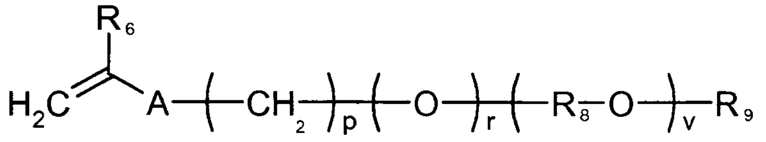 (VI)

where each $R_6$ is independently hydrogen or methyl, -C(O)OH, or -C(O)O$R_7$; $R_7$ is $C_1$ to $C_{30}$ alkyl; A is -$CH_2$C(O)O-, -C(O)O-, -O-, or -$CH_2$O-; p is an integer in the range of 0 to about 30, and r is 0 or 1, with the proviso that when p is 0, r is 0, and when p is in the range of 1 to about 30, r is 1; where $(R_8\text{-O})_v$ is a polyoxyalkylene moiety, which can be arranged as a homopolymer, a random copolymer or a block copolymer of $C_2$ to $C_4$ oxyalkylene units, wherein each $R_8$ is independently $C_2H_4$, $C_3H_6$, $C_4H_8$, or a mixture thereof, and v is an integer in the range of about 1 to about 250, or from about 4 to about 100, or from about 10 to about 80, or even from about 15 to about 60; and $R_9$ is hydrogen or $C_1$ to $C_4$ alkyl.

In one embodiment, suitable VS monomers include, but are not limited to, monomers having the following chemical Formulas:

$H_2C{=}CH\text{-}O(CH_2)_aO(C_3H_6O)_b(C_2H_4O)_cH$;

$H_2C{=}CHCH_2O(C_3H_6O)_d(C_2H_4O)_eH$;

$H_2C{=}C(Q)\text{-}C(O)\text{-}O(CH_2)_aO(C_3H_6O)_b(C_2H_4O)_cH$; or

$H_2C{=}C(Q)\text{-}C(O)\text{-}O(C_3H_6O)_d(C_2H_4O)_eH$

wherein Q is hydrogen or methyl; a is 2, 3, or 4; b is an integer in the range of 1 to about 20, or from about 2 to about 10, or even about 3 to about 7; c is an integer in the range of about 5 to about 50, or from about 8 to about 40, or even from about 10 to about 30; d is an integer in the range of 1 to about 20, or from about 2 to about 10, or even from about 3 to about 7; and e is an integer in the range of about 1 to about 50, or even from about 5 to about 40, or even from about 10 to about 25. In another embodiment, b or c can be zero. In still another embodiment, d or e can be zero.

Examples of suitable VS monomers include, but not limited to, polymerizable emulsifiers commercially available under the trade names EMULSOGEN® R109, R208, R307, RAL 100, RAL109, RAL208, and RAL307 sold by Clariant Corporation; BX-AA-E5P5 sold by Bimax, Inc.; and combinations thereof. In another embodiment, suitable VS monomers include, but are not limited to, EMULSOGEN® R208, R307, and RAL307.

According to the manufacturers: EMULSOGEN® R109 is a randomly ethoxylated/propoxylated 1,4-butanediol vinyl ether having the empirical formula $CH_2{=}CH\text{-}O(CH_2)_4O(C_3H_6O)_4(C_2H_4O)_{10}H$; EMULSOGEN® R208 is a randomly ethoxylated/propoxylated 1,4-butanediol vinyl ether having the empirical formula: $CH_2{=}CH\text{-}O(CH_2)_4O(C_3H_6O)_4(C_2H_4O)_{20}H$; EMULSOGEN® R307 is a randomly ethoxylated/propoxylated 1,4-butanediol vinyl ether having the empirical formula: $CH_2{=}CH\text{-}O(CH_2)_4O(C_3H_6O)_4(C_2H_4O)_{30}H$; EMULSOGEN® RAL109 is a randomly ethoxylated/propoxylated allyl ether having the empirical formula $CH_2{=}CHCH_2O(C_3H_6O)_4(C_2H_4O)_{10}H$; EMULSOGEN® RAL208 is a randomly ethoxylated/propoxylated allyl ether having the empirical formula $CH_2{=}CHCH_2O(C_3H_6O)_4(C_2H_4O)_{20}H$; EMULSOGEN® RAL307 is a randomly ethoxylated/propoxylated allyl ether having the empirical formula $CH_2{=}CHCH_2O(C_3H_6O)_4(C_2H_4O)_{30}H$; and BX-AA-E5P5 is a randomly ethoxylated/propoxylated allyl ether having the empirical formula $CH_2{=}CHCH_2O(C_3H_6O)_5(C_2H_4O)_5H$.

In one embodiment, suitable VS monomers that can be utilized in conjunction with the present invention include, but are not limited to, polyalkylene glycol monoallyl ethers (e.g., $CH_2{=}CHCH_2$ $(OCH_2CH_2)_nOH$ such as polyglycol A350 ($M_w$ = 350); polyglycol A500 ($M_w$ = 500); and/or polyglycol A1100 ($M_w$ = 1100); and/or $CH_2{=}CHCH_2$ $(OCH_2CH_2)_n(OCH_2CHCH_3)_mOH$ such as polyglycol A31/1000 - 3:1 ratio of EO:PO ($M_w$ = 1000); polyglycol A32/550 - 3:2 ratio of EO:PO ($M_w$ = 550); polyglycol A11/1800 - 1:1 ratio of EO:PO ($M_w$ = 1800); polyglycol A91/550 - 3:2 ratio of EO:PO ($M_w$ = 550); polyglycol A 11-4 - 3:1 ratio of EO:PO ($M_w$ = 750); and/or polyglycol A 20-20 - 1:1 ratio of EO:PO ($M_w$ = 2100), polyalkylene glycol monovinyl ethers (e.g., $CH_2{=}CH(OCH_2CH_2)_nOH$ such as polyglycol R-500 ($M_w$ = 500); polyglycol R-1100 ($M_w$ = 1100); and/or polyglycol R-5000 ($M_w$ = approximately 6000), polyethyleneglycol monomethylether monomethacrylate (i.e., $CH_3(OCH_2CH_2)_nOCOC(CH_3){=}CH_2$ such as Genagen M 750 MA ($M_w$ = 810 to 870) and/or Genagen M 1100 MA ($M_w$ = 1160 to 1220), or suitable mixtures of any two or more thereof.

The amount of VS monomers utilized in the preparation of the multi-purpose polymers can vary widely and depends, among other things, on the final rheological properties desired in the polymer. When utilized, the monomer reaction mixture contains, in one embodiment, at least about 0.01 weight percent of one or more VS monomers based on the total monomer mixture weight, or from even at least about 0.1 weight percent of one or more VS monomers based on the total monomer mixture weight. The monomer mixture comprises, in one embodiment, not more than about 25

weight percent of VS monomer, not more than about 20 weight percent of VS monomer, not more than about 15 weight percent of VS monomer, not more than about 10 weight percent of VS monomer, or even not more than about 7.5 weight percent of VS monomer, based on the total monomer mixture weight. In another embodiment, the monomer mixture comprises from about 0.01 weight percent to about 25 weight percent, or from about 0.1 weight percent to about 20 weight percent, or from about 0.5 weight percent to about 15 weight percent, or from about 1 weight percent to about 10 weight percent, or even from about 2 weight percent to about 7.5 weight percent of VS monomer, based on the total monomer mixture weight. Here, as well as elsewhere in the specification and claims, individual numerical values (including carbon atom numerical values), or limits, can be combined to form additional non-disclosed and/or non-stated ranges.

**Polymerizable Silicone Macromer (PSM):**

The inventive multi-purpose polymers can be prepared from monomer mixtures containing one or more polymerizable silicone macromers having one or more side, or lateral, chains that contain one or more repeating polyoxyalkylene units. The siloxane monomers useful in conjunction with the present invention can be represented by the following Formula:

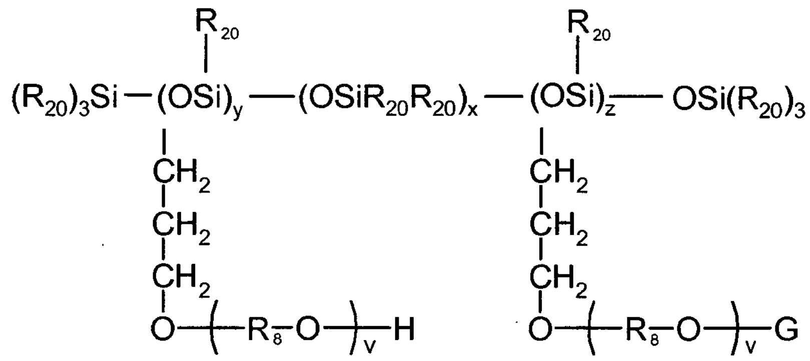

where each $R_{20}$ is independently selected from linear or branched $C_1$ to $C_{30}$ alkyl, $C_4$ to $C_{20}$ aryl, or $C_2$ to $C_{20}$ alkeneyl; where $(R_8\text{-}O)_v$ is a polyoxyalkylene moiety, which can be arranged as a homopolymer, a random copolymer or a block copolymer of $C_2$ to $C_4$ oxyalkylene units, wherein each $R_8$ is independently $C_2H_4$, $C_3H_6$, $C_4H_8$, or a mixture thereof, and v is an integer in the range of about 1 to about 250, or from about 4 to about 100, or from about 10 to about 80, or even from about 15 to about 60; x is an integer in the range of 0 to about 200; y is an integer in the range of 0 to about 200; and z is an integer in the range of 1 to about 200; and where G is selected from any moiety that contains at least one free radically polymerizable carbon-carbon double bond.

G is selected from any moiety containing a free radically polymerizable carbon-carbon double bond. In one embodiment, G is a residue obtained from the esterification or etherification reaction of a silicone copolyol with a carbon-carbon double bond containing reactant. Upon esterification or etherification the carbon-carbon double bond remains intact and is available for free radical polymerization when the silicone macromer is polymerized into the backbone of the multi-purpose polymers. In another embodiment, the carbon-carbon double bond containing reactant is selected from cyclic anhydrides, (e.g., itaconic anhydride, citraconic anhydride, maleic anhydride and isomers thereof), (meth)acrylic acid, vinyl alcohol, and allyl alcohol. In still another embodiment, G can be a residue represented by the following Formulae:

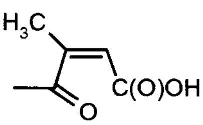 , 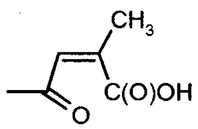 , 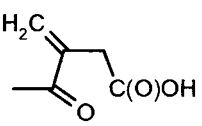 , 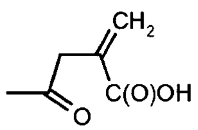 , 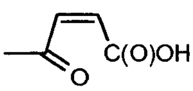 ,

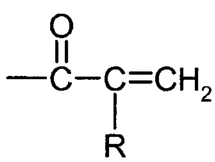, $-CH=CH_2$ , $-CH_2-CH=CH_2$

wherein this specific instance R represents hydrogen and methyl and the open bond line represents a covalent bond

with an oxygen atom on the silicone copolyol.

The esterification reaction occurs between a terminal hydroxyl group on the silicone copolyol and the anhydride or carboxylic acid group provided by the cyclic anhydride and (meth)acrylic acid reactants, respectively. Likewise, the etherification reaction occurs between a terminal hydroxyl group on the silicone copolyol and the hydroxyl group situated on the vinyl or allyl alcohol. Esterification and etherification reactions are well known in the art. The reaction of silicone copolyols and cyclic anhydrides to form free radically polymerizable silicone macromers are disclosed in PCT Publication No. WO 2007/101048, which is herein incorporated by reference.

In the esterification reaction between the silicone copolyol and cyclic anhydrides disclosed above, the half ester or monoester of the polymerizable silicone macromer is depicted. In this embodiment a residual carboxyl functional group remains on the moiety contributed by the cyclic anhydride residue following the esterification reaction. It is also within the scope of this invention that silicone diester macromers can be formed by adjusting the stoichiometry of the reactants to allow the residual carboxyl group to react with a terminal hydroxyl group provided by a second silicone copolyol to give a silicone macromer diester.

In another embodiment, each $R_{20}$ is independently selected from linear or branched $C_2$ to $C_{20}$ alkyl, $C_5$ to $C_{14}$ aryl, or $C_3$ to $C_{15}$ alkeneyl; where a is an integer in the range of 1 to about 50; b is an integer in the range of about 1 to about 50; x is an integer in the range of 1 to about 100; y is an integer in the range of 1 to about 100; and z is an integer in the range of 2 to about 100; and where G is as defined above. In still another embodiment, each $R_{20}$ is independently selected from linear or branched $C_3$ to $C_{10}$ alkyl, $C_6$ to $C_{10}$ aryl, or $C_4$ to $C_{10}$ alkeneyl; where a is an integer in the range of 5 to about 25; b is an integer in the range of about 5 to about 25; x is an integer in the range of 5 to about 50; y is an integer in the range of 5 to about 50; and z is an integer in the range of 5 to about 50; and where G is as defined above. Here, as well as elsewhere in the specification and claims, individual numerical values (including carbon atom numerical values), or limits, can be combined to form additional non-disclosed and/or non-stated ranges.

In one embodiment, the PSM monomer comprises about 0.01 weight percent to about 10 weight percent, or from about 0.1 weight percent to about 7.5 weight percent, or from about 0.5 weight percent to about 5 weight percent, or even from about 1 weight percent to about 2.5 weight percent, based on a total monomer mixture weight basis. Here, as well as elsewhere in the specification and claims, individual numerical values (including carbon atom numerical values), or limits, can be combined to form additional non-disclosed and/or non-stated ranges.

**XL Monomer:**

The inventive multi-purpose polymers can be prepared from a monomer mixture comprising one or more crosslinking monomers for introducing branching and controlling molecular weight. Suitable polyunsaturated crosslinkers are well known in the art. Mono-unsaturated compounds carrying a reactive group that is capable of causing a formed copolymer to be crosslinked before, during, or after polymerization has taken place can also be utilized. Other useful crosslinking monomers include polyfunctional monomers containing multiple reactive groups such as epoxide groups, isocyanate groups, and hydrolyzable silane groups. Various polyunsaturated compounds can be utilized to generate either a partially or substantially cross-linked three dimensional network.

Examples of suitable polyunsaturated crosslinking monomer components include, but are not limited to, polyunsaturated aromatic monomers such as divinylbenzene, divinyl naphthylene, and trivinylbenzene; polyunsaturated alicyclic monomers, such as 1,2,4-trivinylcyclohexane; di-functional esters of phthalic acid such as diallyl phthalate; polyunsaturated aliphatic monomers, such as dienes, trienes, and tetraenes, including isoprene, butadiene, 1,5-hexadiene, 1,5,9-decatriene, 1,9-decadiene, 1,5-heptadiene; and the like.

Other suitable polyunsaturated crosslinking monomers include, but are not limited to, polyalkenyl ethers such as triallyl pentaerythritol, diallyl pentaerythritol, diallyl sucrose, octaallyl sucrose, and trimethylolpropane diallyl ether; polyunsaturated esters of polyalcohols or polyacids such as 1,6-hexanediol di(meth)acrylate, tetramethylene tri(meth)acrylate, allyl (meth)acrylate, diallyl itaconate, diallyl fumarate, diallyl maleate, trimethylolpropane tri(meth)acrylate, trimethylolpropane di(meth)acrylate, and polyethylene glycol di(meth)acrylate; alkylene bisacrylamides, such as methylene bisacrylamide, propylene bisacrylamide, and the like; hydroxy and carboxy derivatives of methylene bisacrylamide, such as N,N'-bismethylol methylene bisacrylamide; polyethyleneglycol di(meth)acrylates, such as ethyleneglycol di(meth)acrylate, diethyleneglycol di(meth)acrylate, and triethyleneglycol di(meth)acrylate; polyunsaturated silanes, such as dimethyldivinylsilane, methyltrivinylsilane, allyldimethylvinylsilane, diallyldimethylsilane, and tetravinylsilane; polyunsaturated stannanes, such as tetraallyl tin, and diallyldimethyl tin; and the like.

Useful monounsaturated compounds carrying a reactive group include N-methylolacrylamide; N-alkoxy(meth)acrylamide, wherein the alkoxy group is a $C_1$ to $C_{18}$ alkoxy; and unsaturated hydrolyzable silanes such as triethoxyvinylsilane, tris-isopropoxyvinylsilane, and 3-triethoxysilylpropyl methacrylate; and the like.

Useful polyfunctional crosslinking monomers containing multiple reactive groups include, but are not limited to, hydrolyzable silanes such as ethyltriethoxysilane and ethyltrimethoxysilane; epoxy-substituted hydrolyzable silanes, such as 2-(3,4-epoxycyclohexyl)ethyltriethoxysilane and 3-glycidoxypropyltrimethyoxysilane; polyisocyanates, such as

1,4-diisocyanatobutane, 1,6-diisocyanatohexane, 1,4-phenylenediisocyanate, and 4,4'-oxybis(phenylisocyanate); unsaturated epoxides, such as glycidyl methacrylate and allylglycidyl ether; polyepoxides, such as diglycidyl ether, 1,2,5,6-diepoxyhexane, and ethyleneglycoldiglycidyl ether; and the like.

Also useful are polyunsaturated crosslinkers derived from ethoxylated polyols, such as diols, triols and bis-phenols, ethoxylated with about 2 to about 100 moles of ethylene oxide per mole of hydroxyl functional group and endcapped with a polymerizable unsaturated group such as a vinyl ether, allyl ether, acrylate ester, methacrylate ester, and the like. Examples of such crosslinkers include bisphenol A ethoxylated dimethacrylate; bisphenol F ethoxylated dimethacrylate, ethoxylated trimethylol propane trimethacrylate, and the like. Other ethoxylated crosslinkers useful in the multi-purpose polymers include ethoxylated polyol-derived crosslinkers disclosed in United States Patent No. 6,140,435.

Examples of particularly suitable XL monomers include, but are not limited to, acrylate and methacrylate esters of polyols having at least two acrylate or methacrylate ester groups, such as trimethylolpropane triacrylate (TMPTA), ethoxylated-3 trimethylolpropane triacrylate (TMPEO3TA), ethoxylated-15 trimethylolpropane triacrylate (TMPEO15TA), trimethylolpropane dimethacrylate, triethylene glycol dimethacrylate (TEGDMA), ethoxylated (30) bisphenol A dimethacrylate (EOBDMA); polyalkenyl ethers (APE) such as triallyl pentaerythritol, diallyl pentaerythritol, and trimethylolpropane diallyl ether (TMPDAE); sucrose allyl ethers (AS) such as diallyl sucrose, octaallyl sucrose; alkylene bisacrylamides, such as methylene bisacrylamide (MBA), propylene bisacrylamide; and suitable mixtures of any two or more thereof.

When utilized, crosslinking monomers are present in the monomer reaction mixture in an amount of up to about 5 weight percent, based on total monomer mixture weight. In another embodiment, the XL monomers are present in an amount in the range of about 0.001 weight percent to about 5 weight percent, or from about 0.01 weight percent to about 4 weight percent, or from about 0.05 weight percent to about 3 weight percent, or from about 0.1 weight percent to about 2 weight percent, or even from about 0.5 weight percent to about 1 weight percent of the monomer mixture based on the total monomer mixture weight. Here, as well as elsewhere in the specification and claims, individual numerical values (including carbon atom numerical values), or limits, can be combined to form additional non-disclosed and/or non-stated ranges.

**Chain Transfer Agent:**

The inventive multi-purpose polymers can optionally be prepared from a monomer mixture comprising one or more chain transfer agents (CTA), which are well known in the polymer arts.

Suitable chain transfer agents for use in this invention, without being limited thereto, are selected from a variety of thio- and disulfide-containing compounds, such as $C_1$ to $C_{18}$ alkyl mercaptans, mercaptocarboxylic acids, mercaptocarboxylic esters, thioesters, $C_1$ to $C_{18}$ alkyl disulfides, aryldisulfides, polyfunctional thiols, and the like; phosphites and hypophosphites; haloalkyl compounds, such as carbon tetrachloride, bromotrichloromethane, and the like; and unsaturated chain transfer agents, such as alpha-methylstyrene.

Polyfunctional thiols include trifunctional thiols, such as trimethylolpropane-tris-(3-mercaptopropionate), tetrafunctional thiols, such as pentaerythritol-tetra-(3-mercaptopropionate), pentaerythritol-tetra-(thioglycolate), and pentaerythritol-tetra-(thiolactate); hexafunctional thiols, such as dipentaerythritol-hexa-(thioglycolate); and the like.

Alternatively, the chain transfer agent can be any catalytic chain transfer agent which reduces molecular weight of addition polymers during free radical polymerization of vinyl monomers. Examples of catalytic chain transfer agents include, for example, cobalt complexes (e.g., cobalt (II) chelates). Catalytic chain transfer agents can often be utilized in relatively low concentrations relative to thiol-based CTAs.

Examples of suitable chain transfer agents include, but not limited to, octyl mercaptan, n-dodecyl mercaptan, t-dodecyl mercaptan, hexadecyl mercaptan, octadecyl mercaptan (ODM), isooctyl 3-mercaptopropionate (IMP), butyl 3-mercaptopropionate, 3-mercaptopropionic acid, butyl thioglycolate, isooctyl thioglycolate, dodecyl thioglycolate, and the like. The chain transfer agents can be added to a monomer reaction mixture in amounts of up to about 10 weight percent of polymerizable monomer mixture, based on total monomer mixture weight. When present, the chain transfer agent comprises at least about 0.05 weight percent of the mixture, based on the total monomer weight. Here, as well as elsewhere in the specification and claims, individual numerical values (including carbon atom numerical values), or limits, can be combined to form additional non-disclosed and/or non-stated ranges.

**Multi-Purpose Polymers and Methods for Producing Same:**

The inventive multi-purpose polymers can be synthesized by conventional polymerization techniques, such as, for example, by emulsion polymerization, inverse emulsion polymerization, solution polymerization, precipitation polymerization, mass polymerization, and dispersion polymerization. Such polymerization techniques are well known in the polymer art.

In one embodiment of the invention, the multi-purpose polymer is polymerized from its constituent monomers by emulsion polymerization which can be performed as a simple batch process, as a metered addition process, or the

reaction can be initiated as a small batch and then the bulk of the monomers can be continuously metered into the reactor (seed process). Typically the polymerization process is carried out at a reaction temperature in the range of about 20°C to about 95°C, however, higher or lower temperatures can be used. To facilitate emulsification of the monomer mixture, the emulsion polymerization is carried out in the presence of at least one surfactant. In one embodiment, the emulsion polymerization is carried out in the presence of surfactant in the amount of about 1 weight percent to about 10 weight percent, or in the range of about 3 weight percent to about 8 weight percent, or even in the range of about 5 weight percent to about 7 weight percent, on a total emulsion weight basis. Here, as well as elsewhere in the specification and claims, individual numerical values (including carbon atom numerical values), or limits, can be combined to form additional non-disclosed and/or non-stated ranges.

The emulsion polymerization reaction mixture also includes one or more free radical initiators. In one embodiment the one or more free radical initiators are present in an amount in the range of about 0.01 weight percent to about 3 weight percent based on total monomer weight. The polymerization can be performed in an aqueous or aqueous alcohol medium at neutral to moderately alkaline pH.

In a typical polymerization, a mixture of monomers is added with mixing agitation to a solution of emulsifying surfactant, such as a nonionic surfactant, in one embodiment this is a linear or branched alcohol ethoxylate, or mixtures of nonionic surfactants and anionic surfactants; such as fatty alcohol sulfates or alkyl sulfonates, in a suitable amount of water, in a suitable reactor, to prepare a monomer emulsion. The emulsion is deoxygenated by any convenient method, such as by sparging with nitrogen, and then a polymerization reaction is initiated by adding a polymerization catalyst (initiator) such as sodium persulfate, or any other suitable addition polymerization catalyst, as is well known in the emulsion polymerization art. The reaction is agitated until the polymerization is complete, typically for a time in the range of about 4 to about 16 hours. The monomer emulsion can be heated to a temperature in the range of about 20°C to about 95°C prior to addition of the initiator, if desired. Unreacted monomer can be eliminated by addition of more catalyst, as is well known in the emulsion polymerization art. The resulting polymer emulsion product can then be discharged from the reactor and packaged for storage or use. Optionally, the pH or other physical and chemical characteristics of the emulsion can be adjusted prior to discharge from the reactor. Typically, the product emulsion has a total solids (TS) content in the range of about 10 weight percent to about 60 weight percent. Typically, the total polymer content of the product emulsion is in the range of about 15 weight percent to about 50 weight percent, generally not more than about 40 weight percent. Here, as well as elsewhere in the specification and claims, individual numerical values (including carbon atom numerical values), or limits, can be combined to form additional non-disclosed and/or non-stated ranges.

Suitable surfactants for facilitating emulsion polymerizations include nonionic, anionic, amphoteric, cationic surfactants, and mixtures thereof. Most commonly, nonionic and anionic surfactants are utilized or mixtures thereof. The physical properties of the neutralized polymer (e.g., viscosity, spreadability, clarity, texture, and the like) can be varied by appropriate selection of the hydrophobic and hydrophilic properties of the emulsifying surfactant, as is well known in the art.

Nonionic surfactants suitable for facilitating emulsion polymerizations are well known in the polymer art, and include, without limitation, linear or branched alcohol ethoxylates, $C_8$ to $C_{12}$ alkylphenol alkoxylates, such as octylphenol ethoxylates, polyoxyethylene polyoxypropylene block copolymers, and the like. Other useful nonionic surfactants include $C_8$ to $C_{22}$ fatty acid esters of polyoxyethylene glycol, mono and diglycerides, sorbitan esters and ethoxylated sorbitan esters, $C_8$ to $C_{22}$ fatty acid glycol esters, block copolymers of ethylene oxide and propylene oxide having an HLB value of greater than about 12, ethoxylated octylphenols, and combinations thereof.

Suitable alkylphenol alkoxylate surfactants include, but are not limited to, an octylphenol sold under the trade name IGEPAL® CA-897 by Rhodia, Inc. In another embodiment, linear alcohol alkoxylates include polyethylene glycol ethers of cetearyl alcohol (a mixture of cetyl and stearyl alcohols) sold under the trade names PLURAFAC® C-17, PLURAFAC® A-38 and PLURAFAC® A-39 by BASF Corp. In still another embodiment, polyoxyethylene polyoxypropylene block copolymers include copolymers sold under the trade names PLURONIC® F127, and PLURONIC® L35 by BASF Corp.

Other suitable nonionic surfactants include, but are not limited to, Ethoxylated linear fatty alcohols such as DISPONIL® A 5060 (Cognis), Ethal LA-23 and Ethal LA-50 (Ethox Chemicals), branched alkyl ethoxylates such as GENAPOL® X 1005 (Clariant Corp.), secondary $C_{12}$ to $C_{14}$ alcohol ethoxylates such as TERGITOL® S15-30 and S15-40 (Dow Chemical Co.), ethoxylated octylphenol-based surfactants such as TRITON® X-305, X-405 and X-705 (Dow Chemical Co.), IGEPAL® CA 407, 887, and 897 (Rhodia, Inc.), ICONOL® OP 3070 and 4070 (BASF Corp.), SYNPERONIC® OP 30 and 40 (Uniqema), block copolymers of ethylene oxide and propylene oxide such as PLURONIC® L35 and F127 (BASF Corp.), and secondary $C_{11}$, alcohol ethoxylates such as EMULSOGEN® EPN 407 (Clariant Corp.). Numerous other suppliers are found in the trade literature.

Anionic surfactants suitable for facilitating emulsion polymerizations are well known in the polymer art, and include sodium lauryl sulfate, sodium dodecyl benzene sulfonate, sodium dioctyl sulfosuccinate, sodium di-sec-butyl naphthylene sulfonate, disodium dodecyl diphenyl ether sulfonate, and disodium n-octadecyl sulfosuccinate, and the like.

Suitable polymeric stabilizers (also known as protective colloids) for the emulsion polymerization process are

water-soluble polymers, including, for example, synthetic polymers, such as polyvinyl alcohol, partially hydrolyzed polyvinyl acetate, polyvinylpyrrolidone, polyacrylamide, polymethacrylamide, carboxylate-functional addition polymers, polyalkyl vinyl ethers and the like; water-soluble natural polymers, such as gelatin, pectins, alginates, casein, starch, and the like; and modified natural polymers, such as methylcellulose, hydroxypropylcellulose, carboxymethylcellulose, allyl modified hydroxyethylcellulose, and the like. In some cases, it can be of advantage to use mixtures of a synthetic and a natural protective colloid, for example, a mixture of polyvinyl alcohol and casein. Further suitable natural polymers are mixed ethers such as methylhydroxyethylcellulose and carboxymethylmethylcellulose. Polymeric stabilizers can be utilized in amounts up to about 10 weight percent based on the total emulsion weight, or up to about 7.5 weight percent, or up to about 5 weight percent, or up to about 2.5 weight percent, or up to about 2 weight percent based on the total emulsion weight. In another embodiment, when utilized, a polymeric stabilizer is included in an amount in the range of about 0.001 weight percent to about 10 weight percent, or from about 0.01 weight percent to about 7.5 weight percent, or from about 0.1 weight percent to about 5 weight percent, or from about 0.5 weight percent to about 2.5 weight percent, or even from about 1 weight percent to about 2 weight percent, based on the total emulsion weight. Here, as well as elsewhere in the specification and claims, individual numerical values (including carbon atom numerical values), or limits, can be combined to form additional non-disclosed and/or non-stated ranges.

The polymeric stabilizers which are used according to this invention are termed water-soluble when they are miscible in water in any proportion or have a solubility in 20°C water of at least about 0.1 weight percent and do not precipitate from these aqueous solutions on dilution with water at the foregoing temperature. The molecular weight of the water-soluble synthetic polymeric stabilizers is typically in the range of about 5,000 to about 2,000,000, or from about 25,000 to about 1,500,000 Daltons. The viscosity of aqueous solutions of the polymeric stabilizers is typically in the range of about 1 to about 10,000 mPa·s at a concentration of about 2 percent to about 10 weight percent and a temperature of about 20°C. Here, as well as elsewhere in the specification and claims, individual numerical values (including carbon atom numerical values), or limits, can be combined to form additional non-disclosed and/or non-stated ranges.

A useful polymeric stabilizer is an allyl modified hydroxyethylcellulose, such as TYLOSE® AM-HEC grades available from Clariant. The reactive allyl groups in the side chain increase the grafting power of the cellulose ether resulting in a stable emulsion. A TYLOSE® stabilizer is allyl modified hydroxyethylcellulose powder (particle size less than 180 μm) TYLOSE® AM H40 YP2 (AMHEC).

Exemplary free radical initiators include, but are not limited to, the water-soluble inorganic persulfate compounds, such as ammonium persulfate, potassium persulfate, and sodium persulfate; peroxides such as hydrogen peroxide, benzoyl peroxide, acetyl peroxide, and lauryl peroxide; organic hydroperoxides, such as cumene hydroperoxide and t-butyl hydroperoxide; organic peracids, such as peracetic acid; and oil soluble, free radical producing agents, such as 2,2'-azobisisobutyronitrile, and the like, and suitable mixtures of two or more thereof. Peroxides and peracids can optionally be activated with reducing agents, such as sodium bisulfite or ascorbic acid, sulfinic acid derivatives, e.g., Bruggolite® FF6 and FF7 (Brüggemann Chemical) transition metals, hydrazine, and the like. In one embodiment, suitable free-radical polymerization initiators include, but are not limited to, water soluble azo polymerization initiators, such as 2,2'-azobis(tert-alkyl) compounds having a water solubilizing substituent on the alkyl group. In another embodiment, azo polymerization catalysts include, but are not limited to, the VAZO® free-radical polymerization initiators, available from DuPont, such as VAZO® 44 (2,2'-azobis(2-(4,5-dihydroimidazolyl)propane), VAZO® 56 (2,2'-azobis(2-methylpropionamidine) dihydrochloride), and VAZO® 68 (4,4'-azobis(4-cyanovaleric acid)).

Optionally, other emulsion polymerization additives, which are well known in the emulsion polymerization art, such as solvents, buffering agents, chelating agents, inorganic electrolytes, chain terminators, and pH adjusting agents can be included in the polymerization system.

One suitable general emulsion polymerization procedure for the preparation of multi-purpose polymers is provided below.

In one embodiment, a monomer emulsion is prepared in a reactor equipped with a nitrogen inlet and an agitator by combining a desired amount of each monomer in a quantity of water containing an emulsifying amount of a nonionic surfactant, or a mixture of a nonionic surfactant and an anionic surfactant, under a nitrogen atmosphere, and with mixing agitation. The degree of agitation required to form an emulsion from a monomer mixture of the type described above is well known to those of skill in the art. The so-formed emulsion is substantially deoxygenated by any suitable method known in the art, such as by sparging with nitrogen, and then a free radical initiator is added to the emulsion, with continuous mixing agitation, to initiate polymerization. The temperature of the emulsion can be adjusted, before or after addition of the initiator, to a temperature in the range of about 20°C to about 60°C, if desired. After the addition of initiator, the temperature of the polymerization reaction mixture is typically adjusted to a temperature in the range of about 60°C to 95°C and held at such temperature for a time sufficient to complete the polymerization, typically in the range of about 3 to about 14 hours. Optionally, unreacted residual monomers can be destroyed or further polymerized by the addition of various redox reagents or catalysts. The resulting polymer emulsion can then be cooled and discharged from the reactor and collected.

One skilled in the polymer art will recognize that the amounts of each monomer component can be adjusted

to obtain polymers having any desired ratio of monomer components. Varying proportions of water can also be utilized, as desired. Water miscible solvents, such as alcohols, and other polymerization additives, as described above, may also be included in the reaction mixture. Suitable alcohols include, but are not limited to, glycols such as ethylene glycol, propylene glycol, butylene glycol, hexylene glycol, glycerol, and the like.

The product polymer emulsions can be prepared to, in one embodiment, contain about 1 percent to about 60 percent total polymer solids, or from about 10 percent to about 40 percent total polymer solids, or even from about 15 percent to about 25 percent total polymer solids based on the weight of the polymer. Here, as well as elsewhere in the specification and claims, individual numerical values (including carbon atom numerical values), or limits, can be combined to form additional non-disclosed and/or non-stated ranges. Prior to any neutralization, the polymer emulsions, as produced, typically have a pH in the range of about 7 or greater, a Brookfield viscosity of not more than about 100 mPa·s at ambient room temperature (spindle #2, 20 rpm).

Optionally, the produced multi-purpose polymer emulsions can be further processed by adjusting the pH to a value in the range of about 1 to not more than about 7, if an acidic pH is desired, with acidic materials, such as organic acids, mineral acids, and the like. The multi-purpose polymer emulsions typically swell to form smooth, viscous solutions that are flowable and sprayable, or gels at neutral to acidic pH, and the polymers are generally substantially stable at such pH values. The multi-purpose polymer emulsions can be diluted with water or solvent, or concentrated by evaporating a portion of the water. Alternatively, the obtained multi-purpose polymer emulsion can be substantially dried to a powder or crystalline form by utilizing equipment well known in the art, such as, for example, a spray drier, a drum drier, a freeze drier, and the like.

The inventive multi-purpose polymers can be prepared by, for example, emulsion polymerization, solution polymerization, precipitation polymerization, bulk polymerization, dispersion polymerization and utilized by incorporating various known additives and conventional adjuvants, and solvents other than water, as needed. When emulsion polymerization selected as the polymerization technique, the polymerization reaction can be run via batch and semi-batch techniques.

A multi-purpose polymer at a weight concentration of about 2 percent in deionized water, in its neutralized or acidic form at a pH in the range of about 1 to about 7, can provide a Brookfield viscosity ranging from about 300 mPa·s to about 100,000 mPa·s or more (Brookfield RVT, 20 rpm, at about 25°C ambient room temperature).

The molecular weight (weight average) of the multi-purpose polymers range from about 5,000 daltons to about 10,000,000 daltons in one aspect, from about 10,000 daltons to about 5,000,00 daltons in another aspect, and from about 50,000 daltons to about 3,000,000 daltons in a further aspect of the invention, as measured by GPC using a polystyrene standard.

The multi-purpose polymers can be utilized in a variety of products for personal care, health care, home care, institutional and industrial (collectively "I&I") care, and in a variety of products for medical and industrial applications. The inventive multi-purpose polymers can be employed as emulsifiers, stabilizers, suspending agents, deposition aids for enhancing the efficacy, deposition or delivery of chemically and physiologically active ingredients and cosmetic materials, film formers, thickeners, rheology modifiers, hair fixatives, conditioning fixatives, conditioners, moisturizers, spreading aids, carriers, and as an agent for improving the psychosensory, and aesthetic properties of a formulation in which they are included. Additionally, the cationic character of the multi-purpose polymers of the invention makes these polymers useful as antistats, and, under certain conditions, may also provide biocidal, bacteriostatic, preservative, and anti-microbial activity.

**Personal Care and Health Care Applications:**

The multi-purpose polymers of the invention are suitable for the preparation of personal care (cosmetics, toiletries, cosmeceuticals), topical health care products and medical products, including without limitation, hair care products, such as shampoos (including combination shampoos, such as "two-in-one" conditioning shampoos); post-shampoo rinses; setting and style maintenance agents including setting aids, such as gels and sprays, grooming aids, such as pomades, conditioners, perms, relaxers, hair smoothing products, and the like; skin care products (facial, body, hands, scalp and feet), such as creams, lotions, conditioners, deodorants, antiperspirants and cleansing products; anti-acne products; chemical peeling products, anti-aging products ((exfoliant, keratolytic, anti-cellulite, anti-wrinkle, (reducing wrinkles and fine lines and/or pigment marks and/or acne,marks and/or to unblock the pores of the skin, and the like, as disclosed in U.S. Published Application No. 2009/0029928)), skin protectants such as sunscreens, sunblock, barrier creams, oils, silicones, and the like; skin color products (whiteners, lighteners, sunless tanning accelerators, and the like); hair colorants (hair dyes, hair color rinses, highlighters, bleaches and the like); pigmented skin colorants (face and body make-ups, foundation creams, mascara, rouge, lip products, and the like); bath and shower products (body cleansers, body wash, shower gel, liquid soap, soap bars, syndet bars, conditioning liquid bath oil, bubble bath, bath powders, therapeutic cleaners, acne control products, facial cleansers and the like); nail care products (polishes, polish removers, strengtheners, lengtheners, hardeners, cuticle removers, softeners, and the like); and any aqueous acidic to

substantially neutral composition to which an effective amount of multi-purpose polymer can be incorporated for achieving a beneficial or desirable, physical or chemical, effect therein during storage and/or usage.

Toiletries and health and beauty aids, commonly referred to as HBAs, containing a multi-purpose polymer, can include, without limitation, hair-removal products (shaving creams and lotions, depilatories, after-shave skin conditioners, and the like); deodorants and antiperspirants; oral care products (mouth, teeth and gums), such as mouthwash, dentifrice, such as toothpaste, tooth powder, tooth polishes, tooth whiteners, breath fresheners, denture adhesives, and the like; facial and body hair bleach; and the like. Other health and beauty aids that can contain multi-purpose polymers, include, without limitation, sunless tanning applications containing artificial tanning accelerators, such as dihydroxyacetone (DHA), tyrosine, tyrosine esters, and the like; skin de-pigmenting, whitening, and lightening formulations containing such active ingredients as kojic acid, hydroquinone, arbutin, fruital, vegetal or plant extracts, (lemon peel extract, chamomile, green tea, paper mulberry extract, Trametes extract (the genus Trametes includes Trametes versicolor, Trametes pubescens, Trametes hirsuta, Trametes ochracea, Trametes elegans, Trametes colliculosa, Trametes gbbosa, Trametes palustris, Trametes villosa, Trametes suaveolens, Trametes cervina, Trametes cingulata, and the like. In one aspect the Trametes extract is Trametes versicolor), ascorbyl acid derivatives (ascorbyl palmitate, ascorbyl stearate, magnesium ascorbyl phosphate, and the like); foot care products, such as keratolytic corn and callous removers, foot soaks, foot powders (medicated, such as antifungal athlete's foot powder, ointments, sprays, and the like, and antiperspirant powders, or non-medicated moisture absorbent powder), liquid foot sprays (non-medicated, such as cooling, and deodorant sprays, and medicated antifungal sprays, antiperspirant sprays, and the like), and foot and toenail conditioners (lotions and creams, nail softeners, and the like).

Topical health and beauty aids that can include multi-purpose polymers (e.g., as spreading aids, deposition aids and film formers) include, without being limited thereto, skin protective spray, cream, lotion, gel, flexible porous dissolvable solid structures such as disclosed in U.S. Published Patent Application No. 2009/0232873, stick and powder products, such as insect repellants, itch relief, antiseptics, disinfectants, sun blocks, sun screens, skin tightening and toning milks and lotions, wart removal compositions, and the like.

Other health care products in which multi-purpose polymers can be included are medical products, such as topical and non-topical pharmaceuticals, and devices. In the formulation of pharmaceuticals, a multi-purpose polymer can be employed as a thickener and/or lubricant in such products as creams, pomades, gels, pastes, ointments, tablets, gel capsules, purgative fluids (enemas, emetics, colonics, and the like), suppositories, anti-fungal foams, eye products (ophthalmic products, such as eye-drops, artificial tears, glaucoma drug delivery drops, contact lens cleaner, and the like), ear products (wax softeners, wax removers, otitis drug delivery drops, and the like), nasal products (drops, ointments, sprays, and the like), and wound care (liquid bandages, wound dressings, antibiotic creams, ointments, and the like), without limitation thereto.

The film-forming and acid-swellable character of the multi-purpose polymer makes the multi-purpose polymer particularly suitable as a vehicle for topical medical compositions for promoting and enhancing the transdermal delivery of active ingredients to or through the skin, for enhancing the efficacy of anti-acne agents formulations and topical analgesics, and for controlling release of drugs, such as antacids from tablets, or syrups, at low pH, such as in the stomach; controlling drug release from tablets, lozenges, chewables, and the like in the mildly acidic environment of the mouth; or from suppositories, ointments, creams, and the like in the mildly acidic environment of the vagina; to promote deposition of dandruff control agents from shampoos, salves, and the like; to enhance the deposition of colorants on skin from pigmented cosmetics (make-ups, lipsticks, rouges, and the like) and on hair from hair dyes, and the like.

The polymers can be employed, without being limited thereto, as a lubricant coating for medical devices, such as soft tissue implants, surgical gloves, catheters, cannulae, and the like, as removable protective film coatings for medical instruments, wound dressings, and the like, as a muco-adhesive, especially in the acid environment of the stomach, as a carrier and thickener in formulated products for medical applications, such as disinfectant hand creams, antiviral products (for anionic viruses), antibiotic ointments, sprays and creams, non-drip, sprayable disinfectant in hospitals, hard surface antimicrobial finish applied during routine maintenance, and the like.

**Home Care and Institutional and Industrial Applications:**

The polymers can be used in home care, and I&I applications, for example, as a rheology modifier, fabric conditioning agent, antistatic agent, especially to improve formulation efficiency through "cling-on-surface" or improving efficacy of disinfectants, and biocidal formulations, and to synergistically improve fabric softening efficacy in combination with traditional fabric softeners. Typical household and I&I products that may contain polymers of the invention, include, without being limited thereto, laundry and fabric care products, such as detergents, fabric softeners (liquids or sheets), ironing sprays, dry cleaning aids, anti-wrinkle sprays, spot removers and the like; hard surface cleansers for the kitchen and bathroom and utilities and appliances employed or located therein, such as toilet bowl gels, tub and shower cleaners, hard water deposit removers, floor and tile cleansers, wall cleansers, floor and chrome fixture polishes, alkali-strippable vinyl floor cleaners, marble and ceramic cleaners, air freshener gels, liquid cleansers for dishes, and the like; disinfectant

cleaners, such as toilet bowl and bidet cleaners, disinfectant hand soaps, room deodorizers, and the like.

**Institutional and Industrial Applications:**

The polymers can be utilized as rheology modifiers, dispersants, stabilizers, promoters, or antimicrobials, and the like, in institutional and industrial product applications, such as, without being limited thereto, textiles (processing, finishing, printing, and dyeing aids, protective washable surface coatings, manufacture of synthetic leather by saturation of non-woven fabrics, and the like, manufacturing of woven fabrics, non-woven fabrics, natural and synthetic fibers and the like); water treatment flocculents (waste water, cooling water, potable water purification, and the like); chemical spill containments (acid-spill absorbent, and the like); leather and hide processing (processing aids, finishing, coating, embossing, and the like); pulp and papermaking (flocculents, surface coatings, such as pigmented coatings, antistatic coatings, and the like, pulp binders, surface sizings, dry and wet strength enhancers, manufacture of wet-laid felts, and the like); printing (inks, anti-wicking ink-jet printer inks, thickeners for ink formulations containing cationic dyes for printing acrylic fabrics, and the like); paints (pigment and grinding additive, crosslinking agent for epoxy latex emulsions, particulate-suspending aid for clays, pigments, and the like); industrial plant effluent treatment (flocculents for phenolics in paper mill effluent, and the like); metal working (acid etch cleaners, low pH metal coatings, pickling agents in cold rolled steel processing, and the like); adhesives (clear adhesives, adhesion promoters for metal, plastic, wood, and the like, non-woven floc adhesive tie coatings, bonding, and the like); wood preservation; and industrial construction products for buildings and roads (cement plasticizers, asphalt emulsion stabilizers at low pH, acid etch for cement, consistency modifiers of concrete, mortar, putty, and the like). The polymers are particularly useful as thickeners for rust removers, acid truck cleaners, scale removers, and the like.

As discussed above, the inventive multi-purpose polymers can be employed as emulsifiers, stabilizers, suspending agents, deposition aids for enhancing the efficacy, deposition or delivery of chemically and physiologically active ingredients and cosmetic materials, film formers, rheology modifiers, hair fixatives, conditioning fixatives, conditioners, moisturizers, spreading aids, carriers, and as an agent for improving the psychosensory, and aesthetic properties of a formulation for an application in which they are included.

The polymers are particularly useful as emulsification aids for water-insoluble (hydrophobic) oily materials such as natural and synthetic oils, fats, and waxes, including, for example, vegetable oils, animal oils and fats, paraffin oils and waxes, silicone oils and waxes; and the like. Many oily materials are used as solvents, carriers, emollients, or conditioning agents, for example, in hair and skin care products.

The polymers are surprisingly useful stabilizers and/or deposition aids for water insoluble materials such as silicone fluids, rigid silicones, amino silicones, silicone emulsions and dimethicone copolyols. Silicone fluids which are commonly used in shampoo products, such as the so-called "two-in-one" combination cleansing/conditioning shampoos. The polymers are surprisingly effective for stabilizing and depositing two-in-one type shampoo formulations containing suitable silicone products of low, medium and high molecular weight silicone polyether fluids (193C, 5330) and resins; low, medium, high viscosity dimethyl silicone fluids/gums/resins or in the form of non-ionic small and large particle size emulsions (MEM 1664, MEM 1310, 5-7137, 2-1352, MEM 1784, MEM 1310, MEM 1491, 5-7137, and MEM 2220), and anionic emulsions (MEM 1784); aminosilicone fluids with low and high amine content (8500, 2-8566, AP-8087); amino glycol copolymer; amino phenyl resin; low and high viscosity cationic emulsions (949, 2-8194), nonionic microemulsions; silicone quaternary microemulsions (5-7113); phenyl silicone (556) fluids, silicone wax (AMS C-30), silicone elastomer blend (9040) and the like, and mixtures thereof.

The multi-purpose polymers are particularly useful as suspending agents for particulates, such as mica, pearlizing agents, beads, and the like, making them suitable for dermal products containing particulates, micro-abrasives, and abrasives, such as bath and shower gels, masks and skin cleansers containing exfoliative scrub agents. Numerous cosmetically useful particulate exfoliating agents are known in the art, and the selection and amount is determined by the exfoliating effect desired from the use of the composition, as recognized by those skilled in the cosmetic arts.

If desired, the clarity and/or appearance of the personal care, home care, health care, and institutional and industrial care compositions of the invention can be adjusted. The clarity of the compositions may vary from substantially transparent with little visual haze where insoluble component additives such as beads, air bubbles, pearlizing agents, are clearly visible to visually opaque. Visually distinct, multiple phase compositions where one phase is clear and another phase is opaque are also envisioned. In one embodiment of the invention, a pattern comprising phases that are visually distinct from each other may be formed by mixing clear and opaque components. The visual distinction between each phase can be in color, texture, density, and the type of insoluble component or benefit agent contained therein. The specific pattern can be chosen from a wide variety of patterns, including, but not limited to striping, marbling, geometrics, spirals, and combinations thereof. Compositions of this invention demonstrate excellent stability with time in suspending insoluble components and/or benefit agents and stabilizing the visually distinct phases. Multiple-phase compositions are disclosed in U.S. Published Patent Application Nos. 2006/0079417, 2006/0079418, 2006/0079419, 2006/0079420, 2006/0079421, 2006/0079422, 2007/0009463, 2007/0072781, 2007/0280976, and 2008/0317698 to the Proctor and

Gamble Company. The multi-purpose polymers are suitable for use in the multi-phase compositions disclosed therein.

Suitable exfoliating agents include, but are not limited to, biological abrasives, inorganic abrasives, synthetic polymers, and the like, and mixtures thereof. Biological abrasives include, without limitation, shell, seed, and kernel or stone granules or powders, obtained from nuts, such as from walnut (Juglans regia) shells, almonds, pecans, and the like; fruital sources, such as apricots, avocados, coconuts, olives, peaches, and the like; vegetal sources, such as corn cob, oat bran, rice, rose hip seed, jojoba (wax, seed powder), microcrystalline cellulose, ground loofa, ground seaweed, and the like; animal sources, such as oyster shell, silk, microcrystalline collagen, and the like. Inorganic abrasives include, without limitation, stannic oxide, talc, silica (hydrated, colloidal and the like), kaolin, precipitated chalk, salts (sodium chloride, dead sea salt, and the like), ground pumice, and the like. Synthetic polymers include, without limitation, microcrystalline polyamides (nylons), microcrystalline polyesters (polycarbonates), polymethyl (meth)acrylate microbeads (PMMA microbeads) and the like. The polymers are also useful for suspending gaseous bubbles in a liquid medium. 1.

The multi-purpose polymers are useful as thickeners, deposition aids, and film-formers in a variety of dermatological, pharmaceutical, and cosmeceutical compositions employed for topically ameliorating skin and scalp conditions caused by perspiration, inflammation, swelling, drying, dandruff, photo-damage, aging, acne, and the like, containing pharmaceutical and cosmeceutical active ingredients, conditioners, surfactants, moisturizers, antioxidants, exfoliants (described above), keratolytic agents, botanicals, vitamins, and the like, and combinations thereof.

Suitable examples of pharmaceutical and cosmeceutical active ingredients include, but are not limited to, caffeine, vitamin C, vitamin D, vitamin E, pantothenic acid (vitamin B5), anti-stretch mark compounds, astringents (e.g., alum, oatmeal, yarrow, witch hazel, bayberry, and isopropyl alcohol), draining compounds, hair growth compounds (e.g., minoxidil), skin and hair nourishing compounds, skin and hair protecting compounds, self-tanning compounds (e.g., mono- or polycarbonyl compounds such as, for example, isatin, alloxan, ninhydrin, glyceraldehyde, mesotartaric aldehyde, glutaraldehyde, erythrulose, tyrosine, tyrosine esters, and dihydroxyacetone), sunscreens (e.g., ethylhexyl methoxy cinnamate, octinoxate, octisalate, oxybenzone), skin lighteners (e.g., kojic acid, hydroquinone, arbutin, fruital, vegetal or plant extracts, such as lemon peel extract, chamomile, green tea, paper mulberry extract, and the like, ascorbyl acid derivatives, such as ascorbyl palmitate, ascorbyl stearate, magnesium ascorbyl phosphate, and the like), lip plumping compounds, anti-aging, anti-cellulite, and anti-acne compounds (e.g., acidic agents such as alpha-hydroxy acids (AHAs), beta-hydroxy acids (BHAs), alpha amino-acids, alpha-keto acids (AKAs), acetic acid, azelaic acid, urea glycolysates, e.g., UGL™ Complex available from Barnet Products Corporation, and mixtures thereof), anti-dandruff compounds (e.g., zinc pyrithione, zinc omadine, imidazole and triazole compounds like climbazole, clotriamzole, miconazole nitrate, itraconazole, flucoazole selenium sulfide, piroctone olamine, salicylic acid, acetyl salicylic acid, magnesium salicylate, sodium salicylate, coal tar, lithium gluconate, lithium succinate, ciclopirox,, sulfacetamide, terbinafine hydrochloride (synthetic allyl amine) and mixtures thereof) anti-inflammatory compounds (e.g., aspirin, ibuprofen, and naproxen), analgesics (e.g., acetaminophen), antioxidant compounds, antiperspirant compounds, deodorant compounds (e.g., 2-amino-2-methyl-1-propanol (AMP), ammonium phenolsulfonate; benzalkonium chloride; benzethonium chloride, bromochlorophene, cetyltrimethylammonium bromide, cetyl pyridinium chloride, chlorophyllin-copper complex, chlorothymol, chloroxylenol, cloflucarban, dequalinium chloride, dichlorophene, dichloro-m-xylenol, disodium dihydroxyethyl sulfosuccinylundecylenate, domiphen bromide, hexachlorophene, lauryl pyridinium chloride, methylbenzethonium chloride, phenol, sodium bicarbonate, sodium phenolsulfonate, triclocarban, triclosan, zinc phenolsulfonate, zinc ricinoleate, and mixtures thereof), copper derived nano deodorant compunds, hair fixative polymers (e.g., natural and synthetic polymers such as, for example, polyacrylates, polyvinyls, polyesters, polyurethanes, polyamides, modified cellulose, starches, and mixtures thereof), hair and skin conditioners (e.g., synthetic oils, natural oils, such as vegetable, plant and animal oils, mineral oils, natural and synthetic waxes, cationic polymers, monomeric and polymeric quaternized ammonium salt compounds, silicones such as silicone oils, resins and gums, proteins, hydrolyzed proteins, fatty acids, fatty amines; and mixtures thereof); and mixtures thereof.

In one cosmeceutical aspect, a multi-purpose polymer can be employed as a thickener and/or a deposition aid for active skin treatment lotions and creams containing, as active ingredients, acidic anti-aging, anti-cellulite, and anti-acne agents, hydroxy carboxylic acids, such as alpha-hydroxy acid (AHA), beta-hydroxy acid (BHA), alpha-amino acid, alpha-keto acids (AKAs), and mixtures thereof. Suitable AHAs include, but are not limited to, lactic acid, glycolic acid, fruit acids, such as malic acid, citric acid, tartaric acid, extracts of natural compounds containing AHA, such as apple extract, apricot extract, and the like, honey extract, 2-hydroxyoctanoic acid, glyceric acid (dihydroxypropionic acid), tartronic acid (hydroxypropanedioic acid), gluconic acid, mandelic acid, benzilic acid, alpha-lopioc acid, AHA salts and derivatives, such as arginine glycolate, ammonium lactate, sodium lactate, alpha-hydroxybutyric acid, alpha-hydroxyisobutyric acid, alpha-hydroxyisocaproic acid, alpha-hydroxyisovaleric acid, atrolactic acid, and the like. Suitable BHAs include, but are not limited to, 3-hydroxy propanoic acid, beta-hydroxybutyric acid, beta-phenyl lactic acid, beta-phenylpyruvic acid, salicylic acid, and the like. Suitable alpha-amino acids include, without being limited to, alpha-amino dicarboxylic acids, such as aspartic acid, glutamic acid, and the like. Representative alpha-keto acids are pyruvic acid, acetopyruvic acid, and the like. Other active acidic agents include retinoic acid and its derivatives, halocarboxylic acids (e.g., trichloroacetic acid), acidic antioxidants (e.g., vitamin C), mineral acids, phytic acid, lysophosphatidic acid, salicylic acid, derivatives of

salicylic acid (e.g., 5-octanoylsalicylic acid), and the like. Typically the active acidic ingredient has a pH in the range of about 0.5 to about 5. When a multi-purpose polymer is incorporated into the foregoing acidic product embodiments, the active acid ingredient can serve as both the active skin treatment agent and acid swelling agent for the multi-purpose polymer to achieve the desired viscosity.

A discussion of the use and formulation of active skin treatment compositions is in Cosmetics & Toiletries®, C&T Ingredient Resource Series, AHAs & Cellulite Products How They Work, published 1995, and Cosmeceuticals, published 1998, both available from Allured Publishing Corporation, incorporated herein by reference. Compositions containing alpha-amino acids acidified with ascorbic acid are described in United States Patent No. 6,197,317 and United States Patent Application Publication No. 2009/0029928, and a commercial cosmeceutical preparation utilizing these acids in an anti-aging, skin care regimen is sold under the tradename, AFAs, by exCel Cosmeceuticals (Bloomfield Hills, Michigan). The term "AFA," as described in the supplier's trade literature, was coined by the developer to describe the amino acid/vitamin C combination as Amino Fruit Acids and as the acronym for "Amino acid Filaggrin based Antioxidants." In addition to ingredients discussed above, other ingredients commonly used for anti-acne products, facial and body hair bleaches, and anti-septic products include oxidizing agents, such as hydrogen peroxide, benzoyl peroxide, and water-soluble inorganic persulfate compounds such as ammonium persulfate, potassium persulfate, and sodium persulfate.

Suitable antiperspirant agents that can be utilized according to the foregoing antiperspirant embodiment include conventional antiperspirant metal salts and complexes of metal salts. In one embodiment of the invention, the metal salts and metal salt complexes utilized as the antiperspirant agents are acidic and are based on aluminum and zirconium and combinations thereof. These salts include, but are not limited to, aluminum halides, aluminum hydroxyhalides, aluminum sulfate, zirconium (zirconyl) oxyhalides, zirconium (zirconyl)hydroxyhalides, and mixtures or complexes thereof. Complexes of aluminum and zirconium salts include, but are not limited to, aluminum and zirconium salt complexes with amino acids, such as, for example, glycine or complexes with a glycol, such as, for example, propylene glycol (PG) or polyethylene glycol (PEG). Exemplary antiperspirant agents include, but are not limited to, aluminum chloride, aluminum chlorohydrate, aluminum dichlorohydrate, aluminum sesquichlorohydrate, zirconyl hydroxychloride, aluminum chlorohydrex PEG (aluminum chlorohydrex polyethylene glycol), aluminum chlorohydrex PG (aluminum chlorohydrex propylene glycol), aluminum dichlorohydrex PEG (aluminum dichlorohydrex polyethylene glycol), aluminum dichlorohydrex PG (aluminum dichlorohydrex propylene glycol), aluminum sesquichlorohydrex PEG (aluminum sesquichlorohydrex polyethylene glycol), aluminum sesquichlorohydrex PG (aluminum sesquichlorohydrex propylene glycol), aluminum zirconium trichlorohyrate, aluminum zirconium tetrachlorohyrate, aluminum zirconium pentachlorohyrate, aluminum zirconium octachlorohyrate, aluminum zirconium chlorohydrex GLY (aluminum zirconium chlorohydrex glycine), aluminum zirconium trichlorohydrex GLY (aluminum zirconium trichlorohydrex glycine), aluminum zirconium tetrachlorohyrex GLY (aluminum zirconium tetrachlorohyrex glycine), aluminum zirconium pentachlorohyrex GLY (aluminum zirconium pentachlorohyrex glycine), and aluminum zirconium octachlorohyrex GLY (aluminum zirconium octachlorohyrex glycine). Other antiperspirant agents include, but are not limited to, ferric chloride and zirconium powder. Mixtures of any of the foregoing antiperspirant agents are also suitable for use in the present invention.

The amount of the antiperspirant agent incorporated into the antiperspirant compositions of the present invention is an amount that is sufficient to reduce the flow of perspiration from the location to which the antiperspirant product is applied, for example to the auxiliary area of the human body, while providing a suitably low pH to neutralize the cationic hydrophobically modified polymer to attain a desired viscosity. If the desired amount of antiperspirant agent loading is reached before the cationic hydrophobically modified polymer is sufficiently neutralized to achieve the desired viscosity profile, auxiliary acidification agents can be added to effect the desired viscosity profile.

Generally, the level of antiperspirant agent utilized in the compositions of the present invention range from about 0.5 weight percent to about 35 weight percent based on the total weight of the antiperspirant composition. In another embodiment, the amount of antiperspirant agent in the composition can range from about 1 weight percent to about 25 weight percent, or from about 5 weight percent to about 20 weight percent, or even from about 10 weight percent to about 15 weight percent, based on the total weight of the composition. The foregoing weight percentages are calculated on an anhydrous metal salt basis exclusive of the complexing agent (e.g., glycine or glycol). Here, as well as elsewhere in the specification and claims, individual numerical values (including carbon atom numerical values), or limits, can be combined to form additional non-disclosed and/or non-stated ranges. Unless otherwise stated herein, the active ingredients can be present in effective amounts to accomplish their function, and are generally included individually at a level of from 0 wt. % to 35 wt %, based on the weight of the total composition in which they are employed.

In one aspect the polymers can be used as a thickener, film former, deposition aid, or as a dye or pigment suspending agent for promoting deposition of colorants on hair and skin. Colorants for hair can be temporary, semi-permanent or permanent hair dyes or color restorers that pigment the hair gradually. Temporary and semi-permanent hair dyes typically are rinses, gels, sprays, shampoos, sticks, and the like, and hair color restorers are typically in the form of hair dressings or emulsions. Permanent hair dyes, and longer-lasting semi-permanent hair dyes, are generally two-part products, one part containing the oxidative dye intermediates and dye couplers, and the other part containing stabilized

oxidizing agent, usually hydrogen peroxide at about pH 3 to 4, and are mixed together immediately before use. It is known that such two-part hair dyeing products are formulated with combinations of surfactant ingredients, usually nonionic surfactants or anionic surfactants, to thicken when the dye mixture is prepared. In addition to the foregoing literature, a general discussion of hair dyeing chemistry and compositions is in Brown et al, SCC Monograph, Permanent Hair Dyes, Society of Cosmetic Chemists (1996), incorporated herein by reference. The polymers may be incorporated in one or both of the two-parts of such hair dyeing systems, either as the thickener for the acidic stabilized oxidizing portion or in the non-oxidizing portion to be thickened upon mixing with the acidic portion.

In another hair care embodiment, the polymers can be utilized in an amount effective to provide to the hair care composition a property, such as a hair fixative property, a hair conditioning property, a viscid property (thickening, rheology modifying), or a combination thereof. Optionally, the hair care composition can include one or more auxiliary film-forming agent, auxiliary hair-fixative agent, auxiliary hair conditioning agent, auxiliary rheology modifying agent, or a mixture thereof.

The term "fixative" as applied to polymers encompasses the properties of film-formation, adhesion, or coating deposited on a surface on which the polymer is applied. The terms "hair styling and hair fixative" as commonly understood in the hair care arts, and as used herein, refer collectively to hair setting agents that are hair fixatives and film formers and which are topically applied to the hair to actively contribute to the ease of styling and/or holding of a hair set, and to maintain the restylability of the hair set. Hence, "hair setting compositions" include hair styling, hair fixative, and hair grooming products that conventionally are applied to the hair (wet or dry) in the form of gels, rinses, emulsions (oil-in-water, water-in-oil or multiphase), such as lotions and creams, pomades, sprays (pressurized or non-pressurized), spritzes, foams, such as mousses, shampoos, solids, such as sticks, semisolids and the like, or are applied from a hair setting aid having the hair setting composition impregnated therein or coated thereon, to leave the hair setting agent in contact on the hair for some period until removed, as by washing.

The term "hair setting composition" encompasses products comprising at least one polymer as a hair setting agent, which is applied to the hair (wet or dry) before, during or after configuring the hair into the shape (curly or straight) desired, without limitation as to product form.

The term "conditioning agents," and grammatical variations thereof, as it relates to compositions for skin care and hair care includes cosmetically and pharmaceutically useful materials that are humectants, moisturizers, and emollients. It is recognized that some conditioning agents can serve more than one function in a composition, such as emulsifying agents, lubricants, and solvents.

The polymers are surprisingly useful in hair setting and hair styling compositions as the sole film-forming, rheology modifying, conditioning, and fixative agent. The polymers are also useful in combination with commercially available auxiliary hair fixative polymers, such as nonionic, cationic, and amphoteric hair setting polymers, cationic conditioning polymers, and combinations thereof. It is surprisingly found that unexpectedly increased viscosity and hair setting efficacy properties are produced by appropriate combinations of a polymer with an auxiliary conventional hair fixative and/or hair conditioning polymer. Conventional polymeric hair fixative and hair styling polymers, well known in the art, include natural gums and resins and neutral or anionic polymers of synthetic origin. Listings of commercially available hair fixative and conditioning fixative polymers can be readily found in the INCI Dictionary, in supplier websites, and in the trade literature. See, for example, the Polymer Encyclopedia published in Cosmetics & Toiletries®, 117(12), December 2002 (Allured Publishing Corporation, Carol Stream, III.), the relevant disclosures of which are incorporated herein by reference.

Suitable commercially available auxiliary fixatives include nonionic, cationic, anionic, and amphoteric polymers, as well as combinations thereof and include without limitation, polyvinylpyrrolidone (PVP), polyvinylpyrrolidone/vinylacetate copolymer (PVP/VA), and the like. Commercially available cationic fixative polymers include, without limitation thereto, polymers having the INCI name, polyquaternium, such as polyquaternium-4, a diallyldimonium chloride/hydroxyethylcellulose copolymer (such as CELQUAT® H-100, National Starch); polyquaternium-11, a quaternized vinyl pyrrolidone/dimethylaminoethyl methacrylate copolymer (such as GAFQUAT® 734, 755, 755N, ISP); polyquaternium-16, a quaternized vinyl pyrrolidone/vinylimidazolium chloride copolymer (such as LUVIQUAT® FC-370, BASF); polyquaternium-28, a vinylpyrrolidone/methacryl-amidopropyltrimethylammonium chloride copolymer (such as GAFQUAT® HS-100, ISP); polyquaternium-46, a quaternized vinylcaprolactam/vinylpyrrolidone/methylvinyl-imidazolium methosulfate copolymer; polyquaternium-55, a quaternized vinyl-pyrrolidone/dimethylaminopropylmethylacrylamide/lauryldimethylpropylmethacryl amido-ammonium chloride copolymer (such as STYLEZE™ W, ISP), and the like; and amino-substituted polymers which are cationic under acidic pH conditions, such as vinylcaprolactam/PVP/dimethylaminoethylmethacrylate copolymer (such as GAFFIX® VC-713, ISP); PVP/dimethylaminoethylmethacrylate copolymer (such as Copolymer 845, ISP), PVP/DMAPA acrylates copolymer (such as STYLEZE™ CC-10, ISP), the pyrrolidone carboxylic acid salt of chitosan, having the INCI name, Chitosan PCA (such as KYTAMER® PC, Amerchol), and the like.

Additional auxiliary fixatives can be selected from one or more of the following polymers. Suitable commercially available nonionic polymers (i.e., neutral) used as hair styling or fixative polymers include, without limitation thereto, polyvinylpyrrolidone (PVP), polyvinylpyrrolidone/vinylacetate copolymer

(PVP/VA), and the like. Commercially available cationic fixative polymers include, without limitation thereto, polymers having the INCI name, polyquaternium, such as polyquaternium-4, a diallyldimonium chloride/hydroxyethylcellulose copolymer (such as CELQUAT® H-100, National Starch); polyquaternium-11, a quaternized vinyl pyrrolidone/dimethylaminoethyl methacrylate copolymer (such as GAFQUAT® 734, 755, 755N, ISP); polyquaternium-16, a quaternized vinyl pyrrolidone/vinylimidazolium chloride copolymer (such as LUVIQUAT® FC-370, BASF); polyquaternium-28, a vinylpyrrolidone/methacryl-amidopropyltrimethylammonium chloride copolymer (such as GAFQUAT® HS-100, ISP); polyquaternium-46, a quaternized vinylcaprolactam/vinylpyrrolidone/methylvinyl-imidazolium methosulfate copolymer; polyquaternium-55, a quaternized vinylpyrrolidone/dimethylaminopropylmethylacrylamide/lauryldimethylpropylmethacryl amido-ammonium chloride copolymer (such as STYLEZE™ W, ISP), and the like; and amino-substituted polymers which are cationic under acidic pH conditions, such as vinylcaprolactam/PVP/dimethylaminoethylmethacrylate copolymer (such as GAFFIX® VC-713, ISP); PVP/dimethylaminoethylmethacrylate copolymer (such as Copolymer 845, ISP), PVP/DMAPA acrylates copolymer (such as STYLEZE™ CC-10, ISP), the pyrrolidone carboxylic acid salt of chitosan, having the INCI name, Chitosan PCA (such as KYTAMER® PC, Amerchol), and the like. Suitable amphoteric fixative polymers include, without limitation thereto, octylacryamide/acrylates/butylaminoethylmethacrylate copolymer (such as the AMPHOMER® polymers, National Starch), acrylates/lauryl acrylate/stearyl acrylate/ethylamine oxide methacrylate copolymers (such as the DIAFORMER® polymers, Clariant Corp.), and the like. acrylamide/acrylates/butylaminoethylmethacrylate copolymer (such as the AMPHOMER® polymers, National Starch), acrylates/lauryl acrylate/stearyl acrylate/ethylamine oxide methacrylate copolymers (such as the DIAFORMER® polymers, Clariant Corp.), and the like.

Additional fixative polymers that can be utilized with the inventive polymers of the invention include without limitation one or more of the following polymers: polyoxyalkoxylated vinyl acetate/crotonic acid copolymers, vinyl acetate crotonic acid copolymers, vinyl methacrylate copolymers, monoalkyl esters of poly(methyl vinyl ether (PVM)/maleic acid (MA)), such as, for example, ethyl, butyl and isopropyl esters of PVM/MA copolymer acrylic acid/ethyl acrylate/N-tert-butyl-acrylamide terpolymers, and poly (methacrylic acid/acrylamidomethyl propane sulfonic acid), acrylates copolymer, acrylates/octylacrylamide copolymer, vinyl acetate (VA)/crotonates/vinyl neodeanoate copolymer, poly(N-vinyl acetamide), poly(N-vinyl formamide), corn starch modified, sodium polystyrene sulfonate, polyquaterniums such as, for example, Polyquaternium-24, Polyquaternium-29, Polyquaternium-32, Polyquaternium-34, Polyquaternium-37, Polyquaternium-39, Polyquaternium-44, Polyquaternium-47, Polyquaternium-68, Polyquaternium-69, Polyquaternium-87, polyether-1, polyurethanes, VA/acrylates/lauryl methacrylate copolymer, adipic acid/dimethylaminohydroxypropyl diethylene AMP/acrylates copolymer, methacrylol ethyl betaine/acrylates copolymer, VP/methacrylamide/vinyl imidazole copolymer, VP/vinylcaprolactam/DMAPA acrylates copolymer, VP/dimethylaminoethylmethacrylate copolymer, VP/DMAPA acrylates copolymer, vinyl caprolactam/VP/dimethylaminoethyl methacrylate copolymer, VA/butyl maleate/isobornyl acrylate copolymer, VA/crotonates copolymer, acrylate/acrylamide copolymer, VA/crotonates/vinyl propionate copolymer, VP/vinyl acetate/vinyl propionate terpolymers, VA/crotonates, VP/vinyl acetate copolymer, VP/acrylates copolymer, VA/crotonic acid/vinyl propionate, acrylates/acrylamide, acrylates/octylacrylamide, acrylates/hydroxyacrylates copolymer, acrylates/hydroxyesteracrylates copolymer, acrylates/stereth-20 methacrylate copolymer, tert-butyl acrylate/acrylic acid copolymer, diglycol/cyclohexanedimethanol/isophthalates/sulfoisophthalates copolymer, VA/butyl maleate and isobornyl acrylate copolymer, vinylcaprolactam/VP/dimethylaminoethyl methacrylate, VA/alkylmaleate half ester/N-substituted acrylamide terpolymers, vinyl caprolactam/VP/ methacryloamidopropyl trimethylammonium chloride terpolymer, methacrylates/acrylates copolymer/amine salt, polyvinylcaprolactam, polyurethanes, hydroxypropyl guar, poly (methacrylic acid/acrylamidomethyl propane sulfonic acid (AMPSA), ethylenecarboxamide (EC)/AMPSA/methacrylic acid (MAA), poylurethane/acrylate copolymers and hydroxypropyl trimmonium chloride guar, acrylates copolymer, acrylates crosspolymer, AMP-acrylates/allyl methacrylate copolymer, polyacrylate-14, polyacrylate-2 crosspolymer, octylacrylamide/acrylates/butylaminoethyl methacrylate copolymer, acrylates/octylacrylamide copolymer, VA/crotonates/vinyl neodeanoate copolymer, poly(N-vinyl acetamide), poly(N-vinyl formamide), polyurethane, methacryloyl ethyl betaines/methacrylates copolymer, corn starch modified, sodium polystyrene sulfonate, polyurethane/acrylates copolymer, chitosan glycolate, cationic polygalactomannans, such as, for example, quaternized derivatives of guar and cassia, such as, for example, guar hydroxypropyl trimmonium chloride, hydroxypropyl guar hydroxypropyl trimmonium chloride and cassia hydroxypropyl trimmonium chloride. Many of the foregoing polymers are referred to by their INCI nomenclature set forth in the International Cosmetic Ingredient Dictionary published by the Cosmetic, Toiletry, and Fragrance Association, Washington D.C. Other suitable auxiliary fixative polymers are disclosed in U.S. Patent No. 7,205,271, the disclosure of which is herein incorporated by reference.

One skin care composition embodiment comprises a polymer in an amount effective to provide to the skin care composition a property, such as a skin conditioning property, a viscid property (thickening, rheology modifying), or a combination thereof. Optionally, the skin care composition can include one or more of an auxiliary skin conditioning agent, an auxiliary rheology modifying agent, or a mixture thereof.

The multi-purpose polymers provide desirable rheological properties to low pH aqueous personal care, health care, home care, and "I&I" products. The cationic or cationogenic nature of the polymers allow them to swell upon

acidification with either inorganic acid or organic acid, including amino acid, or upon alkylation, or by both acidification and alkylation. The polymers of the present invention beneficially can thicken acidic aqueous formulations to provide aesthetically smooth-textured products that flow smoothly and spread easily. Formulations containing the inventive polymers can be processed by adjusting the pH to a value preferably in the range of about 1 to not more than about 7, if an acidic pH is desired, with acidic materials. The form of a polymer containing product can range from a non-pourable, stiff to soft gel, a semisolid paste to a substantially solid stick or bar, and aerosolized foam to squeezable gel, as well as a non-runny, yet flowable, product, suitable for pumpable spray or roll-on products and liquid lotions. The multi-purpose polymers of the invention can be tailored as a component in low viscosity product applications.

In one embodiment, the polymer is added to a desired personal care, health care, home care, and "I&I" formulation and the pH is adjusted downward with an organic acid or mineral acid to optimize acid swelling to the desired viscosity, and then adjusting the final composition to the desired pH. If the pH of a completed composition or formulation containing an acid-swollen multi-purpose polymer is more acidic than required for the intended use of the formulation, the pH can then be further adjusted with any, physiologically tolerable, inorganic or organic base.

The polymers are surprisingly compatible with cationic surfactants (described below) and other cationic compounds suitable as antistatic agents or conditioners employed in hair care, skin care and fabric care products. The term "antistatic agents" refers to ingredients that alter the electrical properties of cosmetic raw materials or of human body surfaces (e.g., skin, hair, etc.) and textiles (e.g., woven, non-woven, etc.), for example, by reducing their tendency to acquire an electrical charge and thus, can condition hair, skin, and fabrics. The cationic compatibility of the multi-purpose polymers makes them suitable for incorporation into formulations containing antistatic agents typically employed in hair care compositions, such as shampoos, conditioning shampoos, post-shampoo conditioning rinses, hair sprays, hair dressings and the like. The antistatic agent can be employed in amounts up to about 30 weight percent of the final composition, but is not limited thereto.

Antistatic agents include, but are not limited to, quaternary ammonium compounds, protein derivatives, synthetic quaternary ammonium polymers, amines, protonated amine oxides, betaines, and the like, which may act as antistatic agents in specific formulations and under controlled pH conditions in addition to any surfactant properties imparted by such materials. In addition to antistatic agents previously discussed, non-limiting examples of quaternary ammonium compounds useful as antistatic agents are acetamidopropyl trimonium chloride, behenamidopropyl dimethylamine, behenamidopropyl ethyldimonium ethosulfate, behentrimonium chloride, cetethyl morpholinium ethosulfate, cetrimonium chloride, cocoamidopropyl ethyldimonium ethosulfate, dicetyldimonium chloride, dimethicone hydroxypropyl trimonium chloride, hydroxyethyl behenamidopropyl dimonium chloride, quaternium-26, quaternium-27, quaternium-53, quaternium-63, quaternium-70, quaternium-72, quaternium-76 hydrolyzed collagen, PPG-9 diethylmonium chloride, PPG-25 diethylmonium chloride, PPG-40 diethylmonium chloride, stearalkonium chloride, stearamidopropyl ethyl dimonium ethosulfate, steardimonium hydroxypropyl hydrolyzed wheat protein, steardimonium hydroxypropyl hydrolyzed collagen, wheat germamidopropalkonium chloride, wheat germamidopropyl ethyldimonium ethosulfate, and the like.

Synthetic quaternary ammonium polymers, include film-forming polymers and conditioning polymers. Non-limiting examples of synthetic quaternary ammonium polymers include polymers and copolymers of dimethyl diallyl ammonium chloride, such as polyquaternium-4, polyquaternium-6, polyquaternium-7, polyquaternium-10, polyquaternium-11 polyquaternium-15, polyquaternium-16, polyquaternium-22, polyquaternium-24, polyquaternium-28, polyquaternium-32, polyquaternium-33, polyquaternium-35, polyquaternium-37, polyquaternium-39, polyquaternium-43, polyquaternium-44, polyquaternium-46, PEG-2-cocomonium chloride, and cassia hydroxypropyltrimonium chloride, quaternium-52, polyquaternium-55, polyquaternium-44, polyquaternium-60, polyquaternium-66, polyquaternium-67, polyquaternium-68, polyquaternium-69, polyquaternium-72, polyquaternium-77, polyquaternium-85, polyquaternium-86, polyquaternium-87, and the like, and combinations thereof.

Suitable commercial conditioning polymers include polymeric quaternary ammonium salts such as, without being limited thereto, polyquaternium-7, a polymeric quaternary ammonium salt of acrylamide and dimethyl diallylammonium chloride monomers (such as MACKERNIUM™-007, McIntyre Group, Ltd.); polyquaternium-10, a polymeric quaternary ammonium salt of hydroxyethylcellulose reacted with a trimethylammonium substituted epoxide (such as the UCARE® Polymers JR, LK, LR, SR series, Amerchol and CELQUAT® SC series, National Starch); polyquaternium-39, a polymeric quaternary ammonium salt of acrylic acid, diallyl dimethylammonium chloride and acrylamide (such as the MERQUAT® and MERQUAT® Plus polymers, Ondeo Nalco); quaternized derivatives of natural gums, e.g., guar hydroxypropyltrimonium chloride (such as the JAGUAR® and JAGUAR® Excel polymers, Rhodia, Inc.), and the like.

A number of non-polymeric quaternary ammonium compounds are used for fabric conditioning and fabric care, generally referred to as fabric softening agents, and are typically employed in amounts of up to about 20 weight percent of the total weight of the formulation, but are not limited thereto. Fabric softening agents useful in combination with the multi-purpose polymers generally include long-chain alkylated quaternary ammonium compounds such as dialkyldimethyl quaternary ammonium compounds, imidazoline quaternary compounds, amidoamine quaternary compounds, dialkyl ester quat derivatives of dihydroxypropyl ammonium compounds; dialkyl ester quat derivatives of methyltriethanol ammonium compounds, ester amide amine compounds, and diester quat derivatives of dimethyldiethanol ammonium

chloride, as described in the review article by Whalley, Fabric Conditioning Agents, HAPPI, pp. 55-58 (February 1995), incorporated herein by reference.

In addition to the previously discussed antistatic agents, non-limiting examples of dialkyldimethyl quaternary ammonium compounds, include N,N-dioleyl-N,N-dimethylammonium chloride, N,N-ditallowyl-N,N-dimethylammonium ethosulfate, N,N-di(hydrogenated-tallowyl)-N,N-dimethylammonium chloride, and the like. Non-limiting examples of imidazoline quaternary compounds include 1-N-methyl-3-N-tallowamidoethylimidazolium chloride, 3-methyl-1-tallowylamidoethyl-2-tallowyl-imidazolinium methylsulfate, available from Witco Chemical Company under the tradename VARISOFT® 475, and the like. Non-limiting examples of amidoamine quaternary compounds include N-alkyl-N-methyl-N,N-bis(2-tallowamidoethyl)ammonium salts where the alkyl group can be methyl, ethyl, hydroxyethyl, and the like. Non-limiting examples of dialkyl ester quat derivatives of dihydroxypropyl ammonium compounds include 1,2-ditallowoyloxy-3-N,N,N-trimethylammoniopropane chloride, 1,2-dicanoloyloxy-3-N,N,N-trimethylammoniopropane chloride, and the like.

In addition, other types of long chain (e.g., natural oil and fatty acid-derived) alkylated quaternary ammonium compounds are suitable fabric softening agents, including, but not limited, to N,N-di(alkyloxyethyl)-N,N-dimethylammonium salts such as N,N-di(tallowyloxyethyl)-N,N-dimethylammonium chloride, N,N-di(canolyloxyethyl)-N,N-dimethylammonium chloride, and the like; N,N-di(alkyloxyethyl)-N-methyl-N-(2-hydroxyethyl)ammonium salts such as N,N-di(tallowyloxyethyl)-N-methyl-N-(2-hydroxyethyl)ammonium chloride, N,N-di(canolyloxyethyl)-N-methyl-N-(2-hydroxyethyl)ammonium chloride, and the like; N,N-di(2-alkyloxy-2-oxoethyl)-N,N-dimethylammonium salts, such as N,N-di(2-tallowyloxy-2-oxoethyl)-N,N-dimethylammonium chloride, N,N-di(2-canolyloxy-2-oxoethyl)-N,N-dimethylammonium chloride, and the like; N,N-di(2-alkyloxyethylcarbonyloxyethyl)-N,N-dimethylammonium salts, such as N,N-di(2-tallowyloxyethylcarbonyloxyethyl)-N,N-dimethylammonium chloride, N,N-di(2-canolyloxyethylcarbonyloxyethyl)-N,N-dimethylammonium chloride, and the like; N-(2-alkanoyloxy-2-ethyl)-N-(2-alkyloxy-2-oxoethyl)-N,N-dimethyl ammonium salts, such as N-(2-tallowoyloxy-2-ethyl)-N-(2-tallowyloxy-2-oxoethyl)-N,N-dimethyl ammonium chloride, N-(2-canoloyloxy-2-ethyl)-N-(2-canolyloxy-2-oxoethyl)-N,N-dimethyl ammonium chloride, and the like; N,N,N-tri(alkyloxyethyl)-N-methyl ammonium salts, such as N,N,N-tri(tallowyloxyethyl)-N-methylammonium chloride, N,N,N-tri(canolyloxyethyl)-N-methylammonium chloride, and the like; N-(2-alkyloxy-2-oxoethyl)-N-alkyl-N,N-dimethyl ammonium salts, such as N-(2-tallowyloxy-2-oxoethyl)-N-tallowyl-N,N-dimethyl ammonium chloride, N-(2-canolyloxy-2-oxoethyl)-N-canolyl-N,N-dimethyl ammonium chloride, and the like.

In one embodiment, the long-chain alkyl groups are derived from tallow, canola oil, or from palm oil, however, other alkyl groups derived from soybean oil and coconut oil, for example, are also suitable, as are lauryl, oleyl, ricinoleyl, stearyl, palmityl, and like fatty alkyl groups. The quaternary ammonium salt compounds can have any anionic group as a counter-ion, for example, chloride, bromide, methosulfate (i.e., methylsulfate), acetate, formate, sulfate, nitrate, and the like.

Examples of suitable quaternary ammonium fabric softening compounds include N-methyl-N,N-bis(tallowamidoethyl)-N-(2-hydroxyethyl)ammonium methylsulfate and N-methyl-N,N-bis(hydrogenated-tallowamidoethyl)-N-(2-hydroxyethyl) ammonium methylsulfate, each of which materials are available from Witco Chemical Company under the trade names VARISOFT® 222 and VARISOFT® 110, respectively; dialkyl esterquat derivatives of methyltriethanol ammonium salts such as the DEHYQUART® AU series of bis(acyloxyethyl) hydroxyethylmethylammonium methosulfate esterquats available from Cognis, such as DEHYQUART® AU35, AU46, AU56, and the like; and N,N-di(tallowoyloxyethyl)-N,N-dimethylammonium chloride, where the tallow chains are at least partially unsaturated. Other suitable fabric softening agents include the well-known dialkyldimethyl ammonium salts such as N,N-ditallowyl-N,N-dimethyl ammonium methylsulfate, N,N-di(hydrogenated-tallowyl)-N,N-dimethyl ammonium chloride, N,N-distearyl-N,N-dimethyl ammonium chloride, N,N-dibehenyl-N,N-dimethylammonium chloride, N,N-di(hydrogenated tallow)-N,N-dimethyl ammonium chloride (trade name ADOGEN® 442), N,N-ditallowyl-N,N-dimethyl ammonium chloride (trade name ADOGEN® 470, PRAEPAGEN® 3445), N,N-distearyl-N,N-dimethyl ammonium chloride (trade name AROSURF® TA-100), all available from Witco Chemical Company; N,N-dibehenyl-N,N-dimethyl ammonium chloride, sold under the trade name KEMAMINE® Q-2802C by Humko Chemical Division of Witco Chemical Corporation; and N,N-dimethyl-N-stearyl-N-benzylammonium chloride sold under the trade names VARISOFT® SDC by Witco Chemical Company and AMMONYX® 490 by Onyx Chemical Company.

Any of the foregoing fabric softening agents, and mixtures thereof, can be utilized in combination with the multi-purpose polymers, particularly in laundry and fabric care products. For ester-containing fabric softening agents, the pH of the compositions can influence the stability of the fabric softening agents, especially in prolonged storage conditions. The pH, as defined in the present context, is measured in the neat compositions at about 20°C. In one embodiment, the pH of the composition is less than about 6. For optimum hydrolytic stability of these compositions, the pH is in the range of from about 2 to about 5, or from about 2.5 to about 3.5.

In addition to protein derivatives previously described, non-limiting examples of protein derivatives include cocodimonium hydroxypropyl hydrolyzed casein, cocodimonium hydroxypropyl hydrolyzed collagen, cocodimonium hydroxypropyl hydrolyzed hair keratin, cocodimonium hydroxypropyl hydrolyzed rice protein, cocodimonium hydroxypropyl

hydrolyzed silk, cocodimonium hydroxypropyl hydrolyzed soy protein, cocodimonium hydroxypropyl hydrolyzed wheat protein, cocodimonium hydroxypropyl hydrolyzed silk amino acids, hydroxypropyl trimonium hydrolyzed collagen, hydroxypropyl trimonium hydrolyzed keratin, hydroxypropyl trimonium hydrolyzed silk, hydroxypropyl trimonium hydrolyzed rice bran, hydroxypropyl trimonium hydrolyzed soy protein, hydroxypropyl trimonium hydrolyzed vegetable protein, hydroxypropyl trimonium hydrolyzed wheat protein, soyethyldimonium ethosulfate, soyethyl morpholinium ethosulfate, and the like.

In addition to the foregoing, the cationic or character of the polymers at acid pH, and its surprising cationic compatibility, makes the multi-purpose polymer useful as a thickener for antistatic, biocidal, antimicrobial, and other preservative compositions, in a variety of personal care, health care, I&I, and medical applications. For example, the polymer can be employed as a thickener in over-the-counter (OTC) health care and pharmaceutical products where cationic biocides are typically employed, such as in oral care compositions for plaque and tartar control, and liquid vehicles containing therapeutic agents, such as syrups, gels, and the like. Under certain controlled pH conditions, the cationic character of the multi-purpose polymer, itself, may also provide antistatic activity or biocidal, antimicrobial, or like preservative activity.

Surfactants are generally employed in personal care, health care, home care, and "I&I" as cleansing agents, emulsifying agents, foam boosters, hydrotropes and suspending agents. The polymers may be employed in formulations containing all classes of surfactants, i.e., anionic surfactants, cationic surfactants, nonionic surfactants, amphoteric surfactants and mixtures thereof. The term "amphoteric surfactant" as used herein includes zwitterionic surfactants. In addition to the foregoing references, discussions of the classes of surfactants are in Cosmetics & Toiletries® C&T Ingredient Resource Series, Surfactant Encyclopedia, 2nd Edition, Rieger (ed), Allured Publishing Corporation (1996); Schwartz, et al., Surface Active Agents, Their Chemistry and Technology, published 1949; and Surface Active Agents and Detergents, Volume II, published 1958, Interscience Publishers; each incorporated herein by reference.

Surprisingly, the polymers are useful as thickeners, stabilizers, suspending agents, and deposition aids in compositions containing various amounts and/or concentrations one or more various surfactants (e.g., anionic, cationic, amphoteric, non-ionic, and/or combinations of any two or more thereof). In one embodiment, the amount of surfactant, or surfactants, present is in the range of about 1 weight percent to about 40 weight percent, or from about 2.5 weight percent to about 35 weight percent, or from about 5 weight percent to about 30 weight percent, or from about 10 weight percent to about 25 weight percent, or even about 15 weight percent to about 22.5 weight percent. Here, as well as elsewhere in the specification and claims, individual numerical values, or limits, can be combined to form additional non-disclosed and/or non-stated ranges. In another embodiment, when two or more different surfactants and/or different types of surfactants are utilized, the ratio of any two or more surfactants and/or types of surfactants can be any ratio typically used in home care, personal care, health care, home care, and/or I&I as known to those of skill in the art.

The amount of multi-purpose polymer that can be employed depends upon the purpose for which they are included in the formulation and can be readily determined by person skilled in the formulation arts. Thus, as long as the physicochemical and functional properties of the compositions containing a multi-purpose polymer are achieved, a useful amount of multi-purpose polymer, active weight percent, on a total composition weight basis, typically can vary in the range of about 0.01 percent to about 30 wt. percent, but is not limited thereto. In a given composition or application, therefore, the multi-purpose polymers can, but need not, serve more than one function, such as thickener and conditioner, film-former, deposition aid, rheology modifier, suspending aid, hair fixative, and carrier, and the like, as discussed in the non-limiting disclosure above.

Compositions containing a multi-purpose polymer can be packaged and dispensed from containers, such as jars, bottles, tubes, spray bottles, wipes, cans, roll-on containers, stick containers, and the like, without limitation. There is no limitation as to the form of product in which the multi-purpose polymer can be incorporated, so long as the purpose for which the product is used is achieved. For example, personal care and health care products containing a multi-purpose polymer can be applied to the skin, hair, scalp and nails in the form of, without being limited thereto, gels, mousses, sprays (liquid or foam), emulsions (creams, lotions, pastes), liquids (rinses, shampoos), bars, ointments, suppositories, impregnated wipes, patches, and the like.

**Additives and Adjuvants:**

Product formulations comprising the multi-purpose polymer(s) can contain the various additives and adjuvants previously described and/or conventionally or popularly included in personal care, health care, home care, "I&I" care products, and in industrial processes, including, without being limited thereto, acidifying or alkalizing pH adjusting agents and buffering agents; auxiliary fixatives and film formers (e.g., nonionic, anionic, cationic, or amphoteric polymers of synthetic or natural origin); auxiliary rheology modifiers (e.g., viscosity-increasing polymeric, gum, or resin thickeners or gellants); additives (e.g., auxiliary emulsifiers, auxiliary emulsion stabilizers, waxes, auxiliary dispersants, and the like), viscosity control agents (e.g., electrolytes), auxiliary conditioning agents (e.g., synthetic oils, natural oils, animal oils, natural and synthetic waxes, silicones, monomeric and polymeric quaternized ammonium compounds (previously

described) and derivatives thereof), sheen enhancers; moisturizers; emollients; humectants; lubricants; sunscreen agents; UV absorbing agents; oxidizing agents; reducing agents; surfactants (e.g., anionic, cationic, nonionic, amphoteric, zwitterionic, and silicone derivatives thereof, and mixtures thereof); polymer film modifying agents (e.g., plasticizers, tackifiers, de-tackifiers, wetting agents, and the like); chelating agents; opacifiers; pearlizing agents; proteinaceous materials and derivatives thereof; vitamins and derivatives thereof; botanicals; antifungal agents; antidandruff agents; anti-inflammatory agents; analgesics; preservatives; fragrances; fragrance solubilizers; colorants (e.g., pigments and dyes); propellants (e.g., fluorinated hydrocarbons, liquid volatile hydrocarbons, compressed gases, and the like; and mixtures thereof.

Additives and adjuvant ingredients, products, or materials, which may be employed with the inventive multi-purpose polymers discussed herein are in some cases referred to by the international nomenclature commonly referred to as INCI name given them in the International Cosmetic Ingredient Dictionary, published by the Cosmetic, Toiletry, and Fragrance Association, Washington D.C. (see www.ctfa-online.org - hereafter INCI Dictionary), such as can be found in any edition thereof, for example, Volumes 1 and 2, Sixth Edition, (1995) or Volumes 1-3, Seventh and Eighth Editions, (1997, 2000), or by their commonly used chemical names. Numerous commercial suppliers of materials listed by INCI name, trade name or both can be found in the INCI Dictionary and in numerous commercial trade publications, including but not limited to the 2001 McCutcheon 's Directories, Volume 1: Emulsifiers & Detergents and Volume 2: Functional Materials, published by McCutcheon's Division, The Manufacturing Confectioner Publishing Co., Glen Rock, N.J. (2001); and 2001 Cosmetic Bench Reference, edition of Cosmetics & Toiletries® 115 (13), published by Allured Publishing Corporation, Carol Stream, Ill. (2001); the relevant disclosures of each are incorporated herein by reference. Such components and the formulation of compositions are also described in detail in well known references, such as Cosmetics Science and Technology, First Edition (Sagarin (ed)), published 1957, and Second Edition (Balsam, et al. (eds)), published 1972 to 1974; and The Chemistry and Manufacture of Cosmetics, Second Edition (deNavarre (ed)), published 1975, and Third Edition (Schlossman (ed)), published 2000, both available from Allured Publishing Corporation; Rieger (ed), Harry's Cosmeticology, 8th Edition, Chemical Publishing, Co., Inc., New York, N.Y. (2000); and various formularies available to those skilled in the pharmaceutical arts, such as Remington's Pharmaceutical Sciences, Fourteenth Edition, Mack Publishing Company, Easton, Pa. (1970); the relevant disclosures of each are incorporated herein by reference.

It is known that formulated compositions for personal care and topical, dermatological, health care, which are applied to the skin and mucous membranes for cleansing or soothing, are compounded with many of the same or similar physiologically tolerable ingredients and formulated in the same or similar product forms, differing primarily in the purity grade of ingredient selected, by the presence of medicaments or pharmaceutically accepted compounds, and by the controlled conditions under which products may be manufactured. Likewise, many of the ingredients employed in products for home care and I&I are the same or similar to the foregoing, differing primarily in the amounts and material grade employed. It is also known that the selection and permitted amount of ingredients also may be subject to governmental regulations, on a national, regional, local, and international level. Thus, discussion herein of various useful ingredients for personal care and health care products may apply to home care and I&I products and industrial applications.

Solvents

The polymers prepared as aqueous emulsions are particularly useful for water-based formulations, and formulations containing water-miscible auxiliary solvents, but are not limited thereto. Useful solvents commonly employed are typically liquids, such as water (deionized, distilled or purified), alcohols, polyols, and the like, and mixtures thereof. Non-aqueous or hydrophobic auxiliary solvents are commonly employed in substantially water-free products, such as nail lacquers, aerosol propellant sprays, or for specific functions, such as removal of oily soils, sebum, make-up, or for dissolving dyes, fragrances, and the like, or are incorporated in the oily phase of an emulsion. Non-limiting examples of auxiliary solvents, other than water, include linear and branched alcohols, such as ethanol, propanol, isopropanol, hexanol, and the like; aromatic alcohols, such as benzyl alcohol, cyclohexanol, and the like; saturated $C_{12}$ to $C_{30}$ fatty alcohol, such as lauryl alcohol, myristyl alcohol, cetyl alcohol, stearyl alcohol, behenyl alcohol, and the like. Non-limiting examples of polyols include polyhydroxy alcohols, such as glycerin, propylene glycol, butylene glycol, hexylene glycol, $C_2$ to $C_4$ alkoxylated alcohols and $C_2$ to $C_4$ alkoxylated polyols, such as ethoxylated, propoxylated, and butoxylated ethers of alcohols, diols, and polyols having about 2 to about 30 carbon atoms and 1 to about 40 alkoxy units, polypropylene glycol, polybutylene glycol, and the like. Non-limiting examples of non-aqueous auxiliary solvents include silicones, and silicone derivatives, such as cyclomethicone, and the like, ketones such as acetone and methylethyl ketone; natural and synthetic oils and waxes, such as vegetable oils, plant oils, animal oils, essential oils, mineral oils, $C_7$ to $C_{40}$ isoparaffins, alkyl carboxylic esters, such as ethyl acetate, amyl acetate, ethyl lactate, and the like, jojoba oil, shark liver oil, and the like. Some of the foregoing non-aqueous auxiliary solvents may also be conditioners and emulsifiers.

Propellants

Suitable propellants that can be utilized in compositions comprising the multi-purpose polymers include but are not limited to Where applicable, any known aerosol propellant can be utilized to deliver the personal care, home care, health care and institutional care compositions containing the esters in combination with one or more of the foregoing active ingredients and/or with the one or more additives and/or adjuvants, conventionally or popularly included in personal care, health care, home care, and institutional care products discussed above. Exemplary propellants include, but are not limited to, lower boiling hydrocarbons such as $C_3$ to $C_6$ straight and branched chain hydrocarbons. Exemplary hydrocarbon propellants include propane, butane, isobutene, and mixtures thereof. Other suitable propellants include ethers, such as, dimethyl ether, hydrofluorocarbons, such as, 1,1-difluoroethane, and compressed gasses, such as air and carbon dioxide. These compositions can contain from about 0.5 to about 60 wt. % of the propellant in one embodiment and from about 0.5 to about 35 wt. % in another embodiment, based on the total weight of the composition.

Surfactants

Anionic surfactants include substances having a negatively charged hydrophobe or that carry a negative charge when the pH is elevated to neutrality or above, such as acylamino acids, and salts thereof, for example, acylglutamates, acyl peptides, sarcosinates, and taurates; carboxylic acids, and salts thereof, for example, alkanolic acids and alkanoates, ester carboxylic acids, and ether carboxylic acids; phosphoric acid ester and salts thereof; sulfonic acids and salts thereof, for example, acyl isethionates, alkylaryl sulfonates, alkyl sulfonates, and sulfosuccinates; and sulfuric acid esters, such as alkyl ether sulfates and alkyl sulfates.

Non-limiting examples of anionic surfactants include mono-basic salts of acylglutamates that are slightly acidic in aqueous solution, such as sodium acylglutamate and sodium hydrogenated tallow glutamate; salts of acyl-hydrolyzed protein, such as potassium, palmitoyl hydrolyzed milk protein, sodium cocoyl hydrolyzed soy protein, and TEA-abietoyl hydrolyzed collagen; salts of acyl sarcosinates, such as ammonium myristoyl sarcosine, sodium cocoyl sarcosinate, and TEA-lauroyl sarcosinate; salts of sodium methyl acyltaurates, such as sodium lauroyl taurate and sodium methyl cocoyl taurate; alkanoic acids and alkanoates, such as fatty acids derived from animal and vegetable glycerides that form water-soluble soaps and water-insoluble emulsifying soaps, including sodium stearate, aluminum stearate, and zinc undecylenate; ester carboxylic acids, such as dinonoxynol-9-citrate; salts of acyl lactylates such as calcium stearoyl lactylate and laureth-6 citrate; ethercarboxylic acids derived from ethyoxylated alcohols or phenols having varying lengths of polyoxyethylene chains, such as nonoxynol-8 carboxylic acid, and sodium trideceth-13 carboxylate; mono- and di-esters of phosphoric acid and their salts, such as phospholipids, dilaureth-4-phosphate, DEA-oleth-10 phosphate and triethanolamine lauryl phosphate; salts of acylisethionate, such as sodium cocoyl isethionate; alkylarylbenzene sulfonates, such as alpha-olefin sulfonate (AOS) and alkali metal, alkaline earth metal, and alkanolamine salts thereof, and sodium dodecylbenzene sulfonate; alkyl sulfonates, such as sodium $C_{12}$ to $C_{14}$ olefin sulfonate, sodium cocomonoglyceride sulfonate, sodium $C_{12}$ to $C_{15}$ pareth-15 sulfonate, and sodium lauryl sulfoacetate; sulfosuccinates, such as mono- and di-esters of sulfosuccinic acid, salts thereof and alkoxylated alkyl and alkylamido derivatives thereof, such as di-$C_4$ to $C_{10}$ alkyl sodium sulfosuccinate, disodium laureth sulfosuccinate, disodium oleamido MEA-sulfosuccinate, and disodium $C_{12}$ to $C_{15}$, pareth sulfosuccinate; alkyl ether sulfates, such as sodium and ammonium lauryl ether sulfate (having about 1 to about 12 moles ethylene oxide); alkyl sulfates, such as sodium, ammonium and triethanolamine salts of $C_{12}$ to $C_{18}$ alkylsulfates, sodium $C_{12}$ to $C_{14}$ olefin sulfates, sodium laureth-6 carboxylate, sodium $C_{12}$ to $C_{18}$ pareth sulfate, and the like.

Cationic surfactants can have a hydrophobe that carries a positive charge or that is uncharged at pH values close to neutrality or lower, such as alkylamines, alkyl imidazolines, ethoxylated amines, and quaternary ammonium compounds. Cationic surfactants used in cosmetics are, in one embodiment, N-derivatives and the neutralizing anion may be inorganic or organic. Among the cationic surfactant materials useful herein are quaternary ammonium compounds corresponding to the general formula: $(R_{10}R_{11}R_{12}R_{13}N^+)\ E^-$, wherein each of $R_{10}$, $R_{11}$, $R_{12}$, and $R_{13}$ are independently selected from an aliphatic group having from 1 to about 22 carbon atoms, or an aromatic, alkoxy, polyoxyalkylene, alkylamido, hydroxyalkyl, aryl or alkylaryl group having 1 to about 22 carbon atoms in the alkyl chain; and $E^-$ is a salt-forming anion such as those selected from halogen, (e.g., chloride, bromide), acetate, citrate, lactate, glycolate, phosphate, nitrate, sulfate, and alkylsulfate. The aliphatic groups can contain, in addition to carbon and hydrogen atoms, ether linkages, ester linkages, and other groups such as amino groups. The longer chain aliphatic groups, e.g., those of about 12 carbons, or higher, can be saturated or unsaturated.

Alkylamines can be salts of primary, secondary and tertiary fatty $C_{12}$ to $C_{22}$ alkylamines, substituted or unsubstituted, and substances sometimes referred to as "amidoamines." Non-limiting examples of alkyl amines and salts thereof include dimethyl cocamine, dimethyl palmitamine, dioctylamine, dimethyl stearamine, dimethyl soyamine, soyamine, myristyl amine, tridecyl amine, ethyl stearylamine, N-tallowpropane diamine, ethoxylated stearylamine, dihydroxy ethyl stearylamine, arachidylbehenylamine, dimethyl lauramine, stearylamine hydrochloride, soyamine chloride,

stearylamine formate, N-tallowpropane diamine dichloride, and amodimethicone (INCI name for a silicone polymer and blocked with amino functional groups, such as aminoethylamino propylsiloxane). Non-limiting examples of amidoamines and salts thereof include stearamido propyl dimethyl amine, stearamidopropyl dimethylamine citrate, palmitamidopropyl diethylamine, and cocamidopropyl dimethylamine lactate. Other cationic surfactants include distearyldimonium chloride, dicetyldimonium chloride, guar hydroxypropyltrimonium chloride, and the like. At low pH, amine oxides may protonate and behave similarly to N-alkyl amines.

Non-limiting examples of alkyl imidazolines include alkyl hydroxyethyl imidazoline, such as stearyl hydroxyethyl imidazoline, coco hydroxyethyl imidazoline, ethyl hydroxymethyl oleyl oxazoline, and the like. Non-limiting examples of ethyoxylated amines include PEG-cocopolyamine, PEG-15 tallow amine, quaternium-52, and the like.

Quaternary ammonium compounds are monomeric or polymeric materials containing at least one nitrogen atom that is linked covalently to four alkyl and/or aryl substituents, and the nitrogen atom remains positively charged regardless of the environmental pH. Quaternary ammonium compounds comprise a large number of substances that are used extensively as surfactants, conditioners, antistatic agents, and antimicrobial agents and include, alkylbenzyldimethyl ammonium salts, alkyl betaines, heterocyclic ammonium salts, and tetraalkylammonium salts. Long-chain (fatty) alkylbenzyldimethyl ammonium salts are, in one embodiment, utilized as conditioners, as antistatic agents, and as fabric softeners, discussed in more detail below. Other quaternary ammonium compounds include quaternary ammonium silicones.

Non-limiting examples of alkylbenzyldimethylammonium salts include stearalkonium chloride, benzalkonium chloride, quaternium-63, olealkonium chloride, didecyldimonium chloride, and the like. Alkyl betaine compounds include alkylamidopropyl betaine, alkylamidopropyl hydroxysultaine, and sodium alkylamido propyl hydroxyphostaine. Non-limiting examples of alkyl betaine compounds include oleyl betaine, coco-betaine, cocoamidopropyl betaine, coco-hydroxy sultaine, coco/oleamidopropyl betaine, coco-sultaine, cocoamidopropylhydroxy sultaine, and sodium lauramidopropyl hydroxyphostaine. Heterocyclic ammonium salts include alkylethyl morpholinium ethosulfate, isostearyl ethylimidonium ethosulfate, and alkylpyridinium chlorides, and are generally used as emulsifying agents. Non-limiting examples of heterocyclic ammonium salts include cetylpyridinium chloride, isostearylethylimidonium ethosulfate, and the like. Non-limiting examples of tetraalkylammonium salts include cocamidopropyl ethyldimonium ethosulfate, hydroxyethyl cetyldimonium chloride, quaternium-18, and cocodimonium hyroxypropyl hydrolyzed protein, such as hair keratin, and the like.

Nonionic surfactants are generally uncharged amphiphiles and usually are alkoxylated to varying degrees. Classes of nonionic surfactants include alcohols, alkanolamides, amine oxides, esters, and ethers. Nonionic alcohols are usually hydroxy derivatives of long-chain $C_8$ to $C_{18}$ alkane hydrocarbons, such as cetearyl alcohol, hydrogenated tallow alcohol, lanolin alcohols, alkanolamides, and the like. Alkanolamides contain at least one alkoxyl or one polyoxyethylene grouping and include alkanol-derived amides, such as acylamide DEA, N-alkyl pyrrolidone, palmamide MEA, peanutamide MIPA, and the like and ethoxylated amides, such as PEG-50 tallow amide. Amine oxides include alkylamine oxides, such as lauramine oxide; and acylamidopropyl morpholine oxides, such as cocamidopropylamine oxide; and the like. Esters include ethoxylated carboxylic acids, such as PEG-8 dilaurate, PEG-8 laurate, and the like; ethoxylated glycerides, such as PEG-4 castor oil, PEG-120 glyceryl stearate, triolein PEG-6 esters, and the like; glycol esters and derivatives thereof, such as glycol stearate SE, propylene glycol ricinoleate, and the like; monoglycerides, such as glyceryl myristate, glyceryl palmitate lactate, and the like; polyglyceryl esters, such as polyglyceryl-6-distearate, polyglyceryl-4 oleyl ether, and the like, polyhydric alcohol esters and ethers, such as methyl gluceth-20 sesquistearate, sucrose distearate; and the like; sorbitan/sorbitol esters, such as polysorbate-60, sorbitan sequiisostearate, and the like; and triesters of phosphoric acid, such as trideceth-3 phosphate, trioleth-8 phosphate, and the like. Ethers include ethoxylated alcohols, such as ceteareth-10, nonoxynol-9, and the like; ethoxylated lanolin, such as PEG-20 lanolin, PPG-12-PEG-65 lanolin oil, and the like; ethoxylated polysiloxanes, such as dimethicone copolyol, and the like; propoxylated POE ethers, such as meroxapol 314, poloxamer 122, PPG-5-ceteth-20, and the like; and alkyl polyglycosides, such as lauryl glucose, and the like.

Nonionic surfactants can be used as emulsifiers, suspending agents, solubilizers, foam boosters, and in some cases, as hydrotropes. Non-limiting examples of suitable nonionic surfactants include, but are not limited to, linear or branched alcohol ethoxylates, $C_8$ to $C_{12}$ alkylphenol alkoxylates, such as octylphenol ethoxylates, polyoxyethylene polyoxypropylene block copolymers, and the like; $C_8$ to $C_{22}$ fatty acid esters of polyoxyethylene glycol mono- and diglycerides; sorbitan esters and ethoxylated sorbitan esters; $C_8$ to $C_{22}$ fatty acid glycol esters; block copolymers of ethylene oxide and propylene oxide; and the like. Non-limiting examples of surfactant foam boosters or hydrotropes include alkanolamides, such as acetamide MEA, monoethanolamide, diethanolamide, cocamide DEA, isopropanolamide, and the like; amine oxides, such as hydrogenated tallowamine oxide; short chain alkyl aryl sulfonates, such as sodium toluene sulfonate; sulfosuccinates, such as disodium stearyl sulfosuccinate; and the like.

Amphoteric and zwitterionic surfactants are those compounds that have the capacity of behaving either as an acid or a base, by carrying a positive charge in strongly acidic media, carrying a negative charge in strongly basic media, and forming zwitterionic species at intermediate pH. The major classes of amphoteric surfactants are acyl/dialkyl ethylenediamines and derivatives thereof, such as disodium cocoamphocarboxymethylhydroxy-propyl sulfate, disodium cocoamphodipropionate, sodium cocoamphoacetate, sodium lauroampho PG-acetatephosphate, sodium tallowamphopropionate, sodium undecylenoamphopropionate, and the like; and N-alkylamino acids, such as aminopropyl laurylglutamide, dihydroxyethyl soya glycinate, lauraminopropionic acid, and the like.

Some suitable zwitterionic surfactants for use in the present compositions include those broadly described as derivatives of aliphatic quaternary ammonium, phosphonium, and sulfonium compounds, wherein which the aliphatic radicals can be straight chain or branched, and wherein one of the aliphatic substituents contains about 8 to about 18 carbon atoms and another substituent contains an anionic water-solubilizing group, such as carboxy, sulfonate, sulfate, phosphate, phosphonate, and the like. Classes of zwitterionics include alkylamino sulfonates, alkyl betaines and alkylamido betaines, such as stearamidopropyldimethylamine, diethylaminoethyl-stearamide, dimethylstearamine, dimethylsoyamine, soyamine, myristylamine, tridecylamine, ethylstearylamine, N-tallowpropane diamine, ethoxylated (5 moles ethylene oxide) stearylamine, dihydroxy ethyl stearylamine, arachidylbehenylamine, and the like. Some suitable betaine surfactants include but are not limited to alkyl betaines, alkyl amidopropyl betaines, alkyl sulphobetaines, alkyl glycinates, alkyl carboxyglycinates, alkyl amphopropionates, alkyl amidopropyl hydroxysultaines, acyl taurates, and acyl glutamates, wherein the alkyl and acyl groups have from 8 to 18 carbon atoms. Non-limiting examples of suitable amphoteric surfactants include, but are not limited to, cocamidopropyl betaine, sodium cocoamphoacetate, cocamidopropyl hydroxysultaine, and sodium cocoamphopropionate, which are particularly suitable as mild-type cleansers for skin and hair.

pH Adjusting Agents

Suitable acidic pH adjusting agents are selected from organic acids, including amino acids, and inorganic mineral acids. Non-limiting examples of acidic pH adjusting agents include acetic acid, salicylic acid, citric acid, fumaric acid, glutamic acid, glycolic acid, hydrochloric acid, lactic acid, nitric acid, phosphoric acid, sodium bisulfate, sulfuric acid, tartaric acid, and the like, and mixtures thereof. Suitable alkaline or basic pH adjusting agents can be added either to a previously acid-swollen, or water-swollen multi-purpose polymer or to a formulation containing a multi-purpose polymer. Non-limiting examples of alkaline pH adjusting agents include alkali metal hydroxides, such as sodium hydroxide, and potassium hydroxide; ammonium hydroxide; organic bases, such as triethanolamine, diisopropylamine, dodecylamine, diisopropanolamine, aminomethyl propanol, cocamine, oleamine, morpholine, triamylamine, triethylamine, tromethamine (2-amino-2-hydroxymethyl)-1,3-propanediol, and tetrakis(hydroxypropyl)ethylene-diamine; and alkali metal salts of inorganic acids, such as sodium borate (borax), sodium phosphate, sodium pyrophosphate, and the like, and mixtures thereof. The acidic and alkaline pH adjusting agent can be utilized in any amount necessary to obtain a desired pH value in the final composition.

Silicones

Silicones are commonly used in shampoo products, such as the so-called "two-in-one" combination cleansing/conditioning shampoos, and in skin care products as well. Silicones provide conditioning properties as well as psychosensory and aesthetic properties to a formulation in which they are included, making the hair and skin feel softer and smoother to the touch. Silicones can also functions as emulsifiers and as emollients in a personal care formulation. The most common class of silicone polymers are the linear polydimethyl siloxanes having the general formula $(CH_3)_3$-$Si(CH_3)_2$-O-$(Si(CH_3)_2$-$O)_w$-$Si(CH_3)_3$ where w is an integer greater than 2. Silicones can also be branched materials wherein one or more alkyl groups in a polymer are replaced with oxygen to create a branch point. The silicon atoms may carry a wide variety of substituents which can be the same or different. The chemically least reactive substituents are the methyl or phenyl groups. Functional endblocking groups may carry nitrogen or hydroxyl moieties, as in the case of dimethiconol. Background material on silicones including sections discussing silicone fluids, gums, and resins, as well as manufacture of silicones, are found in Encyclopedia of Polymer Science and Engineering, Vol. 15, 2d ed., pp. 204 to 308, John Wiley & Sons, Inc. (1989).

Silicone fluids are typically water-insoluble oils having a viscosity in the range of a few mPa·s to several hundred thousand mPa·s. They are in the form of fluids (volatile and non-volatile), gums, gums in fluids, resins or in the form of non-ionic small and large particle size emulsions (MEM 1664, MEM 1310, 5-7137, 2-1352, MEM 1784, MEM 1310, MEM 1491, 5-7137, and MEM 2220 from Dow Corning), and anionic emulsions (MEM 1784 from Dow Corning). The refractive index of the polysiloxane fluid will generally be less than about 1.70, typically less than about 1.60. In this context, polysiloxane "fluid" includes oils as well as gums.

Another class of silicones for use in hair care products are the so-called rigid silicones (also known as silicone gums), as described, for example in United States Patent No. 4,902,499, incorporated herein by reference, which generally have a viscosity (at about 20°C) of greater than about 600,000 mPa·s and have a weight average molecular weight of at least about 500,000 Daltons as determined by intrinsic viscosity measurement. Gums are also described in United States Patent No. 4,152,416; Noll and Walter, Chemistry and Technology of Silicones, New York: Academic

Press (1968); and in General Electric Silicone Rubber Product Data Sheets SE 30, SE 33, SE 54 and SE 76. Specific non-limiting examples of gums for use in the compositions of the present invention include polydimethylsiloxane, (polydimethylsiloxane) (methylvinylsiloxane) copolymer, poly(dimethylsiloxane) (diphenyl siloxane)(methylvinylsiloxane) copolymer and mixtures thereof.

Volatile silicones are particularly useful in combination with the polymers of the present invention and are often used as lubricants in hair care products, such as shampoos. Volatile silicones include cyclic and linear polydimethylsiloxanes, and the like. Cyclic volatile silicones typically contain about 3 to about 7 silicon atoms, alternating with oxygen atoms, in a cyclic ring structure. Each silicon atom is also substituted with two alkyl groups, typically methyl groups. Linear volatile silicones are silicone fluids, as described above, having viscosities of not more than about 25 mPa·s. A description of volatile silicones is found in Todd and Byers, Volatile Silicone Fluids for Cosmetics, Cosmetics and Toiletries, Vol. 91(1), pp. 27 to 32 (1976), and in Kasprzak, Volatile Silicones, Soap/Cosmetics/Chemical Specialties, pp. 40 to 43 (December 1986), each of which is incorporated herein by reference.

Another class of silicones includes aminosilicone category which contains any amine functionalized silicone; i.e., a silicone containing at least one primary amine, secondary amine, tertiary amine, or a quaternary ammonium group. Aminosilicones can be graft or terminal. In one embodiment, the aminosilicone has a viscosity of from about 1,000 cs to about 1,000,000 cs, or from about 2,000 cs to about 600,000 cs, or even from about 4,000 cs to about 400,000 cs. Examples of aminosilicone fluids with low and high amine content (8500, 2-8566, AP-8087 from Dow Corning); amino glycol copolymer; amino phenyl resin; low and high viscosity cationic emulsions (949, 2-8194 from Dow Corning), nonionic microemulsions; silicone quat microemulsions (5-7113 from Dow Corning), and mixtures of any two or more thereof. Here, as well as elsewhere in the specification and claims, individual numerical values, or limits, can be combined to form additional non-disclosed and/or non-stated ranges.

Other silicone oils include the dimethicone copolyols, which are linear or branched copolymers of dimethylsiloxane (dimethicone) and alkylene oxides. The dimethicone polyols can be random or block copolymers. A generally useful class of dimethicone polyols are block copolymers having blocks of polydimethylsiloxane and blocks of polyalkylene oxide, such as blocks of polyethylene oxide, polypropylene oxide, or both. Dimethicone copolyols are disclosed in U.S. Patent Nos. 5,136,063 and 5,180,843, the disclosures of which are incorporated herein by reference. In addition, dimethicone copolyols are commercially available under the Silsoft® and Silwet® brand names from the General Electric Company (GE-OSi). Specific product designations include but are not limited to Silsoft 305, 430, 475, 810, 895, Silwet L 7604 (GE-OSi); Dow Corning® 5103 and 5329 from Dow Corning Corporation; and Abil® dimethicone copolyols, such as, for example WE 09, WS 08, EM 90 and EM 97 from Evonik Goldschmidt Corporation; and Silsense™ dimethicone copolyols, such as Silsense Copolyol-1 and Silsense Copolyol-7, available from Lubrizol Advanced Materials, Inc.

Silicone materials, including volatile silicones, silicone gums, and silicone copolymers, mixtures of dimethicones and dimethiconols; phenyl-modified silicones, alkyl/alkoxy-modified silicones, polyether functional silicones, polyglycerin-modified silicones, polyether/alkyl-modified silicones, polyglycerin/alkyl-modified silicones, silicone cross-polymers, silicone resin, silicone resin gels, silicone polyglucosides, are available from a variety of commercial sources such as Dow Corning, Shin-Etsu, Wacker, General Electric Company, Momentive Performance Materials, and Lubrizol.

The silicones used in the present invention can have a particle or droplet size of from about 0.1 microns to about 300 microns, or from about 5 microns to about 80 microns, or from about 20 microns to about 60 microns, or even from about 30 microns to about 50 microns. Here, as well as elsewhere in the specification and claims, individual numerical values, or limits, can be combined to form additional non-disclosed and/or non-stated ranges.

In one aspect, the silicone agent(s) can be present in an amount of 0.001 wt. % to 40 wt. % in one aspect, from 0.01 wt. % to 20 wt. % in another aspect, and from 0.1 wt. % to 10 wt. % based on the total weight of the composition.

Conditioners

In addition to the silicone and quaternary (monomeric and polymeric) conditioners previously disclosed, exemplary conditioners include synthetic oil conditioners selected from hydrocarbon oils, polyolefins, e.g., poly-$\alpha$-olefins such as polybutenes, polyisobutenes and polydecenes. The polyolefins can be hydrogenated. Fluorinated or perfluorinated oils are also contemplated within the scope of the present invention. Fluorinated oils include perfluoropolyethers described in European Patent 0 486 135 and the fluorohydrocarbon compounds described in WO 93/11103. The fluoridated oils may also be fluorocarbons such as fluoramines, e.g., perfluorotributylamine, fluoridated hydrocarbons, such as perfluorodecahydronaphthalene, fluoroesters, and fluoroethers.

Suitable hydrocarbon oils for use as conditioning agents in the compositions of the present invention include, but are not limited to, hydrocarbon oils having at least about 10 carbon atoms, such as cyclic hydrocarbons, straight chain aliphatic hydrocarbons (saturated or unsaturated), and branched chain aliphatic hydrocarbons (saturated or unsaturated), including polymers and mixtures thereof. Straight chain hydrocarbon oils typically contain about 12 to 19 carbon atoms. Branched chain hydrocarbon oils, including hydrocarbon polymers, typically will contain more than 19 carbon atoms.

Specific non-limiting examples of these hydrocarbon oils include paraffin oil, mineral oil, saturated and unsaturated dodecane, saturated and unsaturated tridecane, saturated and unsaturated tetradecane, saturated and unsaturated pentadecane, saturated and unsaturated hexadecane, polybutene, polydecene, and mixtures thereof. Branched-chain isomers of these compounds, as well as of higher chain length hydrocarbons, can also be used, examples of which include highly branched, saturated or unsaturated, alkanes such as the permethyl-substituted isomers, e.g., the permethyl-substituted isomers of hexadecane and eicosane, such as 2,2,4,4,6,6,8,8-dimethyl-10-methylundecane and 2,2,4,4,6,6-dimethyl-8-methylnonane, available from Permethyl Corporation. Hydrocarbon polymers such as polybutene and polydecene. A preferred hydrocarbon polymer is polybutene, such as the copolymer of isobutylene and butene. A commercially available material of this type is L-14 polybutene from BP Chemical Company. Suitable natural oil conditioners include but are not limited to peanut, sesame, avocado, coconut, cocoa butter, almond, safflower, corn, cotton seed, sesame seed, walnut oil, castor, olive, jojoba, palm, palm kernel, soybean, wheat germ, linseed, sunflower seed; eucalyptus, lavender, vetiver, litsea, cubeba, lemon, sandalwood, rosemary, chamomile, savory, nutmeg, cinnamon, hyssop, caraway, orange, geranium, cade, and bergamot oils, fish oils, glycerol tricaprocaprylate; and mixtures thereof.

Suitable natural and synthetic wax conditioning agents include but are not limited to carnauba wax, candelila wax, alfa wax, paraffin wax, ozokerite wax, olive wax, rice wax, hydrogenated jojoba wax, bees wax, modified bees wax, e.g., cerabellina wax, marine waxes, polyolefin waxes, e.g., polyethylene wax; and mixtures thereof.

Other suitable organic conditioners for use as the conditioning agent in the compositions of the present invention include, but are not limited to, fatty esters having at least 10 carbon atoms. These fatty esters include esters derived from fatty acids or alcohols (e.g. mono-esters, polyhydric alcohol esters, and di- and tri-carboxylic acid esters). The fatty esters hereof may include or have covalently bonded thereto other compatible functionalities, such as amides and alkoxy moieties (e.g., ethoxy or ether linkages, etc.).

Exemplary fatty esters include, but are not limited to isopropyl isostearate, hexyl laurate, isohexyl laurate, isohexyl palmitate, isopropyl palmitate, decyl oleate, isodecyl oleate, hexadecyl stearate, decyl stearate, isopropyl isostearate, dihexyldecyl adipate, lauryl lactate, myristyl lactate, cetyl lactate, oleyl stearate, oleyl oleate, oleyl myristate, lauryl acetate, cetyl propionate, and oleyl adipate.

Other fatty esters suitable for use in the compositions of the present invention are mono-carboxylic acid esters of the general formula $R^{50}C(O)OR^{51}$, wherein $R^{50}$ and $R^{51}$ are alkyl or alkenyl radicals, and the sum of carbon atoms in $R^{50}$ and $R^{51}$ is at least 10 in one aspect, and at least 22 in another aspect of the invention.

Still other fatty esters suitable for use in the compositions of the present invention are di- and tri-alkyl and alkenyl esters of carboxylic acids, such as esters of $C_4$ to $C_8$ dicarboxylic acids (e.g. $C_1$ to $C_{22}$ esters, preferably $C_1$ to $C_6$, of succinic acid, glutaric acid, adipic acid). Specific non-limiting examples of di- and tri-alkyl and alkenyl esters of carboxylic acids include isocetyl stearyol stearate, diisopropyl adipate, and tristearyl citrate.

Other fatty esters suitable for use in the compositions of the present invention are those known as polyhydric alcohol esters. Such polyhydric alcohol esters include alkylene glycol esters, such as ethylene glycol mono and di-fatty acid esters, diethylene glycol mono- and di-fatty acid esters, polyethylene glycol mono- and di-fatty acid esters, propylene glycol mono- and di-fatty acid esters, polypropylene glycol monooleate, polypropylene glycol 2000 monostearate, ethoxylated propylene glycol monostearate, glyceryl mono- and di-fatty acid esters, polyglycerol poly-fatty acid esters, ethoxylated glyceryl monostearate, 1,3-butylene glycol monostearate, 1,3-butylene glycol distearate, polyoxyethylene polyol fatty acid ester, sorbitan fatty acid esters, and polyoxyethylene sorbitan fatty acid esters.

In one aspect, the conditioning agent(s) can be present in an amount of 0.001 wt. % to 40 wt. % in one aspect, from 0.01 wt. % to 20 wt. % in another aspect, and from 0.1 wt. % to 10 wt. % based on the total weight of the composition.

Emulsifiers

Exemplary emulsifiers include but are not limited to $C_{12}$-$C_{18}$ fatty alcohols; alkoxylated $C_{12}$-$C_{18}$ fatty alcohols;$C_{12}$-$C_{18}$ fatty acids; and alkoxylated $C_{12}$-$C_{18}$ fatty acids, the alkoxylates each having 10 to 30 units of ethylene oxide, propylene oxide, and combinations of ethylene oxide/propylene oxide; $C_8$-$C_{22}$ alkyl mono- and oligoglycosides; ethoxylated sterols; partial esters of polyglycerols; esters and partial esters of polyols having 2 to 6 carbon atoms and saturated and unsaturated fatty acids having 12 to 30 carbon atoms; partial esters of polyglycerols; and organosiloxanes; and combinations thereof.

The fatty alcohols, acids and alkoxylated fatty alcohols and fatty acids are as described in the emollient description above. In one aspect of the invention the fatty alcohols and fatty acids each are ethoxylated with 10 to 30 units of ethylene oxide.

The $C_8$-$C_{22}$ alkyl mono- and oligoglycoside emulsifiers are prepared by reacting glucose or an oligosaccharide with primary fatty alcohols having 8 to 22 carbon atoms. Products which are obtainable under the trademark Plantacare® comprise a glucosidically bonded $C_8$-$C_{16}$ alkyl group on an oligoglucoside residue whose average degree of oligomerization is 1 to 2. Exemplary alkyl glucosides and oligoglycosides are selected from octyl glucoside, decyl glucoside, lauryl glucoside, palmityl glucoside, isostearyl glucoside, stearyl glucoside, arachidyl glucoside and behenyl glucoside,

and mixtures thereof.

Exemplary ethoxylated sterols include ethoxylated vegetable oil sterols such as, for example, soya sterols. The degree of ethoxylation is greater than about 5 in one aspect, and at least about 10 in another aspect. Suitable ethoxylated sterols are PEG-10 Soy Sterol, PEG-16 Soy Sterol and PEG-25 Soy Sterol.

The partial esters of polyglycerols have 2 to 10 glycerol units and are esterified with 1 to 4 saturated or unsaturated, linear or branched, optionally hydroxylated $C_8$-$C_{30}$ fatty acid residues. Representative partial esters of polyglycerols include diglycerol monocaprylate, diglycerol monocaprate, diglycerol monolaurate, triglycerol monocaprylate, triglycerol monocaprate, triglycerol monolaurate, tetraglycerol monocaprylate, tetraglycerol monocaprate, tetraglycerol monolaurate, pentaglycerol monocaprylate, pentaglycerol monocaprate, pentaglycerol monolaurate, hexaglycerol monocaprylate, hexaglycerol monocaprate, hexaglycerol monolaurate, hexaglycerol monomyristate, hexaglycerol monostearate, decaglycerol monocaprylate, decaglycerol monocaprate, decaglycerol monolaurate, decaglycerol monomyristate, decaglycerol monoisostearate, decaglycerol monostearate, decaglycerol monooleate, decaglycerol monohydroxystearate, decaglycerol dicaprylate, decaglycerol dicaprate, decaglycerol dilaurate, decaglycerol dimyristate, decaglycerol diisostearate, decaglycerol distearate, decaglycerol dioleate, decaglycerol dihydroxystearate, decaglycerol tricaprylate, decaglycerol tricaprate, decaglycerol trilaurate, decaglycerol trimyristate, decaglycerol triisostearate, decaglycerol tristearate, decaglycerol trioleate, decaglycerol trihydroxystearate, and mixtures thereof.

The saturated $C_{12}$-$C_{30}$ fatty alcohol emulsifiers are as described in the emollient description set forth above. In one aspect of the invention, the fatty alcohol emulsifier is selected from but not limited to cetyl alcohol, stearyl alcohol, arachidyl alcohol, behenyl alcohol and lanolin alcohol or mixtures of these alcohols, and as are obtainable in the hydrogenation of unsaturated vegetable oil and animal fatty acids.

Emulsifiers based on the esters and partial esters of polyols having 2 to 6 carbon atoms and linear saturated and unsaturated fatty acids having 12 to 30 carbon atoms are, for example, the monoesters and diesters of glycerol or ethylene glycol or the monoesters of propylene glycol with saturated and unsaturated $C_{12}$ to $C_{30}$ fatty acids.

The partially esterified polyglycerol emulsifiers include 2 to about 10 glycerol units and esterified with 1 to 5 saturated or unsaturated, linear or branched, optionally hydroxylated $C_8$ to $C_{30}$ fatty acid residues.

The organosiloxane emulsifiers are polymeric emulsifiers that contain at least one hydrophobic portion and at least one hydrophilic portion. The polymer backbone contains repeating siloxy units that can have cyclic, linear or branched repeating units, e.g. di($C_1$-$C_5$)alkylsiloxy units, typically dimethylsiloxy units.

The hydrophilic portion of the organosiloxane is generally achieved by substitution onto the polymeric backbone of a residue that confers hydrophilic properties to a portion of the molecule. The hydrophilic residue may be substituted on a terminus of the polymeric organosiloxane, or on any one or more repeating units of the polymer. Generally, the hydrophilic residue is derived from ethylene oxide units that are grafted onto the polymer backbone. In general, the repeating dimethylsiloxy units of modified polydimethylsiloxane emulsifiers are hydrophobic in nature due to the methyl groups, and confer the hydrophobicity properties to the molecule. In addition, longer chain alkyl residues, hydroxy terminated polypropyleneoxy residues, hydroxy terminated polyether residues comprising a combination of ethylene oxide and propylene oxide residues, and/or other types of residues can be substituted onto the siloxy backbone to confer additional emulsification properties to the backbone. Polyether substituted organosiloxane emulsifiers are known as dimethicone copolyols and are widely commercially available. The dimethicone polyols can be random or block copolymers. A generally useful class of dimethicone polyols is block copolymers having blocks of polydimethylsiloxane and blocks of polyalkylene oxide, such as blocks of polyethylene oxide, polypropylene oxide, or both.

In one aspect, the emulsifying agent(s) can be present in an amount of 0.001 wt. % to 40 wt. % in one aspect, from 0.01 wt. % to 20 wt. % in another aspect, and from 0.1 wt. % to 10 wt. % based on the total weight of the composition.

Emollients

Suitable emollients include but are not limited to an emollient selected from silicone fluids (e.g., volatile silicone oils and non-volatile silicone oils); mineral oils; petrolatums; vegetable oils; fish oils; fatty alcohols; fatty acids; fatty acid and fatty alcohol esters; alkoxylated fatty alcohols; alkoxylated fatty acid esters; benzoate esters; panthenol; Guerbet esters; alkyl ether derivatives of polyethylene glycols, such as, for example methoxypolyethylene glycol (MPEG); and polyalkylene glycols; lanolin and lanolin derivatives; and the like. The emollient can be used alone or in combination with one or more emollients of the present invention.

Volatile silicone oils include cyclic and linear polydimethylsiloxanes, low molecular weight organo-functional silicones, and the like. Cyclic volatile silicones (cyclomethicones) typically contain about 3 to about 7 silicon atoms, alternating with oxygen atoms, in a cyclic ring structure. Each silicon atom is typically substituted with two alkyl groups, such as, for example, methyl groups. Volatile linear polydimethylsiloxanes (dimethicones) typically contain about 2 to about 9 silicon atoms, alternating with oxygen atoms in a linear arrangement. Each silicon atom is also substituted with two alkyl groups (the terminal silicon atoms are substituted with three alkyl groups), such as, for example, methyl groups. The linear volatile silicones typically have viscosities of less than about 5 mPa·s at 25°C., while the cyclic volatile silicones

typically have viscosities of less than about 10 mPa·s at 25°C. "Volatile" means that the silicone has a measurable vapor pressure, or a vapor pressure of at least 2 mm of Hg at 20°C. Non-volatile silicones have a vapor pressure of less than 2 mm Hg at 20°C. A description of volatile silicones is found in Todd and Byers, "Volatile Silicone Fluids for Cosmetic Formulations", Cosmetics and Toiletries, Vol. 91, pp. 29-32 (January 1976), and in Kasprzak, "Volatile Silicones", Soap/Cosmetics/Chemical Specialties, pp. 40-43 (December 1986), each incorporated herein by reference.

Exemplary volatile cyclomethicones are D4 cyclomethicone (octamethylcyclotetrasiloxane), D5 cyclomethicone (decamethylcyclopentasiloxane), D6 cyclomethicone, and blends thereof (e.g., D4/D5 and D5/D6). Volatile cyclomethicones and cyclomethicone blends are commercially available from G.E. Silicones as SF1173, SF1202, SF1256, and SF1258, Dow Corning Corporation as Dow Corning® 244, 245, 246, 345, and 1401 Fluids. Blends of volatile cyclomethicones and volatile linear dimethicones are also contemplated.

Exemplary volatile linear dimethicones include hexamethyldisiloxane, octamethyltrisiloxane, decamethyltetrasiloxane, dodecamethylpentasiloxane and blends thereof. Volatile linear dimethicones and dimethicone blends are commercially available from Dow Corning Corporation as Dow Corning 200® Fluid (e.g., product designations 0.65 CST, 1 CST, 1.5 CST, and 2 CST) and Dow Corning® 2-1184 Fluid.

Exemplary volatile low molecular weight organo-functional silicones include phenyl trimethicone, caprylyl trimethicone, caprylyl methicone, and hexyl methicone, and blends thereof. Low molecular weight organo-functional silicones are commercially available from Clariant under the trade name Silcare® 41M10, Silcare® 31 M60, Silcare® 41M10, and Silcare® 41 M15.

The non-volatile silicone oils useful as emollients in the present invention are linear and typically have viscosities of from about 10 mPa·s to about 100,000 mPa·s at 25°C. They typically contain above about 10 dialkyl/diaryl or monoalkyl/monoaryl substituted silicon atoms, alternating with oxygen atoms in a linear arrangement. They include polyalkylsiloxane, polyarylsiloxane, and polyalkylarylsiloxane polymers. Exemplary non-volatile silicone oils include the polydimethylsiloxanes (dimethicones), polydiethylsiloxanes, polymethylphenylsiloxanes, and the like. In one aspect of the invention, the non-volatile silicone oil is selected from a non-volatile polydimethylsiloxane having a viscosity range from about 10 mPa·s to about 100,000 mPa·s at 25°C. Non-volatile dimethicones are commercially available from Dow Corning Corporation as Dow Corning 200® Fluid (product designations 10 CST through 10,000 CST).

Mineral oils and petrolatums include cosmetic, USP and NF grades and are commercially available from Penreco under the Drakeol® and Penreco® trade names. Mineral oil includes hexadecane and paraffin oil.

Exemplary vegetable oils suitable an emollient component in the present invention include but are not limited to peanut oil, sesame oil, avocado oil, coconut oil, cocoa butter, almond oil, safflower oil, corn oil, cotton seed oil, sesame seed oil, walnut oil, castor oil, olive oil, jojoba oil, palm oil, palm kernel oil, soybean oil, wheat germ oil, linseed oil, sunflower seed oil; and the mono-, di-, and triglycerides thereof. Exemplary mono-, di- and triglycerides are, for example, caprylic triglyceride, capric triglyceride, caprylic/capric triglyceride, and caprylic/capric/lauric triglyceride, caprylic/capric/stearic triglyceride, and caprylic/capric/linoleic triglyceride.

Ethoxylated mono- and diglycerides are also suitable as an emollient component of the present invention, such as, for example, PEG-8 Caprylic/Capric Glycerides.

Suitable fatty alcohol emollients include but are not limited to fatty alcohols containing 8 to 30 carbon atoms. Exemplary fatty alcohols include capryl alcohol, pelargonic alcohol, capric alcohol, lauryl alcohol, myristyl alcohol, cetyl alcohol, isocetyl alcohol, stearyl alcohol, isostearyl alcohol, cetearyl alcohol, oleyl alcohol, ricinoleyl alcohol, arachidyl alcohol, icocenyl alcohol, behenyl alcohol, and mixtures thereof.

Suitable fatty acid emollients include but are not limited to fatty acids containing 10 to 30 carbon atoms. Exemplary fatty acids are selected from capric acid, lauric acid, myristic acid, palmitic acid, stearic acid, oleic acid, linoleic acid, arachidic acid, behenic acid, and mixtures thereof.

Suitable fatty acid and fatty alcohol ester emollients include but are not limited to hexyl laurate, decyl oleate, isopropyl stearate, isopropyl isostearate, butyl stearate, octyl stearate, cetyl stearate, myristyl myristate, octyldodecyl stearoylstearate, octylhydroxystearate, diisopropyl adipate, isopropyl myristate, isopropyl palmitate, ethyl hexyl palmitate, isodecyl oleate, isodecyl neopentanoate, diisopropyl sebacate, isostearyl lactate, lauryl lactate, diethyl hexyl maleate, PPG-14 butyl ether and PPG-2 myristyl ether propionate, cetearyl octanoate, and mixtures thereof.

Alkoxylated fatty alcohols are ethers formed from the reaction of a fatty alcohol with an alkylene oxide, generally ethylene oxide or propylene oxide. Suitable ethoxylated fatty alcohols are adducts of fatty alcohols and polyethylene oxide. In one aspect of the invention, the ethoxylated fatty alcohols can be represented by the formula $R-(OCH_2CH_2)_n-OH$, wherein R represents the aliphatic residue of the parent fatty alcohol and n represents the number of molecules of ethylene oxide. In another aspect of the invention, R is derived from a fatty alcohol containing 8 to 30 carbon atoms. In one aspect, n is an integer ranging from 2 to 50, 3 to 25 in another aspect, and 3 to 10 in a further aspect. In a still further aspect, R is derived from a fatty alcohol emollient set forth above. Exemplary ethoxylated fatty alcohols are but are not limited to capryl alcohol ethoxylate, lauryl alcohol ethoxylate, myristyl alcohol ethoxylate, cetyl alcohol ethoxylate, stearyl alcohol ethoxylate, cetearyl alcohol ethoxylate oleyl alcohol ethoxylate, and, behenyl alcohol ethoxylate, wherein the number of ethylene oxide units in each of the foregoing ethoxylates can range from 2 and above in one aspect, and

from 2 to about 150 in another aspect. It is to be recognized that the propoxylated adducts of the foregoing fatty alcohols and mixed ethoxylated/propoxylated adducts of the foregoing fatty alcohols are also contemplated within the scope of the invention. The ethylene oxide and propylene oxide units of the ethoxylated/propoxylated fatty alcohols can be arranged in random or in blocky order.

More specific examples of ethoxylated alcohols are but are not limited to Beheneth 5-30 (the 5-30 meaning the range of repeating ethylene oxide units), Ceteareth 2-100, Ceteth 1-45, Cetoleth 24-25, Choleth 10-24, Coceth 3-10, C9-11 pareth 3-8, C11-15 pareth 5-40, C11-21 Pareth 3-10, C12-13 pareth 3-15, Deceth 4-6, Dodoxynol 5-12, Glycereth 7-26, Isoceteth 10-30, Isodeceth 4-6, Isolaureth 3-6, isosteareth 3-50, Laneth 5-75, Laureth 1-40, Nonoxynol 1-120, Nonylnonoxynol 5-150, Octoxynol 3-70, Oleth 2-50, PEG 4-350, Steareth 2-100, and Trideceth 2-10.

Specific examples of propoxylated alcohols are but are not limited to PPG-10 Cetyl Ether, PPG-20 Cetyl Ether, PPG-28 Cetyl Ether, PPG-30 Cetyl Ether, PPG-50 Cetyl Ether, PPG-2 Lanolin Alcohol Ether, PPG-5 Lanolin Alcohol Ether, PPG-10 Lanolin Alcohol Ether, PPG-20 Lanolin Alcohol Ether, PPG-30 Lanolin Alcohol Ether, PPG-4 Lauryl Ether, PPG-7 Lauryl Ether, PPG-10 Oleyl Ether, PPG-20 Oleyl Ether, PPG-23 Oleyl Ether, PPG-30 Oleyl Ether, PPG-37 Oleyl Ether, PPG-50 Oleyl Ether, PPG-11 Stearyl Ether, PPG-15 Stearyl Ether, PPG-2 Lanolin Ether, PPG-5 Lanolin Ether, PPG-10 Lanolin Ether, PPG-20 Lanolin Ether, PPG-30 Lanolin Ether, and PPG-1 Myristyl Ether.

Specific examples of ethoxylated/propoxylated alcohols are but are not limited to PPG-1 Beheneth-15, PPG-12 Capryleth-18, PPG-2-Ceteareth-9, PPG-4-Ceteareth-12, PPG-10-Ceteareth-20, PPG-1-Ceteth-1, PPG-1-Ceteth-5, PPG-1-Ceteth-10, PPG-1-Ceteth-20, PPG-2-Ceteth-1, PPG-2-Ceteth-5, PPG-2-Ceteth-10, PPG-2-Ceteth-20, PPG-4-Ceteth-1, PPG-4-Ceteth-5, PPG-4-Ceteth-10, PPG-4-Ceteth-20, PPG-5-Ceteth-20, PPG-8-Ceteth-1, PPG-8-Ceteth-2, PPG-8-Ceteth-5, PPG-8-Ceteth-10, PPG-8-Ceteth-20, PPG-2 C12-13 Pareth-8, PPG-2 C12-15 Pareth-6, PPG-4 C13-15 Pareth-15, PPG-5 C9-15 Pareth-6, PPG-6 C9-11 Pareth-5, PPG-6 C12-15 Pareth-12, PPG-6 C12-18 Pareth-11, PPG-3 C12-14 Sec-Pareth-7, PPG-4 C12-14 Sec-Pareth-5, PPG-5 C12-14 Sec-Pareth-7, PPG-5 C12-14 Sec-Pareth-9, PPG-1-Deceth-6, PPG-2-Deceth-3, PPG-2-Deceth-5, PPG-2-Deceth-7, PPG-2-Deceth-10, PPG-2-Deceth-12, PPG-2-Deceth-15, PPG-2-Deceth-20, PPG-2-Deceth-30, PPG-2-Deceth-40, PPG-2-Deceth-50, PPG-2-Deceth-60, PPG-4-Deceth-4, PPG-4-Deceth-6, PPG-6-Deceth-4, PPG-6-Deceth-9, PPG-8-Deceth-6, PPG-14-Deceth-6, PPG-6-Decyltetradeceth-12, PPG-6-Decyltetradeceth-20, PPG-6-Decyltetradeceth-30, PPG-13-Decyltetradeceth-24, PPG-20-Decyltetradeceth-10, PPG-2-Isodeceth-4, PPG-2-Isodeceth-6, PPG-2-Isodeceth-8, PPG-2-Isodeceth-9, PPG-2-Isodeceth-10, PPG-2-Isodeceth-12, PPG-2-Isodeceth-18, PPG-2-Isodeceth-25, PPG-4-Isodeceth-10, PPG-12-Laneth-50, PPG-2-Laureth-5, PPG-2-Laureth-8, PPG-2-Laureth-12, PPG-3-Laureth-8, PPG-3-Laureth-9, PPG-3-Laureth-10, PPG-3-Laureth-12, PPG-4 Laureth-2, PPG-4 Laureth-5, PPG-4 Laureth-7, PPG-4-Laureth-15, PPG-5-Laureth-5, PPG-6-Laureth-3, PPG-25-Laureth-25, PPG-7 Lauryl Ether, PPG-3-Myreth-3, PPG-3-Myreth-11, PPG-20-PEG-20 Hydrogenated Lanolin, PPG-2-PEG-11 Hydrogenated Lauryl Alcohol Ether, PPG-12-PEG-50 Lanolin, PPG-12-PEG-65 Lanolin Oil, PPG-40-PEG-60 Lanolin Oil, PPG-1-PEG-9 Lauryl Glycol Ether, PPG-3-PEG-6 Oleyl Ether, PPG-23-Steareth-34, PPG-30 Steareth-4, PPG-34-Steareth-3, PPG-38 Steareth-6, PPG-1 Trideceth-6, PPG-4 Trideceth-6, and PPG-6 Trideceth-8.

Alkoxylated fatty acids are formed when a fatty acid is reacted with an alkylene oxide or with a pre-formed polymeric ether. The resulting product may be a monoester, diester, or mixture thereof. Suitable ethoxylated fatty acid ester emollients suitable for use in the present invention are products of the addition of ethylene oxide to fatty acids. The product is a polyethylene oxide ester of a fatty acid. In one aspect of the invention, the ethoxylated fatty acid esters can be represented by the formula $R\text{-}C(O)O(CH_2CH_2O)_n\text{-}H$, wherein R represents the aliphatic residue of a fatty acid and n represents the number of molecules of ethylene oxide. In another aspect, n is an integer ranging from 2 to 50, 3 to 25 in another aspect, and 3 to 10 in a further aspect. In still another aspect of the invention, R is derived from a fatty acid containing 8 to 24 carbon atoms. In a still further aspect, R is derived from a fatty acid emollient set forth above. It is to be recognized that propoxylated and ethoxylated/propoxylated products of the foregoing fatty acids are also contemplated within the scope of the invention. Exemplary alkoxylated fatty acid esters include but are not limited to capric acid ethoxylate, lauric acid ethoxylate, myristic acid ethoxylate, stearic acid ethoxylate, oleic acid ethoxylate, coconut fatty acid ethoxylate, and polyethylene glycol 400 propoxylated monolaurate, wherein the number of ethylene oxide units in each of the foregoing ethoxylates can range from 2 and above in one aspect, and from 2 to about 50 in another aspect. More specific examples of ethoxylated fatty acids are PEG-8 distearate (the 8 meaning the number of repeating ethylene oxide units), PEG-8 behenate, PEG-8 caprate, PEG-8 caprylate, PEG-8 caprylate/caprate, PEG cocoates (PEG without a number designation meaning that the number of ethylene oxide units ranges from 2 to 50), PEG-15 dicocoate, PEG-2 diisononanoate, PEG-8 diisostearate, PEG-dilaurates, PEG-dioleates PEG-distearates, PEG Ditallates, PEG-isostearates, PEG-jojoba acids, PEG-laurates, PEG-linolenates, PEG-myristates, PEG-oleates, PEG-palmitates, PEG-ricinoleates, PEG-stearates, PEG-tallates, and the like.

Guerbet ester emollients are formed from the esterification reaction of a Guerbet alcohol with a carboxylic acid. Guerbet ester emollients are commercially available from the Noveon Consumer Specialties Division of Lubrizol Advanced Materials, Inc. under product designations G-20, G-36, G-38, and G-66.

Lanolin and lanolin derivatives are selected from lanolin, lanolin wax, lanolin oil, lanolin alcohols, lanolin fatty

acids, alkoxylated lanolin, isopropyl lanolate, acetylated lanolin alcohols, and combinations thereof. Lanolin and lanolin derivatives are commercially available from the Noveon Consumer Specialties Division of Lubrizol Advanced Materials, Inc. under the trade names Lanolin LP 108 USP, Lanolin USP AAA, Acetulan™, Ceralan™, Lanocerin™, Lanogel™ (product designations 21 and 41), Lanogene™, Modulan™, Ohlan™, Solulan™ (product designations 16, 75, L-575, 98, and C-24), Vilvanolin™ (product designations C, CAB, L-101, and P).

Hydrophobically modified alkoxylated methyl glucosides, such as, for example, PEG-120 Methyl Glucose Dioleate, PEG-120 Methyl Glucose Trioleate, and PEG-20 Methyl Glucose Sesquistearate, available from Lubrizol Advanced Materials, Inc., under the trade names Glucamate® DOE-120, Glucamate™ LT, and Glucaniate™ SSE-20, respectively, are also suitable non-ionic surfactants. Other suitable hydrophobically modified alkoxylated methyl glucosides are disclosed in U.S. Patent Nos. 6,573,375 and 6,727,357, the disclosures of which are hereby incorporated by reference in their entirety.

The emollient(s) can be utilized individually or in combination in an amount ranging from about 0.5 wt. % to about 30 wt. % by weight of the total personal care composition in one aspect 0.1 wt. % to 25 wt. % in another aspect, and 5 wt. % to 20 wt. % in a further aspect. While emollients are generally employed in personal care compositions, they can be employed in home care, health care, and institutional care compositions in the same wt. ratios as set forth for personal care compositions so long as they effect a desired physical attribute (e.g., humectant properties) in such compositions.

While overlapping weight ranges for the various components and ingredients that can be contained in the compositions of the invention have been expressed for selected embodiments and aspects of the invention, it should be readily apparent that the specific amount of each component in the disclosed personal care, home care, health care, and institutional care compositions will be selected from its disclosed range such that the amount of each component is adjusted such that the sum of all components in the composition will total 100 wt. %. The amounts employed will vary with the purpose and character of the desired product and can be readily determined by one skilled in the formulation arts and from the literature.

It is also to be recognized that the choice and amount of ingredients in personal care, home care, health care and institutional care compositions that include the multi-purpose polymers can vary depending on the intended product and its function, as is well known to those skilled in the formulation arts. An extensive listing of ingredients and their conventional functions and product categories have been disclosed and can be readily ascertained from the literature, some of which can serve more than one function and that one or more of the disclosed ingredients can be combined with the multi-purpose polymers to obtain a desired personal care, home care, health care and institutional care composition.

Other oily materials that are useful in combination with the polymers include, for example, acetylated lanolin alcohols; lanolin alcohol concentrates; esters of lanolin fatty acids such as the isopropyl esters of lanolin fatty acid; polyol fatty acids; ethoxylated alcohols, such as ethoxylate and castor oils; sterols; sterol esters; sterol ethoxylates; and like materials. Many of such esters and ethoxylates are also useful as non-ionic surfactants.

Numerous ingredients are known in the art as conditioning agents for hair or skin, and humectants, and in addition to those previously discussed, non-limiting examples include PCA (DL-pyrrolidone carboxylic acid) and its salts, such as lysine PCA, aluminum PCA, copper PCA, chitosan PCA, and the like, allantoin; urea; hyaluronic acid and its salts; ceramides; sorbic acid and its salts; sugars and starches and derivatives thereof; lactamide MEA; and the like.

**Methods:**

Viscosity: The reported viscosity of each polymer containing composition is measured in milli-Pascal seconds (mPa·s) (cP), employing a Brookfield rotating spindle viscometer (Brookfield Engineering Laboratories, Inc., Model RVT) at 20 revolutions per minute (rpm), at ambient room temperature of about 20°C to about 25°C (referred to as Brookfield viscosity). Spindle sizes utilized for a particular viscosity measurement are selected according to viscosity tables supplied by the manufacturer.

A "thin or low viscosity" typically refers to a pourable, runny product having a viscosity of up to about 1,000 mPa·s a "medium viscosity" refers to a product having a viscosity in the range of above 1,000 to about 3,000 mPa·s; a "high viscosity" refers to a product having a viscosity in the range of above 3,000 to about 10,000 mPa·s; and "gel" refers to a product having a viscosity greater than 10,000 mPa·s, unless otherwise indicated.

B. Yield Value: Yield value is defined as the initial resistance to flow under stress. It is measured by the Brookfield Yield Value (BYV) Extrapolation Method using a Brookfield viscometer (Model RVT). The Brookfield viscometer is used to measure the torque necessary to rotate a spindle through a liquid sample at speeds of 0.5 to 100 rpm. Multiplying the torque reading by the appropriate constant for the spindle and speed gives the apparent viscosity. Yield Value is an extrapolation of measured values to a shear rate of zero. The BYV is calculated by the following equation:

$$BYV,\ dyn/cm^2 = (\eta_{\alpha 1} - \eta_{\alpha 2})/100$$

where $\eta_{\alpha 1}$ and $\eta_{\alpha 2}$ = apparent viscosities obtained at two different spindle speeds (0.5 rpm and 1.0 rpm, respectively). These techniques and the usefulness of the Yield Value measurement are explained in Technical Data Sheet Number 244 (Revision: 5/98) from the Noveon Consumer Specialties Division of Lubrizol Advanced Materials, Inc., herein incorporated by reference. Low yield values (<50 dyns/$cm^2$) are indicative of smooth and Newtonian-like flow properties.

Clarity: When reported, the clarity of the polymer-containing composition is measured in % T (transmittance) by a Brinkmann PC 920 colorimeter at least about 24 hours after the composition is made. Clarity measurements are taken against deionized water (clarity rating of 100 percent). Compositions having a clarity of about 60 percent or more are substantially clear; compositions having a clarity in the range of about 45 percent to 59 percent are judged substantially translucent.

D. Turbidity: When reported, the turbidity of a polymer-containing composition is determined in Nephelometric Turbidity Units (NTU) employing a Nephelometric turbidity meter with distilled water (NTU = 0) as the standard. Compositions having an NTU value of about 90 or greater are judged turbid.

E. Stability: The stability of the polymer product emulsion or formulated composition is evaluated by one or more of the following procedures.

Shelf Storage: A sample of test product is stored at one or more of the following temperatures: (a) at ambient room temperature in the range of about 20°C to about 25°C for a period of at least one week and up to about six months; (b) at elevated temperature in an oven at a selected temperature in the range of about 30°C to about 50°C (unless otherwise indicated) for a period of up to about 5 weeks (accelerated aging storage).

Stability is determined by periodically visually observing the stored sample for visible sedimentation or a noticeable increase in measurable Brookfield viscosity determined at a selected interval as indicated in the following examples. At ambient temperature storage, the sample is visually checked daily for one week, then biweekly during a total storage period of about two months and monthly thereafter during a total storage period of up to about six months. Under either storage temperature, compositions are judged stable if: (a) no sedimentation is observed, or if some sedimentation occurs and it is not more than about 2 percent of the total volume of the sample; and (b) the product viscosity did not increase, or if an increase occurs, the increase is not more than about 100 mPa·s.

F. Amine Hydrolysis by Acid-Base Titration: An acid-base titration is performed for each monomer to follow its hydrolytic stability periodically for 1 or 2 weeks in an excess amount of water at 1:1 weight ratio (1:10 mole ratio). A 100 percent hydrolysis of amine monomer is expected to yield 1 mole of amine alcohol and 1 mole of methacrylic acid which can be measured by acid-base titration (acid number, mg KOH/g).

G. Suspension Testing Procedure: The ability of a polymer system to suspend active and/or aesthetically pleasing insoluble oily and particulate materials is important from the standpoint of product efficacy and appeal. Suspension ability is tested using the clear bath gel samples as formulated in Table 4. A six dram vial (approximately 70 mm high X 25 mm in diameter) is filled to the 50 mm mark with a bath gel test formulation. Aesthetic beads (Unispheres UEA-509, available from Induchem AG, Switzerland, composition: lactose, cellulose, hydroxypropyl methylcellulose) are dispersed throughout the bath gel sample and photographed to establish the initial position of the beads in the gel. The vials are placed in a 45°C oven to age for a 12 week period. At the conclusion of the 12 week oven test period the samples are checked for bead suspension properties. The suspension results are visually ranked using a scale of 4 to 0 where: 4 = no noticeable settling relative to the initial bead position in the gel, 3 = slight settling or less than approximately ¼ drop in distance relative to the initial bead position in the gel, 2 = approximately ¼ drop in distance relative to the initial position in the gel, 1 = greater than ¼ drop to ½ drop in distance relative to the initial position in the bath gel, and 0 = greater than ½ drop in distance relative to the intial position in the bath gel.

H. Mannequin Head Test Procedure: This test is conducted to determine the styling efficacy of a fixative formulation containing a multi-purpose polymer of the invention. Plastic mannequin heads implanted with natural human hair (Asian variety) are styled and evaluated according to the following procedure.

The hair on the crown area (approximately 12 cm x 12 cm) of a mannequin head is cut to a length of 3 to 4 inches. Each mannequin head is prepared prior to the application of the fixative formulation by shampooing the crown area hair with a detersive surfactant solution (10% sodium lauryl sulfate (30% active) in aqueous solution) followed by rinsing with tap water. The hair is combed during the shampooing and rinsing steps. A sample of a fixative formulation weighing 3.5 to 3.6 g is evenly applied to the wet hair on the washed and rinsed crown area of the mannequin head.

The sample fixative formulation is worked into the hair by hand and then combed through several times to ensure that the fixative is spread evenly. Using the comb, the hair is pulled upwards from the mannequin head to spike it. The hair is allowed to dry overnight at about 50% relative humidity. The hair set is manually observed for stiffness and flexibility by touch and feel by hand.

I. Wet Combability Test Procedure: The ability of shampoo fromulations to condition hair is determined by a

wet combability test using a Dia-Stron Miniature Tensile Tester (Model MTT 170, Dia-Stron Limited, England) networked to a personal computer loaded with a software application (Uvwin version 1.33) that records and calculates the maximum combing force curve (gram force) required to comb through a treated hair tress. The total combing work (Joules) is calculated by the software which integrates the area under the plotted curve.

Conditioning efficacy is determined by comparing the difference of the combing force in hair tresses before and after treatment with a 2-in-1 conditioning shampoo formulated with the multi-purpose polymers. The measurement is performed on European brown hair tresses. The hair tresses (2.5 g/tress) are pre-washed with a surfactant iso-propanol mixture (10% sodium lauryl sulfate and 10% iso-propanol) and thoroughly rinsed. Each tress is then washed with surfactant blank containing 12% sodium laureth sulfate and 2% cocamidoproyl betaine. After rinsing, the tress is combed along its length several times to remove major tangles and then slowly immersed in water. Excess water is removed by pinching each tress between the index finger and the middle finger and gently pulling the tress through the gap of the fingers. Each untreated tress is mounted and combed through by the comb element of the tester at a rate of 2000 mm/min. After the untreated tresses are combed to obtain the control value, the tresses are wetted with flowing tap water at 38.5°C. Excess water is removed using the two finger method as previously described. Two-in-one conditioning shampoos (0.25 g) formulated with the multi-purpose polymers of the invention are applied to each hair tress and gently lathered in for 1 min. and subsequently rinsed under flowing tap water (0.75gal/min.) at 40°C for 1 min. The tresses are shampooed a second time and rinsed as previously described. The wet combability test is performed on the tresses and the combing work is calculated. The difference in the value of the combing force of the group of wetted tresses before and after treatment with the 2-in-1 conditioning shampoos containing the multi-purpose polymers of the invention reflects the conditioning efficacy of the hair. The data are expressed as the percent reduction in combing work of individual hair tresses as a result of the treatment.

J. Silicone Deposition Measurement: The amount of silicone (silicon atoms) deposited on hair tress samples from a 2-in-1 shampoo composition containing the multi-purpose polymers is measured by X-Ray fluorescence (XRF) spectroscopy. A wavelength dispersive XRF spectrometer (PANalytical Axios Advanced Sequential 4 kW spectrometer - Model Number PW4400) interfaced with SuperQ 4 software application and fitted with a rhodium tube with an InSb crystal is utilized to facilitate high sensitivity for silicon atom detection corresponding to Si K alpha band . The samples are analyzed using a qualitative program to measure intensities across a two-theta scan range from 139.75° to 147.99° with a peak maxima at 144.53°. The samples are scanned in a vacuum environment using a tube voltage of 25 kV and a current of 160 mA. Scanning speed is 0.05 °2-Theta/sec. with 0.02 °2-Theta step size.

X-rays from the instrument excite silicon atoms deposited on the surface of the hair tress causing them to emit energy and fluoresce. The silicone fluorescence is detected and recorded as counts per second. Higher count rates are indicative of higher silicon atom deposition. The amount of silicon atoms detected is directly proportional to the amount of silicone conditioner deposited on the hair.

European brown hair tresses (2.5 gm/tress) are pre-washed with a surfactant/iso-propanol mixture (10% sodium lauryl sulfate and 10% iso-propanol) and thoroughly rinsed. Two-in-one conditioning shampoos (0.25 g) formulated with the multi-purpose polymers are applied to each hair tress and gently lathered in for 1 min. and subsequently rinsed under flowing tap water (0.75 gal/min.) at 40°C for 1 min. The tresses are shampooed a second time and rinsed as previously described. After rinsing, the treated tresses are dried at room temperature. Samples for XRF analysis are prepared by cutting each treated hair tress into ½ in. lengths and placing the cut lengths into a sample cup having a 6 $\mu$ thick polyethylene support substrate formed into the bottom. A polyethylene spacer is placed on each cut tress to hold it onto the substrate.

K. Molecular Weight Determination: The weight average molecular weights referenced herein are measured by GPC using a PL-GPC 220 high temperature GPC instrument with RI detector manufactured by Polymer Laboratories (Varian, Inc.). Approximately 0.02 g polymer sample is dissolved in 5 ml of tetrahydrofuran (THF), containing 250 ppm of butylated hydroxytoluene (BHT) and 0.05 molar $NaNO_3$. The test sample solution is gently shaken for about two hours and filtered by passing the sample solution through a 0.45 $\mu$m PTFE disposable disc filter. The chromatographic conditions are: Mobile phase: THF, with 250 ppm BHT and 1.0% acetic acid, 70° C, 1.0 ml/min. Sample size: 100$\mu$l Column set: PLgel (Guard + 2 x Mixed-C), all 5$\mu$m, in series. Waters Empower Pro LC/GPC software is used to analyze the results and to calculate $M_w$ of the polymers. A polystyrene standard is used.

**Examples:**

The following examples further illustrate the preparation and use of embodiments but are not intended to be limiting. The following is a list of material abbreviations and Trade Names utilized in the specification.

| | |
|---|---|
| Control 3 | INCI Name: Polyacrylate-1 Crosspolymer |
| ACE | ACE™ Hydroxyl acrylate monomer is the reaction product of acrylic acid with Cardura™. Cardura is the glycidyl ester of VERSATIC™ acid 10, a highly branched saturated carboxylic acid containing 10 |

| | |
|---|---|
| | carbon atoms |
| EA | Ethyl acrylate |
| MA | Methyl acrylate |
| MMA | Methyl methacrylate |
| Mam | Methacrylamide |
| n-BA | n-Butyl acrylate |
| 2-EHA | 2-Ethylhexyl acrylate |
| DEIA | Diethyl itaconate |
| TMCHMA | 3,3,5-Trimethylcyclohexyl methacrylate |
| DMAEMA | 2-(dimethylamino)ethyl methacrylate |
| DMAPMA | 3-(dimethylamino)propyl methacrylate |
| DMAIPMA | 1-(dimethylamino)propan-2-yl methacrylate |
| DMABMA | 4-(dimethylamino)butyl methacrylate |
| DMADMEMA | 2-(dimethylamino)-2-methylpropyl methacrylate |
| DMADMPMA | 2-(dimethylamino)-2,2-dimethylpropyl methacrylate |
| DMANPA | N,N-dimethylaminoneopentyl acrylate |
| CSEM25 | Ceteareth-25 methacrylate |
| HEMA | 2-Hydroxyethyl methacrylate |
| TEGDMA | Triethyleneglycol dimethacrylate |
| TMPDAE | Trimethylolpropane diallylether |
| TMPTA | Trimethylolpropane triacrylate |
| MBA | Methylenebisacrylamide |
| AS | Allyl ether of sucrose |
| APE | Allyl ether of pentaerythritol |
| VEOVA-10 | Vinyl neodecanoate |
| VP | N-Vinyl pyrrolidone |
| SMA | Stearyl methacrylate |
| $H_2O_2$ | Hydrogen peroxide |
| Polyglykol | A randomly ethoxylated/propoxylated allyl ether having the empirical formula $CH_2$=$CHCH_2O(C_3H_6O)_4(C_2H_4O)_6$-H; (Polyglykol A32/550, Clariant Corporation) |
| RAL307 | A randomly ethoxylated/propoxylated allyl ether having the empirical formula $CH_2$=$CHCH_2O(C_3H_6O)_4(C_2H_4O)_{30}$-H; (EMULSOGEN™ RAL307, Clariant Corporation) |
| E407 | Secondary $C_{11}$ ethoxylate having 40 ethylene oxide units per alcohol unit (EMULSOGEN™ EPN 407, Clariant Corp.) |
| IPA | Isopropyl alcohol |
| EtOH | Ethanol |
| Si Macromer | PEG/PPG-23/6 dimethicone citraconate macromonomer |

**Synthesis of DMAPMA or DMADMPMA - Via Transesterification:**

To a reaction flask equipped with a distillation column with a condenser, thermometer, and a nitrogen/air inlet is added 2.5 equivalents of methyl methacrylate (MMA), (1.0 equivalent) dimethylamino-propanol (DMAPOL) or 3-dimethylamino-2,2-dimethyl-1-propanol (DMADMPOL), a suitable amount of a polymerization inhibitor(s) such as phenothiazine or MEHQ, and catalytic amount of a suitable transesterification catalyst. Suitable catalysts include, but are not limited to, Sn salts, Ti salts, Zr salts, and Zn salts. In one embodiment, such catalysts include, but are not limited to, tin chloride, tin triflate, dimethyltin chloride, dibutyltin diacetate, dibutyltin dichloride, dibutyltin dilaurate, dibutyltin ditriflate, dibutyltin oxide, dibutyltin dimethoxide, dibutyltin diethoxide, dioctyltin diacetate, dioctyltin dichloride, dioctyltin dilaurate, dioctyltin ditriflate, dioctyltin oxide, dioctyltin dimethoxide, dioctyltin diethoxide; titanium chloride, titanium methoxide, titanium ethoxide, titanium isopropoxide, titanium butoxide etc. The mixture is heated to reflux with a very gentle flow of $N_2$/air. The temperature is maintained at 100°C to 120°C until the reaction is finished as indicated by GC analysis. Upon completion of the reaction, the desired monomer can be purified by vacuum distillation in good yield.

Regarding the dimethylamino-propanol (DMAPOL) or the 3-dimethylamino-2,2-dimethyl-1-propanol (DMADMPOL), these compounds can be synthesized in accordance with a number of techniques including, but not limited to, those techniques/methods disclosed in United States Provisional Patent Application No. 61/231,780, filed on August 6, 2009, which is incorporated herein in its entirety for all that it discloses.

Acid-Base Titration: A series of amine acrylate monomers are synthesized in accordance with the exemplary method disclosed above and tested for hydrolytic stability. The hydrolytic stability of these amine monomers is tested and compared with the stability of DMAEMA (see Figure 1). Higher acid number indicates a higher degree of amine

hydrolysis. In contrast, all other amines with increased chain length and hydrophobicity show a higher degree of hydrolytic stability as compared to the DMAEMA. Increasing hydrophobicity through carbon chain length between the nitrogen and the ester oxygen atom is found to be an unexpected factor in reducing hydrolysis. Branching effectively enhances the hydrolytic stability of the increased chain length when the chain length between nitrogen and ester oxygen is extended with at least 3 carbon atoms or 2 carbon atoms with alkyl substitution.

$^1H$ NMR Study: In order to confirm the performance of DMADMPMA, the hydrolysis process is monitored by $^1H$ NMR analysis (see Figure 2). Again, DMADMPMA shows excellent hydrolytic stability over DMAEMA.

Polymerization General Procedure: In a typical polymerization, a mixture of monomers is added to a solution of emulsifying surfactant that is undergoing mixing, such as a nonionic surfactant, such as a linear or branched alcohol ethoxylate, or mixtures of nonionic surfactants and anionic surfactants, such as fatty alcohol sulfates or alkyl sulfonates, methyl glucoside derivatives (Glucamate™ DOE 120, Glucamate™ SSE-20, and Glucamate™ LT) in a suitable amount of water that is contained in a 2 L reactor to prepare a monomer emulsion. The emulsion is deoxygenated by any suitable method, such as by sparging with nitrogen, and then a polymerization reaction is initiated by adding a polymerization catalyst (initiator) such as sodium persulfate or any other suitable addition polymerization catalyst, as is well known in the emulsion polymerization art. The reaction is agitated until the polymerization is complete, typically for a time in the range of about 4 hours to about 16 hours. The monomer emulsion can be heated to a temperature in the range of about 20°C to about 95°C prior to addition of the initiator, if desired. Unreacted monomer can be eliminated by addition of more catalyst, as is well known in the emulsion polymerization art. The resulting polymer emulsion product can then be discharged from the reactor and packaged for storage or use. Optionally, the pH or other physical and chemical characteristics of the emulsion can be adjusted prior to discharge from the reactor. Typically, the total polymer content of the product emulsion is in the range of about 15 weight percent to about 60 weight percent total polymer solids (TS), but generally not more than about 40 weight percent.

Control Polymers: Control 1 - This control polymer (Example 0) is made with an acrylate amine version, DMANPA. Control 2 - This control polymer (Example 18) is made with DMAEMA. Control 3 - DMAEMA based commercial polymer (Carbopol®Aqua CC with INCI: Polyacrylate-1 Crosspolymer).

Table 1 sets forth a list of new copolymers and their monomer components. These new polymers are synthesized using at least one amine monomer of the following: DMANPA, DMADMPMA, DMAPMA, DMABMA, and DMAEMA with other copolymerizable monomers. The multi-purpose polymer identified as Ex. No. 3 in Table 1 is prepared according to the general procedure described above, and as described in detail as follows. A monomer emulsion is prepared by adding with mixing agitation about 59.05 grams of ethyl acrylate, about 35 grams of DMADMPMA, about 1.8 grams of HEMA, about 4.0 grams of RAL307 and about 0.15 grams of TEGDMA into a reactor containing about 360 grams of water containing about 24.29 grams of Emulsogen E407 (70%) nonionic surfactant and about 2.0 grams of sodium lauryl sulfate (30%) anionic surfactant. The resulting mixture is agitated (about 200 rpm) at a temperature in the range of about 30 to about 40°C under a nitrogen atmosphere until an emulsion is obtained. A solution of an oxidizing agent of about 0.16 grams of sodium persulfate in about 5 grams of water and a solution of reducing agent of about 0.12 parts by weight of Bruggolite® FF6 in about 5 grams of water are then added to the monomer emulsion, with mixing agitation, to initiate the polymerization reaction. The temperature of the reaction mixture is maintained at a temperature in the range of about 60 to about 70° C. for about 2.5 hours after addition of the initiator. Additional quantities of initiators (about 0.08 grams of sodium persulfate in about 3.0 grams of water and about 0.06 grams of Bruggolite® FF6 in about 3.0 grams of water for each additional quantity of initiator added) are added at about 1.0 hour and about 2.0 hours after the reaction is initiated. The temperature of the reaction is maintained at about 60 to about 70° C for an additional two and half hours to complete the polymerization. The resulting polymer emulsion product is then cooled to room temperature, discharged from the reactor and recovered. The resulting polymer emulsion, Ex. No. 3, had a total polymer solids of about 21% by weight, a pH of about 7.79, and a viscosity of about 8.5 mPa.s. The remaining examples set forth in Table 1 are similarly prepared from the monomer components identified in the table.

Table 2 provides latex properties such as Percent TS, viscosity, pH, and an acid number for each polymer. The high acid number shown for a control polymer (Example 18) containing DMAEMA indicates a significant amount of monomer hydrolysis taking place during the polymerization. In contrast, the polymers of Examples 1, 12 and 13 show lower acid numbers, exhibiting improved hydrolytic stability for DMAPMA and DMABMA based polymers during polymerization. However, all other polymers give much lower acid numbers demonstrating better hydrolytic stability for the DMADMPMA based polymers and its blends (DMADMPMA/DMAPMA and DMADMPMA/DMAEMA) during polymerization.

Table 3 gives thickening properties for polymers listed in Table 1. All 2% TS gels (mucilage of 2 wt.% total polymer solids in deionized water) are made by neutralizing with 50% aqueous glycolic acid to pH 4.0 and then characterized for viscosity (20 RPM), YV, pH, and clarity (measured as turbidity in NTU).

These gels can be used as hair styling composition with and without other monomeric and polymeric quaternary ammonium salt(s) to achieve a hair style having long-lasting hold with high-setting properties.

The control polymer (Example 0) made with DMANPA shows inferior performance in thickening at 2% TS

(mucilage of 2 wt.% total polymer solids in deionized water) as compared to methacrylate versions (polymer of Examples 1, 2, 2a, 3, 12 and 13).

Table 1: Composition of Polymers

| Ex. No. | MA | EA | MMA | nBA | 2EHA | TEGDMA | TMPTA | RAL 307 | Polyglykol A 32/550 | CSEM | HEMA | M1 | M2 | M3 | M4 | M5 |
|---|---|---|---|---|---|---|---|---|---|---|---|---|---|---|---|---|
| 0* | | 55.9 | | | | 0.1 | | 4 | | 3 | 2.0 | | | | | 35 |
| 1 | | 56.15 | | | | 0.05 | | 4 | | 3 | 1.8 | | 35 | | | |
| 2 | | 54.10 | | | | 0.1 | | 4 | | 5 | 1.8 | | 17.5 | | 17.5 | |
| 2a | | 54.15 | | | | 0.05 | | 4 | | 5 | 1.8 | | 17.5 | 17.5 | | |
| 3 | | 59.05 | | | | 0.15 | | 4 | | | 1.8 | | | | 35 | |
| 4 | | 54.15 | | | | 0.05 | | 4 | | 5 | 1.8 | | | | 35 | |
| 5 | | 59.10 | | | | | 0.1 | 4 | | | 1.8 | | | | 35 | |
| 6 | | 54.20 | | | | | | 4 | | 5 | 1.8 | | | | 35 | |
| 7 | | 59.20 | | | | | | 4 | | | 1.8 | | | | 35 | |
| 8 | | 54.15 | | | | 0.05 | | 4 | | 5 | 1.8 | | | | 35 | |
| 9 | | 56.15 | | | | 0.05 | | 4 | | 3 | 1.8 | | | | 35 | |
| 10 | | 59.00 | | | | | 0.2 | 4 | | | 1.8 | | | | 35 | |
| 11 | | 58.90 | | | | | 0.3 | 4 | | | 1.8 | | | | 35 | |
| 12 | | 56.15 | | | | 0.05 | | 4 | | 3 | 1.8 | 35 | | | | |
| 13 | | 56.15 | | | | 0.05 | | 4 | | 3 | 1.8 | | 35 | | | |
| 14 | | 54.15 | | | | 0.05 | | 4 | | 5 | 1.8 | | | 8.75 | 26.25 | |
| 15 | | 54.15 | | | | 0.05 | | 4 | | 5 | 1.8 | | | 17.5 | 17.5 | |
| 16 | | 63.20 | | | | | | | | | 1.8 | | | | 35 | |
| 17 | | 61.00 | | | | | | 4 | | | | | | | 35 | |
| 18 * | | 61.00 | | | | | | 4 | | | | | | 35 | | |
| 19 | | 71.00 | | | | | | 4 | | | | | | | 25 | |
| 20 | | 61.00 | | | | | | | 4 | | | | | | 35 | |
| 21 | | 56.00 | | | | | | 4 | | | | | | | 40 | |
| 22 | | 21.8 | 39.2 | | | | | 4 | | | | | | | 35 | |
| 23 | | 66.00 | | | | | | 4 | | | | | | | 30 | |

(continued)

| Ex. No. | MA | EA | MMA | nBA | 2EHA | TEGDMA | TMPTA | RAL 307 | Polyglykol A 32/550 | CSEM | HEMA | M1 | M2 | M3 | M4 | M5 |
|---|---|---|---|---|---|---|---|---|---|---|---|---|---|---|---|---|
| 24 | 61 | | | | | | | 4 | | | | | | | 35 | |
| 25 | | | | 61 | | | | 4 | | | | | | | 35 | |
| 26 | | | 61 | | | | | 4 | | | | | | | 35 | |
| 27 | | | | | 61 | | | 4 | | | | | | | 35 | |
| 28 | 15.2 | 45.8 | | | | | | 4 | | | | | | | 35 | |
| 29 | | 45.8 | 15.2 | | | | | 4 | | | | | | | 35 | |

*Control Examples, see above; M1 is DMABMA; M2 is DMAPMA; M3 is DMAEMA; M4 is DMADMPMA; and M5 is DMANPA. All amounts are in weight percent of the total amount of the reaction charge.

Table 1 - Continued: Composition of Polymers

| Ex. No. | MA | EA | MMA | VEOVA-10 | DEIA | TEGDMA | RAL 307 | Polyglykol A 32/550 | CSEM | APE | Allyl Sucrose | MBA | TMPDAE | Si Macromer | ACE | M4 |
|---|---|---|---|---|---|---|---|---|---|---|---|---|---|---|---|---|
| 30 | | 45.8 | | 15.2 | | | 4 | | | | | | | | | 35 |
| 31 | | 45.8 | | | 15.2 | | 4 | | | | | | | | | 35 |
| 32 | 15.2 | 45.7 | | | | 0.1 | 4 | | | | | | | | | 35 |
| 33 | | 45.7 | 15.2 | | | 0.1 | 4 | | | | | | | | | 35 |
| 34 | | 45.7 | 15.2 | | | | 4 | | | 0.1 | | | | | | 35 |
| 35 | | 45.7 | 15.2 | | | | | | | | 0.1 | | | | | 35 |
| 36 | | 45.7 | 15.2 | | | | 4 | | | | | 0.1 | | | | 35 |
| 37 | | 45.7 | 15.2 | | | | 4 | | | | | | 0.1 | | | 35 |
| 38 | | 42.7 | 15.2 | | | | 4 | | 3 | | | | 0.1 | | | 35 |
| 39 | | 60.9 | | | | | | 4 | | | | | 0.1 | | | 35 |
| 40 | | 59.9 | | | | | | 4 | | | | | 0.1 | 1 | | 35 |
| 41 | | 45.7 | 15.2 | | | | 2 | 2 | | | | | 0.1 | | | 35 |
| 42 | 15.2 | 45.7 | | | | | 2 | 2 | | | | | 0.1 | | | 35 |
| 43 | 15.2 | 45.7 | | | | | | 4 | | | | | 0.1 | | | 35 |
| 44 | 15.2 | 45.8 | | | | | | 4 | | | | | | | | 35 |
| 44a | 15.2 | 47.8 | | | | | | | | | | | | | 2 | 35 |
| 44b | 15.2 | 44.8 | | | | | | | 3 | | | | | | 2 | 35 |

M4 is DMADMPMA. All amounts are given in weight percent of the total amount of the reaction charge.

Table 2: Latex Properties

| Example Number | TS (wt.%) | Viscosity (mPa·s) | pH | Acid No. (mg KOH/g) |
|---|---|---|---|---|
| 0* | 20.07 | 8.3 | 9.0 | 12.74 |
| 1 | 20.97 | 20.0 | 8.80 | 14.38 |
| 2 | 20.88 | 10.5 | 8.44 | 10.87 |
| 2a | 21.36 | 9.5 | 8.17 | 10.82 |
| 3 | 21.31 | 8.5 | 7.79 | 7.24 |
| 4 | 21.01 | 8.0 | 7.65 | 9.01 |
| 5 | 21.23 | 8.5 | 8.42 | 7.24 |
| 6 | 21.09 | 9.0 | 7.43 | 9.07 |
| 7 | 21.03 | 9.0 | 8.12 | 7.11 |
| 8 | 21.30 | 9.5 | 6.89 | 8.27 |
| 9 | 21.34 | 9.5 | 7.06 | 8.22 |
| 10 | 21.46 | 9.5 | 9.21 | 10.78 |
| 11 | 21.98 | 9.5 | 7.33 | 7.51 |
| 12 | 21.25 | 40.0 | 10.21 | 16.08 |
| 13 | 21.69 | 29.5 | 9.22 | 13.39 |
| 14 | 20.98 | 9.5 | 8.96 | 15.10 |
| 15 | 21.14 | 10.0 | 8.41 | 14.25 |
| 16 | 21.19 | 9.5 | 8.61 | 10.16 |
| 17 | 22.21 | 10.0 | 8.75 | 7.79 |
| 18* | 20.64 | 12.50 | 8.54 | 20.45 |
| 19 | 22.44 | 9.50 | 8.87 | 6.58 |
| 20 | 21.76 | 9.50 | 8.66 | 9.83 |
| 21 | 21.92 | 10.00 | 8.92 | 8.77 |
| 22 | 22.33 | 10.0 | 8.02 | 7.21 |
| 23 | 22.65 | 10.0 | 7.67 | 6.85 |
| 24 | 21.74 | 9.5 | 8.08 | 10.20 |
| 25 | 20.38 | 9.5 | 9.52 | 7.97 |
| 26 | 21.48 | 10.0 | 8.44 | 7.31 |
| 27 | 19.4 | 9.5 | 8.84 | 0.00 |
| 28 | 21.86 | 9.50 | 7.83 | 9.26 |
| 29 | 21.86 | 9.50 | 8.14 | 7.02 |
| 30 | 19.08 | 9.0 | 8.59 | 9.76 |
| 31 | 21.29 | 9.5 | 7.98 | 8.10 |
| 32 | 21.45 | 9.5 | 7.67 | 8.19 |
| 33 | 21.51 | 9.0 | 8.38 | 6.84 |
| 34 | 21.53 | 9.5 | 8.35 | 8.18 |
| 35 | 21.57 | 9.5 | 8.34 | 6.95 |

(continued)

| Example Number | TS (wt.%) | Viscosity (mPa·s) | pH | Acid No. (mg KOH/g) |
|---|---|---|---|---|
| 36 | 21.46 | 9.5 | 8.2 | 8.26 |
| 37 | 21.46 | 9.5 | 8.44 | 6.66 |
| 38 | 25.59 | 12.5 | 7.68 | 8.04 |
| 39 | 28.19 | 13.5 | 8.26 | 7.14 |
| 40 | 26.51 | 12.0 | 8.44 | 6.31 |
| 41 | 28.04 | 13.0 | 8.05 | 7.50 |
| 42 | 27.86 | 14.0 | 8.09 | 6.94 |
| 43 | 28.04 | 14.0 | 7.98 | 7.37 |
| 44 | 28.05 | 13.0 | 8.18 | 6.57 |
| 44a | 27.69 | 12.5 | 8.17 | 8.43 |
| 44b | 26.2 | 13 | 7.94 | 9.77 |
| *Control Example<br>TS = total polymer solids | | | | |

Table 3: Thickening Properties

| Example Number | pH | 2% Viscosity* (mPa·s) | Yield value (dyn/cm$^2$) | Turbidity (NTU) |
|---|---|---|---|---|
| 0* | 3.94 | 430 | 55 | NA |
| 1 | 3.97 | 16,400 | 2,246 | 15.0 |
| 2 | 4.16 | 14,000 | 1,900 | 8.35 |
| 2a | 4.07 | 23,000 | 3,270 | 9.53 |
| 3 | 4.04 | 14,500 | 1,700 | 29.3 |
| 4 | 4.02 | 15,400 | 1,970 | 12.3 |
| 5 | 3.99 | 9,600 | 1,120 | 5.71 |
| 6 | 3.94 | 16,500 | 2,020 | 19.5 |
| 7 | 4.01 | 5,800 | 508 | 4.72 |
| 8 | 4.10 | 14,700 | 1,760 | 10.1 |
| 9 | 4.03 | 14,700 | 1,810 | 18.3 |
| 10 | 3.88 | 4,300 | 490 | 45.7 |
| 11 | 4.08 | 8,000 | 990 | 11.7 |
| 12 | 3.97 | 3,960 | 352 | 26.5 |
| 13 | 4.03 | 20,300 | 2,740 | 12.0 |
| 14 | 4.07 | 15,300 | 1,960 | 8.64 |
| 15 | 3.95 | 22,000 | 3,110 | 4.03 |
| 16 | 4.09 | 4,920 | 424 | 3.60 |
| 17 | 4.08 | 8,600 | 860 | 8.59 |
| 18* | 3.97 | 7,400 | 910 | 5.37 |
| 19 | 3.94 | 1,590 | 106 | 16.30 |
| 20 | 4.01 | 6,400 | 638 | 4.34 |

(continued)

| Example Number | pH | 2% Viscosity* (mPa·s) | Yield value (dyn/cm$^2$) | Turbidity (NTU) |
|---|---|---|---|---|
| 21 | 3.96 | 8,100 | 1,090 | 3.31 |
| 22 | 3.87 | 2,700 | 245 | 14.7 |
| 23 | 3.87 | 7,350 | 776 | 28.7 |
| 24 | 3.89 | 8,800 | | 44.30 |
| 25 | 3.93 | 175 | 2 | 27.40 |
| 26 | 3.60 | 20 | 0 | 160 |
| 27 | 3.68 | 15 | 0 | 179 |
| 28 | 4.06 | 12,200 | 1,330 | 15.4 |
| 29 | 4.04 | 7,150 | 592 | 5.4 |
| 30 | 3.78 | 2,330 | 234 | 24.3 |
| 31 | 4.06 | 5,100 | 366 | 5.94 |
| 32 | 3.89 | 12,000 | 1,470 | 16.5 |
| 33 | 3.77 | 8,700 | 920 | 8.06 |
| 34 | 4.06 | 8,800 | 900 | 8.22 |
| 35 | 4.01 | 4,940 | 440 | 4.0 |
| 36 | 4.0 | 10,100 | 1,160 | 9.8 |
| 37 | 3.98 | 7,100 | 658 | 5.1 |
| 38 | 4.07 | 15,000 | 1,610 | 11.2 |
| 39 | 4.03 | 7,600 | 718 | 4.66 |
| 40 | 4.09 | 10,100 | 1,060 | 10.3 |
| 41 | 3.87 | 6,750 | 628 | 5.20 |
| 42 | 4.04 | 11,900 | 1,210 | 8.97 |
| 43 | 3.96 | 8,850 | 1,040 | 5.15 |
| 44 | 3.97 | 8,000 | 920 | 3.94 |
| 44a | 3.76 | 6,000 | 578 | 4.08 |
| 44b | 4.01 | 10,100 | 1.130 | 9.10 |
| *2 wt. % polymer solids mucilage neutralized to pH 4 with a 50:50 (wt./wt.) $H_2O$/glycolic acid solution. | | | | |

**Surfactant Formulation:**

Several polymers (Table 1) are screened in Clear Bath Gel (Table 4) formulation containing anionic and amphoteric surfactant package at 2-3 different pH levels (pH = 6; pH 4; and pH 6) using the "back-alkaline" technique. The "back-alkaline" techniques/methods disclosed in United States Provisional Patent Application No. 20040241130, which is incorporated herein in its entirety for all that it discloses.

pH 6: Samples at pH 6 are prepared as follows: Ingredient numbers 1 and 2 are premixed, ingredient numbers 3 to 6 are added to the premix with gentle mixing and then the mixture is neutralized to about pH 6 with ingredient number 7. A sample is removed and characterized for pH, viscosity, YV, and turbidity.

pH 4: Sample is further lowered to pH 4 with ingredient number 7 before taking out a sample for characterization (pH, viscosity, YV, and turbidity).

pH 6 (Back-Alkaline): The remaining sample is further adjusted to pH 6 with ingredient number 8 for the final evaluation of properties such as pH, viscosity, YV, and turbidity.

Table 4: Clear Bath Gel Formulation

| Ingredient (INCI - Trade Name) | Active (wt.% TS) | Weight % |
|---|---|---|
| DI Water | q.s | q.s. |
| Polymers from Table 1 (active %) | 1.5 | 1.5 |
| Sodium Laureth Sulfate (3 mole, 28%), Sulfochem™ ES-3 Surfactant | 12 | 40 |
| Cocamidopropyl Betaine (35%), Chembetaine™ CAD Surfactant | 5.83 | 16.67 |
| Preservative (Phenonip) | 0.5 | q.s. |
| Tetra sodium EDTA | 0.05 | 0.2 |
| Glycolic Acid (50%) to about pH 6 and then 4 | q.s | q.s |
| Sodium Hydroxide to about pH 6.0 | q.s | q.s. |

Table 5 provides Clear Bath Gel properties at three different pH levels. Viscosity is slightly increased from pH 6 to 4 with aqueous glycolic acid solution (50:50 (wt./wt.) neutralization. Most of the polymers maintained viscosity values without any significant loss after the "back-alkaline" technique.

Table 5: Clear Bath Gel Properties

| Ex. No. | pH | Viscosity (mPa·s) | Yield Value (dyn/cm$^2$) | Turbidity (NTU) | pH | Viscosity (mPa·s) | Yield Value | Turbidity (NTU) | pH | Viscosity (mPa·s) | Yield Value (dyn/cm$^2$) | Turbidity (NTU) | pH 6 Suspension (visual rating) |
|---|---|---|---|---|---|---|---|---|---|---|---|---|---|
| | pH 6 | | | | pH 4 | | | | pH 6 | | | | |
| 1 | 6.16 | 8,100 | 116 | 143 | 4.19 | 9,800 | 112 | 146 | 6.70 | 10,100 | 100 | 163 | 0 |
| 2 | 6.06 | 10,800 | 140 | 66.1 | 4.19 | 13,200 | 148 | 66.3 | 6.73 | 12,900 | 128 | 77.4 | 0 |
| 2a | 6.25 | 6,750 | 88 | 156 | 4.19 | 9,200 | 100 | 151 | 6.23 | 8,700 | 76 | 171 | 1 |
| 3 | 6.14 | 12,200 | 212 | 77.9 | 4.17 | 15,600 | 204 | 75.3 | 6.42 | 14,600 | 276 | 92.1 | 0 |
| 4 | 6.01 | 10,000 | 100 | 51.6 | 4.11 | 12,600 | 104 | 50.7 | 6.01 | 12,600 | 100 | 61.7 | 2 |
| 5 | 5.98 | 11,000 | 236 | 56.4 | 4.15 | 14,600 | 300 | 54.3 | 6.05 | 14,300 | 256 | 65.7 | 0 |
| 6 | 6.01 | 17,700 | 208 | 9.07 | 4.13 | 21,800 | 220 | 9.43 | 6.15 | 23,200 | 270 | 12.1 | 4 |
| 7 | 6.08 | 14,300 | 296 | 51.8 | 4.11 | 19,200 | 450 | 50.5 | 5.93 | 19,400 | 360 | 57.4 | 0 |
| 8 | 6.06 | 10,300 | 236 | 67.9 | 4.09 | 14,500 | 310 | 65.7 | 6.58 | 12,200 | 168 | 90.0 | 4 |
| 9 | 6.14 | 3,120 | 4 | 363 | 4.10 | 4,740 | 20 | 362 | 7.39 | 2,740 | 12 | 537 | 3 |
| 10 | 6.11 | 4,000 | 16 | 151 | 4.13 | 5,650 | 20 | 157 | 6.14 | 5,500 | 16 | 178 | 0 |
| 11 | 5.96 | 10,500 | 208 | 31.9 | 4.00 | 13,600 | 208 | 32.1 | 5.94 | 14,100 | 204 | 37.3 | 0 |
| 12 | 6.00 | 12,900 | 286 | 15.8 | 3.94 | 16,600 | 286 | 15.8 | 5.97 | 16,400 | 282 | 18.4 | 0 |
| 13 | 6.18 | 7,800 | 128 | 112 | 4.14 | 10,500 | 136 | 117 | 6.08 | 10,600 | 116 | 129 | 0 |
| 14 | 5.96 | 10,500 | 208 | 31.9 | 4.00 | 13,600 | 208 | 32.1 | 5.94 | 14,100 | 204 | 37.3 | 2 |
| 15 | 6.00 | 12,900 | 286 | 15.8 | 3.94 | 16,600 | 286 | 15.8 | 5.97 | 16,400 | 282 | 18.4 | 1 |

Table 6 offers Clear Bath Gel properties only at two different pH levels. Like the polymers from Table 5, most of the polymers in Table 6 also maintained viscosity values without any significant loss after "back-alkaline" technique.

Table 6: Clear Bath Gel Properties

| Example Number | pH | Viscosity (mPa·s) | Yield Value (dyn/cm$^2$) | Turbidity (NTU) | Suspension (visual rating) | pH | Viscosity (mPa·s) | Yield Value (dyn/cm$^2$) | Turbidity (NTU) | Suspension (visual rating) |
|---|---|---|---|---|---|---|---|---|---|---|
| | pH 4 | | | | | pH 6 | | | | |
| 17 | 4.01 | 37,300 | 1150 | 11.4 | 1 | 6.03 | 35,600 | 930 | 12.8 | 1 |
| 18* | 4.00 | 23,600 | 260 | 21.4 | 0 | 5.96 | 24,700 | 270 | 21.7 | 0 |
| 19 | 3.95 | 16,600 | 340 | 171 | 2 | 6.09 | 14,100 | 210 | 212 | 2 |
| 20 | 4.05 | 31,000 | 620 | 8.75 | 0 | 6.95 | 23,500 | 460 | 16.0 | 2 |
| 21 | 4.01 | 32,000 | 530 | 7.89 | 0 | 6.86 | 28,400 | 520 | 12.0 | 2 |
| 22 | 3.97 | 17,900 | 130 | 41.9 | 0 | 6.16 | 19,000 | 130 | 47.1 | 0 |
| 23 | 4.06 | 15,700 | 210 | 87.7 | 4 | 6.19 | 13,700 | 120 | 115.0 | 0 |
| 24 | 4.13 | 6,600 | 54 | 83.2 | 1 | 6.13 | 6,950 | 48 | 90.7 | 0 |
| 25 | 4.09 | 2,590 | 6 | opaque | 0 | 6.21 | 2,040 | 8 | opaque | 0 |
| 26 | 4.02 | 3,950 | 3 | 473 | 0 | 6.12 | 4,490 | 2 | 488 | 0 |
| 27 | 3.97 | 2,910 | 3 | 223 | 0 | 6.03 | 2,540 | 2 | 260 | 0 |
| 28 | 4.04 | 19,400 | 410 | 38.6 | 4 | 6.06 | 18,500 | 310 | 48.6 | 4 |
| 29 | 4.01 | 36,200 | 830 | 11.1 | 0 | 6.07 | 33,800 | 790 | 14.1 | 1 |
| 30 | 3.94 | 12,800 | 80 | 10.3 | 0 | 6.07 | 12,600 | 80 | 11.8 | 0 |
| 31 | 3.86 | 25,900 | 290 | 14.3 | 0 | 6.23 | 22,400 | 250 | 17.1 | 0 |
| 32 | 4.01 | 10,200 | 88 | 78.7 | 4 | 6.24 | 9,500 | 60 | 96.6 | 0 |
| 33 | 4.05 | 22,300 | 250 | 95.4 | 0 | 6.33 | 21,100 | 190 | 109 | 0 |
| 34 | 4.01 | 25,500 | 440 | 24.9 | 1 | 6.13 | 24,200 | 380 | 30.5 | 3 |
| 35 | 4.0 | 25,800 | 260 | 16.3 | 0 | 6.43 | 25,500 | 260 | 19.2 | 0 |
| 36 | 3.99 | 23,100 | 330 | 41.6 | 1 | 6.10 | 21,900 | 270 | 51.9 | 3 |
| 37 | 3.97 | 28,700 | 510 | 15.8 | 0 | 6.17 | 28,200 | 510 | 19.8 | 2 |
| 38 | 4.12 | 17,000 | 310 | 29.3 | 4 | 6.41 | 15,700 | 200 | 37.0 | 3 |
| 39 | 3.98 | 24,100 | 490 | 11.5 | 1 | 6.19 | 21,400 | 360 | 15.6 | 1 |

(continued)

| Example Number | pH | Viscosity (mPa·s) | Yield Value (dyn/cm$^2$) | Turbidity (NTU) | Suspension (visual rating) | pH | Viscosity (mPa·s) | Yield Value (dyn/cm$^2$) | Turbidity (NTU) | Suspension (visual rating) |
|---|---|---|---|---|---|---|---|---|---|---|
| | pH 4 | | | | | pH 6 | | | | |
| 40 | 4.01 | 19,800 | 430 | 21.1 | 3 | 5.95 | 18,700 | 330 | 27.0 | 2 |
| 41 | 3.98 | 24,000 | 410 | 15.4 | 0 | 6.04 | 23,400 | 380 | 18.0 | 1 |
| 42 | 4.10 | 15,400 | 268 | 24.6 | 4 | 6.36 | 13,800 | 172 | 32.5 | 4 |
| 43 | 3.95 | 23,800 | 600 | 11.2 | 4 | 6.43 | 23,100 | 440 | 16.3 | 4 |
| 44 | | | | | | 6.00 | 28,300 | | 11.0 | |
| 44a | | | | | | 6.00 | 23,500 | | 17.3 | |
| 44b | | | | | | 6.00 | 21,850 | | 40.7 | |

**Hydrolytic Stability:**

Improved shelf life performance of multi-purpose copolymers made with hydrolytically stable amine monomers (DMAPMA, and DMADMPMA) is demonstrated in the aging study as described below.

This study is performed at three different conditions such as: (1) latex stored at 45°C for 3 months; (2) latex stored at 50°C for 5 weeks; and (3) latex stored at room temperature for one to about two years.

**Aging Study at Elevated Temperature (Latex stored at 45°C for 3 Months):**

Two polymers (Examples 1 and 2) are stored in a 45°C oven for 3 months. Samples are removed periodically and tested for latex properties as well as their thickening properties (see Table 7). Figure 3 provides the acid number in mg KOH/g (acid number) versus the number of weeks the latex is stored at 45°C. Both polymers exhibit a moderate (DMAPMA monomer) to excellent (using DMADMPMA monomer) shelf life performance at 45°C temperature storage conditions.

Table 7: Thickening Performance of Aged Samples (3 Months at 45°C)

| Ex. No. | Weeks at 45°C | TS (wt. %) | pH | Viscosity (mPa·s) | Acid No. (mg KOH/g) | pH | Viscosity (mPa·s) | Yield Value (dyn /cm$^2$) | Turbidit y (NTU) |
|---|---|---|---|---|---|---|---|---|---|
| | | Latex properties | | | | Thickening Properties | | | |
| 1 | 0 | 20.97 | 8.80 | 20.0 | 14.38 | 3.97 | 16,400 | 2,240 | 15.0 |
| | 1 | 20.97 | 8.76 | 27.5 | 17.18 | 4.05 | 15,200 | 2,070 | 15.1 |
| | 3 | 20.93 | 8.62 | 32.5 | 17.34 | 4.03 | 16,800 | 2,180 | 14.9 |
| | 4 | 21.00 | 8.71 | 50.0 | 18.24 | 3.75 | 11,400 | 1,540 | 16.6 |
| | 6 | 21.08 | 8.60 | 35.0 | 12.22 | 4.26 | 12,900 | 1,710 | 18.2 |
| | 9 | 21.08 | ND | 30.0 | 21.16 | 4.02 | 12,200 | 1,610 | 17.1 |
| | 10 | 21.11 | 8.73 | 31.5 | 23.09 | 4.13 | 11,000 | 1,440 | 17.9 |
| | 12 | 21.13 | 8.78 | 36.5 | 22.89 | 3.96 | 10,500 | 1, 380 | 19.2 |
| 2 | 0 | 20.88 | 8.44 | 10.5 | 10.87 | 4.16 | 14,000 | 1,900 | 8.35 |
| | 1 | 20.88 | 8.31 | 10.5 | 14.20 | 4.16 | 14,300 | 1,960 | 9.13 |
| | 3 | 20.79 | 8.08 | 12.0 | 13.34 | 4.01 | 17,600 | 2,250 | 9.11 |
| | 4 | 20.75 | 8.10 | 30.0 | 12.98 | 3.68 | 11,500 | 1,600 | 8.83 |
| | 6 | 20.76 | 8.05 | 12.0 | 14.34 | 4.18 | 14,700 | 2,100 | 9.54 |
| | 9 | 20.76 | ND | 12.0 | 14.45 | 4.11 | 15, 000 | 2,030 | 10.00 |
| | 10 | 20.85 | 8.02 | 11.0 | 14.40 | 3.91 | 13,100 | 1,800 | 10.10 |
| | 12 | 20.81 | 8.12 | 11.5 | 15.69 | 3.93 | 12,700 | 1,730 | 12.10 |

From aged polymers, two percent gels are prepared by neutralizing with 50% glycolic acid to pH 4. Figure 4 below provides 2% gel viscosity (mPa·s) versus time (number of weeks latex stored at 45°C).

**Aging Study at Elevated Temperature (5 Weeks at 50°C):**

Two polymers (Examples 4 and 7) are stored in a 50°C oven for 5 weeks. Samples are removed weekly and tested for latex properties and their thickening properties (Table 8). Figure 5 provides the acid number in mg KOH/g versus time, (number of weeks latex stored at 50°C) for both polymers which are synthesized using DMADMPMA. Similarly, Figure 6 shows 2% wt.% TS gel viscosity versus time for both Examples 4 and 7 polymers against Control 3. Both Figures demonstrate a steady acid number and viscosity profile over time at 50°C, confirming a better hydrolytic stability for polymers made with the DMADMPMA monomer over Control 3.

Table 8 shows good stability for multi-purpose polymers at 50°C and noticeably no drop in thickening efficiency in contrast to Control 3 which shows a steady degradation in thickening efficiency under the same condition.

Table 8: Thickening Performance for Aged Samples (5 Weeks at 50°C)

| Ex. No. | Weeks at 50°C | TS (wt.%) | pH | Viscosity (mPa·s) | Acid No. (mg KOH/g) | pH | Viscosity (mPa·s) | Yield Va-lue (dyn / cm$^2$) | Turb-idity (NTU) |
|---|---|---|---|---|---|---|---|---|---|
| | | Latex Properties | | | | Gel properties | | | |
| Control 3 | 0 | 20.27 | 8.33 | 20.0 | 29.50 | 3.87 | 15,600 | 1,400 | 3.49 |
| | 1 | 20.27 | 8.43 | 25.0 | 31.01 | 4.10 | 13,300 | 1,300 | 6.06 |
| | 2 | 20.14 | 8.27 | 20.0 | 32.31 | 3.75 | 12,700 | 1,230 | 6.29 |
| | 3 | 20.08 | 8.30 | 20.0 | 35.81 | 4.06 | 11,300 | 1,150 | 7.68 |
| | 4 | 20.08 | 8.18 | 10.0 | 42.53 | 4.14 | 10,500 | 1,150 | 8.46 |
| | 5 | 20.21 | 8.44 | 55.0 | 41.59 | 4.09 | 9,850 | 1,010 | 7,68 |
| 4 | 0 | 21.01 | 7.36 | 0.0 | 9.40 | 4.16 | 14,200 | 1,810 | 11.9 |
| | 1 | 21.01 | 7.28 | 0.0 | 8.65 | 4.06 | 14,400 | 1,850 | 12.3 |
| | 2 | 21.18 | 7.13 | 0.0 | 9.43 | 4.00 | 14,800 | 1,940 | 12.6 |
| | 3 | 21.01 | 7.31 | 0.0 | 8.66 | 4.04 | 12,600 | 1,690 | 14.4 |

| Ex. No. | Weeks at 50°C | TS (wt.%) | pH | Viscosity (mPa·s) | Acid No. (mg KOH/g) | pH | Viscosity (mPa·s) | Yield Va-lue (dyn / cm$^2$) | Turb-idity (NTU) |
|---|---|---|---|---|---|---|---|---|---|
| | | Latex Properties | | | | Gel properties | | | |
| | 4 | 21.01 | 7.08 | 0.0 | 8.79 | 4.17 | 13,000 | 1,650 | 16.1 |
| | 5 | 21.12 | 7.17 | 15.0 | 9.02 | 4.03 | 13,700 | 1,750 | 12.8 |
| 7 | 0 | 21.03 | 7.91 | 0.0 | 7.71 | 4.17 | 5,600 | 496 | 4.86 |
| | 1 | 21.03 | 7.82 | 0.0 | 8.29 | 4.12 | 6,000 | 504 | 5.15 |
| | 2 | 21.17 | 7.68 | 0.0 | 8.13 | 3.95 | 5,700 | 448 | 5.26 |
| | 3 | 21.03 | 7.63 | 0.0 | 7.32 | 4.17 | 5,700 | 502 | 6.80 |
| | 4 | 21.03 | 7.26 | 0.0 | 7.83 | 4.12 | 5,350 | 476 | 6.54 |
| | 5 | 21.26 | 7.65 | 15.0 | 8.58 | 3.95 | 5,750 | 490 | 5.31 |

**Product Color:**

Typically, Control 3 stored at room temperature (RT) or 45°C turns a brown color from milky white. However multi-purpose polymers (Examples 4 and 7), made from DMADMPMA monomer have better color stability before and after storage at an elevated temperature (e.g., 50°C).

**One to Two Year Aging Study at Room Temperature:**

Eight polymers (Examples 1, 2, 4, 6, 7 8, 13, and 14) along with the Control 3 are monitored for their stability and are tested for their performance for about 9 months to approximately one year. Samples are removed periodically and are tested for latex properties and thickening properties. Figure 7 exhibits acid number versus time (number of months latex stored at room temperature). All these polymers exhibit a moderate (Example 1, 2, 13, and 14) to excellent (Example 4, 6, 7, and 8) shelf life performance at ambient room temperature storage conditions (20° to 25° C). In contrast, Control 3 undergoes a steady increase in the acid number during room temperature storage condition, indicating a gradual degradation of DMAEMA backbone.

Figure 8 demonstrates thickening (2% gel) properties of DMADMPMA based polymers that are monitored periodically for one to about two years. All 4 polymers (Examples 4, 6, 7, and 9) showed a steady performance, indicating a good shelf life for these polymers, while Control 3 performance degrades gradually during this period because of its DMAEMA backbone.

Table 9 provides latex (wt.% TS, pH, viscosity, and acid number) and 2% Gel properties (viscosity, YV, and

turbidity) for several multi-purpose polymers. These properties are tested periodically for one to about two years. In general, the new polymers based on the DMADMPMA monomer pass the 1 year shelf life study within experimental error. Table 9 also offers thickening properties of amine blends (DMADMPMA/DMAEMA) based polymers (Examples 14 and 15) that are monitored periodically for 1 year. Polymer Examples 14 and 15 contain approximately 25% and 50% DMAEMA, respectively. Both polymers show a slight drop in their thickening performance but are still better than Control 3 which degrades gradually. Thickening properties of DMAPMA based polymers (Examples 1 and 2) show a steady performance up to six months and then a slight drop in viscosity at the tenth month but still better than Control 3.

Typically, Control 3 product stored at room temperature slowly changes from a milky white to a brown color. In contrast, the polymers that are made from DMAPMA or DMADMPMA monomers have better color stability after one year at room temperature storage conditions.

Table 9: One year Shelf Life Study: Thickening Properties

| Ex. No. | Months at RT* | TS (wt.%) | pH | Viscosity (mPa·s) | Acid No. | pH | 2% Viscosity** (mPa·s) | Yield Value (dyn / $cm^2$) | Turbidity (NTU) |
|---|---|---|---|---|---|---|---|---|---|
| | | Latex properties | | | | Gel Properties | | | |
| Control 3 | 0 | 20.49 | 8.10 | 24.0 | ND | 4.00 | 12,600 | 1,170 | 4.50 |
| | 0.5 | 20.49 | 8.27 | 20.5 | 30.10 | 3.99 | 12,200 | 1,240 | 6.46 |
| | 6.5 | 20.20 | 8.52 | 37.0 | 41.04 | 3.80 | 9,300 | 910 | 4.75 |
| | 10.25 | 20.04 | 8.76 | 4,300 | 48.01 | 4.18 | 7,700 | 794 | 6.56 |
| 1 | 0 | 20.97 | 8.80 | 20.0 | 14.38 | 3.97 | 16,400 | 2,240 | 15.0 |
| | 1 | 21.06 | 8.69 | 28.5 | 14.81 | 3.94 | 13,700 | 1,890 | 16.6 |
| | 2 | 20.97 | 8.69 | 34.5 | 17.01 | 4.15 | 15,900 | 2,140 | 16.3 |
| | 6 | 21.16 | 8.71 | ND | 19.32 | 3.93 | 15,800 | 1,980 | 15.8 |
| | 10.5 | 20.96 | 8.88 | some inc | 22.14 | 3.89 | 12,900 | 1,730 | 16.9 |
| | 13.5 | 21.43 | 8.93 | 120 (#3) | 23.80 | 4.04 | 10,700 | 1,380 | 19.8 |
| 2 | 0 | 20.88 | 8.44 | 10.5 | 10.87 | 4.16 | 14,000 | 1,900 | 8.35 |
| | 1 | 20.91 | 8.21 | 10.5 | 11.84 | 3.78 | 14,000 | 1,910 | 7.96 |
| | 2 | 20.84 | 8.17 | 10.0 | 12.37 | 3.96 | 14,500 | 1,950 | 7.45 |
| | 6 | 20.80 | 8.19 | 10.0 | 15.34 | 3.84 | 16,400 | 2,080 | 7.64 |
| | 10.5 | 20.77 | 8.36 | 10.0 | 14.91 | 4.01 | 13,500 | 1,840 | 7.73 |
| | 13.5 | 20.95 | 8.28 | 11.5 | 14.24 | 4.03 | 12,800 | 1,730 | 8.99 |
| 4 | 0 | 21.01 | 7.65 | 8.0 | 9.01 | 4.02 | 15,400 | 1, 970 | 12.3 |
| | 1 | 21.01 | 7.36 | 9.0 | 9.40 | 4.16 | 14,200 | 1,810 | 11.9 |
| | 5.5 | 20.99 | 7.19 | 8.5 | 9.70 | 4.05 | 14,700 | 1,850 | 12.1 |
| | 9.5 | 20.96 | 7.35 | 8.5 | 10.00 | 4.09 | 13,700 | 1,840 | 11.5 |
| | 12 | 21.03 | 7.23 | 9.0 | 8.99 | 3.81 | 16,100 | 1, 870 | 11.7 |
| 6 | 0 | 21.09 | 7.43 | 9.0 | 9.07 | 3.94 | 16,500 | 2,020 | 19.50 |
| | 5 | 21.22 | 7.26 | 8.0 | 9.63 | 4.04 | 18,100 | 2,020 | 18.20 |
| | 9.5 | 21.31 | 7.29 | 9.0 | 9.64 | 3.99 | 14,100 | 1,850 | 19.20 |
| | 12 | 21.36 | 7.26 | 10.0 | 9.07 | 4.01 | 17,300 | 1,880 | 19.20 |
| | 20 | 21.55 | 7.21 | 9.5 | 11.16 | 4.02 | 15,300 | 1,970 | 18.20 |
| | 22 | 21.54 | | | 9.68 | 3.9 | 14,800 | 1,950 | 18.2 |

(continued)

| Ex. No. | Months at RT* | TS (wt.%) | pH | Viscosity (mPa·s) | Acid No. | pH | 2% Viscosity** (mPa·s) | Yield Value (dyn / $cm^2$) | Turbidity (NTU) |
|---|---|---|---|---|---|---|---|---|---|
| | | Latex properties | | | | Gel Properties | | | |
| 7 | 0 | 21.03 | 8.12 | 9.0 | 7.11 | 4.01 | 5,800 | 508 | 4.72 |
| | 1 | 21.03 | 7.91 | 8.0 | 7.71 | 4.17 | 5,600 | 496 | 4.86 |
| | 2 | 21.03 | | | | 4.09 | 5,700 | 464 | 5.22 |
| | 5 | 21.15 | 7.79 | ND | 8.79 | 3.98 | 6,350 | 544 | 4.94 |
| | 9.5 | 21.25 | 7.91 | good | 11.04 | 4.00 | 5,600 | 464 | 5.25 |
| | 12 | 21.56 | 7.74 | 15.0 (#3) | 8.85 | 3.71 | 5,600 | 486 | 5.02 |
| | 22 | 21.56 | | | 8.64 | 4.03 | 5,750 | 524 | 5.06 |
| 9 | 0 | 21.34 | 7.06 | 9.5 | 8.22 | 4.03 | 14,700 | 1,810 | 18.3 |
| | 4 | 21.30 | 6.94 | 8.5 | 8.35 | 4.02 | 16,000 | 1,850 | 17.9 |
| | 8 | 21.32 | 6.97 | 9.0 | 8.08 | 4.05 | 13,400 | 1,710 | 18.2 |
| | 12 | 21.32 | 7.05 | 9.5 | 7.68 | 4.04 | 12,900 | 1,630 | 19.8 |
| 14 | 0 | 20.98 | 8.96 | 9.5 | 15.10 | 4.07 | 15,300 | 1,960 | 8.64 |
| | 2 | 20.89 | 8.72 | 9.5 | 15.86 | 3.99 | 12,700 | 1,660 | 7.86 |
| | 6 | 20.71 | 8.68 | 9.0 | 15.70 | 4.06 | 14,200 | 1,830 | 8.00 |
| | 9 | 20.95 | 8.67 | 10.0 | 15.72 | 4.07 | 11,700 | 1,480 | 8.54 |
| 15 | 0 | 21.14 | 8.41 | 10.0 | 14.25 | 3.95 | 22,000 | 3,110 | 4.03 |
| | 2 | 20.86 | 8.30 | 9.5 | 16.93 | 3.91 | 18,700 | 2,380 | 3.97 |
| | 6 | 20.74 | 8.36 | 9.5 | 19.42 | 4.11 | 18,700 | 2,540 | 4.43 |
| | 9 | 20.94 | 8.42 | 11.5 | 19.81 | 4.09 | 18,500 | 2,350 | 4.26 |

*RT = ambient room temperature (approximately 20° - 25° C)
**2 wt. % polymer solids mucilage neutralized to pH 4 with a 50:50 (wt./wt.) $H_2O$/glycolic acid solution.

Table 10: One Year Aging Study - Clear Bath Gel Data

| Ex. No. | Months at RT | pH | Viscosity, (mPa·s) | Yield Value (dyn/cm$^2$) | Turbidity (NTU) | pH | Viscosity (mPa·s) | Yield Value (dyn/cm$^2$) | Turbidity (NTU) | pH | Viscosity (mPa·s) | Yield Value (dyn/cm$^2$) | Turbidity (NTU) | Suspension (visual rating) |
|---|---|---|---|---|---|---|---|---|---|---|---|---|---|---|
| | | Set 1 pH 6 | | | | Set 2 pH 4 | | | | Set 3 pH 6 | | | | |
| 1 | 1 | 6.16 | 8,100 | 116 | 143 | 4.19 | 9,800 | 112 | 146 | 6.70 | 10,100 | 100 | 163 | |
| 1 | 13.5 | 6.08 | 7,000 | 64 | 160 | 4.00 | 10,100 | 76 | 171 | 6.06 | 10,200 | 56 | 191 | |
| 2 | 0 | 6.06 | 10,800 | 140 | 66.1 | 4.19 | 13,200 | 148 | 66.3 | 6.73 | 12,900 | 128 | 77.4 | |
| 2 | 13.5 | 6.07 | 9,700 | 92 | 75.9 | 4.09 | 12,500 | 92 | 79.4 | 6.09 | 12,500 | 84 | 91.5 | |
| 2a | 0 | 5.78 | 12,000 | 224 | 65.9 | 4.12 | 15,000 | 232 | 67.5 | 6.17 | 14,800 | 192 | 78.5 | |
| 2a | 12 | 5.94 | 11,100 | 140 | 76.7 | 4.10 | 13, 700 | 152 | 76.7 | 6.09 | 14,100 | 124 | 88.5 | |
| 4 | 0 | 6.14 | 12,200 | 212 | 77.9 | 4.17 | 15,600 | 204 | 75.3 | 6.42 | 14,600 | 276 | 92.1 | 0 |
| 4 | 12 | 5.88 | 12,800 | 196 | 76.8 | 4.05 | 16,800 | 308 | 78.7 | 6.19 | 16,500 | 156 | 95.1 | 1 |
| 6 | 0 | 5.98 | 11,000 | 236 | 56.4 | 4.15 | 14,600 | 300 | 54.3 | 6.05 | 14,300 | 256 | 65.7 | 4 |
| 6 | 12 | 5.96 | 12,000 | 184 | 61.8 | 4.16 | 14,200 | 188 | 61.5 | 6.17 | 14,700 | 148 | 74.4 | 4 |
| 7 | 0 | 6.01 | 17,700 | 208 | 9.07 | 4.13 | 21,800 | 220 | 9.43 | 6.15 | 23,200 | 270 | 12.1 | 0 |
| 7 | 12 | 5.90 | 17,500 | 196 | 11.3 | 4.13 | 22,200 | 210 | 12.1 | 5.86 | 22,600 | 280 | 16.0 | 0 |
| 8 | 0 | 6.08 | 14,300 | 296 | 51.8 | 4.11 | 19,200 | 450 | 50.5 | 5.93 | 19,400 | 360 | 57.4 | 4 |
| 8 | 11 | 6.01 | 13,400 | 236 | 58.7 | 3.93 | 15,400 | 204 | 58.8 | 6.08 | 17,800 | 184 | 71.4 | 2 |
| 9 | 0 | 6.06 | 10,300 | 236 | 67.9 | 4.09 | 14,500 | 310 | 65.7 | 6.58 | 12,200 | 168 | 90.0 | 3 |
| 9 | 11 | 5.89 | 10,400 | 164 | 76.1 | 4.04 | 13,600 | 172 | 76.3 | 5.95 | 13,000 | 134 | 90.5 | 4 |
| 14 | 0 | 5.96 | 10,500 | 208 | 31.9 | 4.00 | 13,600 | 208 | 32.1 | 5.94 | 14,100 | 204 | 37.3 | 2 |
| 14 | 9 | 5.95 | 12,400 | 180 | 36.8 | 4.06 | 16,900 | 220 | 37.0 | 5.94 | 17,300 | 180 | 45.1 | 1 |
| 15 | 0 | 6.00 | 12,900 | 286 | 15.8 | 3.94 | 16,600 | 286 | 15.8 | 5.97 | 16,400 | 282 | 18.4 | 1 |
| 15 | 9 | 5.68 | 18,800 | 370 | 17.2 | 3.98 | 24,200 | 390 | 17.9 | 6.00 | 23,900 | 370 | 22.9 | 2 |
| RT = ambient room temperature (approximately 20° - 25° C) | | | | | | | | | | | | | | |

Table 10 provides bath gel properties (pH, viscosity, YV, clarity and suspension) for several aged multi-purpose polymers using back-alkaline procedure.

**Quaternized Polymers for Hair Fixatives:**

The multi-purpose polymers of Examples 45 and 46, as shown in Table 11, are prepared and subsequently quaternized (theoretical value of 50% degree of quaternization) using diethyl sulfate at approximately 60°C. Both quaternized polymers are pourable with viscosities of 670 and 1,880 mPa·s. The turbidities are 160 and 248 NTU, respectively.

Table 11: Composition

| Example Number | EA | MMA | RAL 307 | CSEM | HEMA | DMADMPMA | Diethyl Sulfate |
|---|---|---|---|---|---|---|---|
| 45 | 54.2 | | 4 | 5 | 1.8 | 35 | 2.75 |
| 46 | 20.0 | 39.2 | 4 | 0 | 1.8 | 35 | 2.75 |

Two formulations of a fixative hair spray are made using the polymer of Example 46. A first sample is formulated by dispersing 5 wt.% TS of the polymer in a hydroalcoholic medium containing 55 wt.% ethanol in water. The second sample is formulated by dispersing 5 wt.% TS of the polymer in water only. The pH of both formulations is adjusted using 50 wt.% glycolic acid solution in water. The alcohol formulation is adjusted to approximately pH 5, and the water only formulation is adjusted to approximately pH 4. Viscosity and % transmittance properties are measured and reported in Table 12.

Table 12: Hair Spray Fixative Formulations Using The Polymer Example 46

| | 55 wt.% Hydroalcoholic Formulation | Water Only Formulation |
|---|---|---|
| pH | 5.12 | 4.09 |
| Viscosity (mPa·s) | 2,290 | 650 |
| Clarity (% transmittance) | 89.4 | 64.4 |

These are tested on mannequin heads with Asian hair using the mannequin head test procedure described above. All samples give fairly stiff and somewhat flexible hold. All samples exhibit good durability.

**Amine Oxide Polymers - Solution Polymers for Hair Fixatives:**

Amine copolymers (Examples 47-50) are prepared using DMADMPMA as a comonomer and converting them to the respective amine oxide using $H_2O_2$ and compared against DMAEMA based amine oxide polymers. The DMADMPMA polymer gives a lower acid number in solution polymerization than the DMAEMA. A low acid number indicates the better hydrolytic stability of DMADMPMA. Table 13 shows composition details for each copolymer and Table 14 describes nice gel characteristics. All amine oxide polymers are tested in Carbopol® Ultrez 21 Polymer gels along with commercial material, PVP K90. All samples are tested on mannequin heads.

Table 13: Composition for Amine Oxide Polymers

| Ex. No. | DMAEMA | DMADMPMA | MMA | SMA | VP | Mam | $H_2O_2$ | Solvent | Acid No. (mg $KOH/_g$) |
|---|---|---|---|---|---|---|---|---|---|
| 47 | 70 | 0 | 10 | 20 | 0 | 0 | 1.1 ratio | IPA | NA |
| 48 | 0 | 70 | 10 | 20 | 0 | 0 | 1.1 ratio | IPA | NA |
| 49 | 60 | 0 | 0 | 0 | 20 | 20 | 1.1 ratio | EtOH | 40.45 |
| 50 | 30 | 30 | 0 | 0 | 20 | 20 | 1.1 ratio | EtOH | 33.34 |

Table 14: Hair Gel Properties

| Sample | K90 | Ex. 47 | Ex. 48 | Ex. 49 | Ex. 50 |
|---|---|---|---|---|---|
| Wt.% TS | 100.00 | 30.00 | 25.00 | 32.68 | 31.40 |
| DI Water | QS | QS | QS | QS | QS |
| Polymer | 2 | 2 | 2 | 2 | 2 |
| Carbopol® Ultrez 21* Polymer | 0.3 | 0.3 | 0.3 | 0.3 | 0.3 |
| Glydant Plus* | 0.3 | 0.3 | 0.3 | 0.3 | 0.3 |
| DI Water | 146.1 | 139.1 | 136.7 | 139.9 | 139.5 |
| Polymer | 3.0 | 10.0 | 12.0 | 9.2 | 9.6 |
| Carbopol® Ultrez 21* Polymer | 0.45 | 0.45 | 0.45 | 0.45 | 0.45 |
| AMP 95 | 0.45 | 0.45 | 0.45 | 0.45 | 0.45 |
| pH | 8.07 | 8.01 | 7.82 | 9.16 | 9.11 |
| Viscosity (mPa·s) | 31,200 | 9,600 | 17,200 | 13,600 | 11,500 |
| Clarity (% transmittance) | 72.0 | 7.2 | 15.6 | 87.2 | 24.9 |
| Yield Value. (dyn/$cm^2$) | 2,940 | 328 | 920 | 850 | 570 |
| Gel Characteristics | Nice, smooth | Nice, smooth | Nice, smooth | Nice, smooth | Nice, smooth |

*Carbopol® Ultrez 21 Rheology Modifier, Lubrizol Advanced Materials, Inc. - INCI Name: Acrylates/C10-30 Alkyl Acrylate Crosspolymer
**Glydant Plus®- DMDM Hydantoin (and) Iodopropynyl Butylcarbamate
***AMP-95 neutralizing amine is 2-amino-2-methyl-1-propanol + 5% water, Dow Chemical Company

The following procedures further exemplify formulations containing the multi-purpose polymers of the invention.

Table 15: Sulfate-Free Acne Cleanser

| Ingredients (INCI - Trade Name) | Weight % |
|---|---|
| Part A | |
| DI Water | q.s. |
| Polymer (Control 3*, 17, 20, 28, 37,38, 39, 40, 42, 43, 44) | 1.5 wt.% TS |
| Sodium $C_{14}$-$C_{16}$ Olefin Sulfonate (40 wt.%; Bio-Terge AS-40) | 17.5 |
| Citric acid (50%) | 0.1 |
| Part B | |
| DI Water | 10.00 |
| Sodium $C_{14}$-$C_{16}$ Olefin Sulfonate (40 wt.%TS; Bio-Terge AS-40) | 17.5 |
| Salicylic acid | 2.00 |
| Part C | |
| Cocamidopropyl Betaine (35%), Chembetaine™ CAD Surfactant | 10.00 |
| Glycerin | 1.00 |
| PEG/PPG-8/3 Laurate (Hydramol PGPL) | 1.00 |
| Tocopheryl acetate | 0.10 |

(continued)

| | |
|---|---|
| Part C | |
| Tea tree oil, propylene glycol (Herbasol Tea Tree Extract PG) | 0.10 |
| Unispheres YE-501** Cosemetic Beads | 0.30 |
| Part D | |
| Citric acid (50% aqueous wt./wt.) to pH 4.0 | 0.5 |
| *Polyacrylate-1 Crosspolymer<br>**Bead carrier for yellow pigment and vitamin E, Induchem U.S.A., Inc. | |

**Procedure:**

The components set forth in Table 15 are formulated into a sulfate-free acne cleanser as follows: (1) Combine Part A ingredients in order and preneutralize with citric acid and mix until uniform; (2) in a separate vessel combine Part B. Mix salicylic acid until completely dissolved; (3) add Part B to Part A and mix until uniform; (4) Add Part C ingredients to Part A in order and mix until uniform; (5) adjust final pH to 4.0 with Citric Acid. Each formulation was evaluated for viscosity and turbidity. Results are reported in Table 16.

Table 16: Polymer Performance in Sulfate-Free Acne Cleanser

| Example Number | Viscosity (24 Hrs, mPa·s) | Turbidity (NTU) |
|---|---|---|
| Control 3* | 7,000 | 22 |
| 17 | 11,350 | 55.4 |
| 20 | 8,900 | 83 |
| 28 | 6,320 | 47.3 |
| 37 | 9,840 | 24.6 |
| 38 | 6,500 | 35.2 |
| 39 | 7,900 | 83.2 |
| 40 | 5,450 | 101 |
| 42 | 7,900 | 29.3 |
| 43 | 10,000 | 17.2 |
| 44 | 14,500 | 11.2 |
| 44a | 12,650 | 13.0 |
| 44b | 11,000 | 40.7 |
| *Polyacrylate-1 Crosspolymer | | |

Table 17: Conditioner

| Ingredients (INCI - Trade Name) | Weight % |
|---|---|
| DI Water | Q.S |
| Polymer (Control 3*, 17, 20, 28, 37,38, 39, 40, 42, 43, and 44, 44a, and 44b) | 0.30 wt.% TS |
| Cetearyl Alcohol | 3.00 |
| Steralkonium Chloride | 1.00 |
| DMDM Hydantoin (and) Iodopropynyl Butylcarbamate, Glydant Plus® | 1.00 |

(continued)

| Ingredients (INCI - Trade Name) | Weight % |
|---|---|
| Citric acid (50% aqueous wt./wt.) | q.s. to pH 4.0 to 4.5 |
| *Polyacrylate-1 Crosspolymer | |

**Procedure:**

The components set forth in Table 17 are formulated as a conditioner as follows: (1) Heat water to 72°C to 75°C and when the temperature is reached add cetearyl alcohol and mix until the fatty alcohol is completely melted; (2) switch off the hot plate and add steralkonium chloride. When all of the steralkonium chloride is melted, begin cooling and remove from hot plate so that it cools faster; (3) when temperature reaches 30°C to 35°C, add Glydant® plus and mix well, and add the polymer and mix until uniform; (4) adjust pH with citric acid (pH 4 to 4.5). Viscosity at 24 hrs. is measured for each of the formulations and set forth in Table 18.

Table 18: Polymer Performance in Conditioner Formulation

| Example Number | Viscosity (24 Hrs, mPa·s) |
|---|---|
| Control 3* | 11,450 |
| 17 | 11,200 |
| 20 | 9,300 |
| 28 | 14,000 |
| 37 | 15,550 |
| 38 | 11,550 |
| 39 | 10,100 |
| 40 | 10,150 |
| 42 | 9,200 |
| 43 | 9,100 |
| 44 | 7,900 |
| 44a | 4,730 |
| 44b | 3,030 |
| *Polyacrylate-1 Crosspolymer | |

Table 19: Anti-Dandruff Shampoo

| Ingredients (INCI - Trade Name) | Weight % |
|---|---|
| DI Water | 49.39 |
| Polyquaternium-10, Ucare® Polymer JR-30M, Conditioner, Dow Chemical | 0.25 |
| Polymer Example Number. 44 | 6.96 |
| Sodium Lauryl Sulfate (30%), Sulfochem™ SLS-K Surfactant, Lubrizol Advanced Materials, Inc. | 16.00 |
| Sodium Laureth Sulfate (2 mole, 28%), Sulfochem™ ES-2 Surfactant, Lubrizol Advanced Materials, Inc. | 16.00 |
| Cocamidopropyl Betaine (35%), Chembetaine™ CAD Surfactant, Lubrizol Advanced Materials, Inc. | 4.00 |
| Zinc Pyrithione, Zinc Omadine® FPS, Anti-dandruff agent | 2.50 |
| Dimethicone (and) Laureth-4 (and) Laureth-23, Dow Corning® 1664 Emulsion | 3.00 |
| Blue 1 (0.1 %) | 0.50 |

(continued)

| Ingredients (INCI - Trade Name) | Weight % |
|---|---|
| Fragrance, XBF-800404-Lavender Breeze | 0.30 |
| DMDM Hydantoin (and) Iodopropynyl Butylcarbamate, Glydant Plus® | 0.30 |
| Fragrance, Urtica Dioica (Stinging Nettle) Extract, Propylene Glycol, Herbasol® Stinging Nettle Extract PG | 0.30 |
| Citric acid (50% aqueous wt./wt.) to pH 5.0 | 0.50 |
| Viscosity (mPa·s) | 11,000 mPa·s |

**Procedure:**

The components set forth in Table 19 are formulated into a anti-dandruff shampoo as follows: (1) Combine ingredients in order and mix until uniform except polymer and citric acid; (2) post-add polymer to the above mixture and mix until uniform; (3) adjust final pH to 5.0 with citric acid.

Table 20: Two-in-one Conditioning Shampoo

| INGREDIENTS (INCI / Trade Name) | WEIGHT % |
|---|---|
| DI Water | 82.50 |
| Sodium Benzoate | 0.25 |
| Mica/$TiO_2$ (Timiron® MP-1001 from EMD) | 0.05 |
| Sodium Laureth Sulfate (1 mole, 28%), (Sulfoche™ ES-1 Surfactant, Lubrizol Advanced Materials, Inc.) | 12.00 |
| Cocamidopropyl Betaine (35%), (Chembetaine™ CGF Surfactant from Lubrizol Advanced Materials, Inc.) | 2.00 |
| Dow Corning® 1784 Emulsion, (Dimethyl Silicone Emulsion from Dow Corning) | 2.00 |
| Jaguar C-13s, (Guar Hydroxypropyl Trimonium Chloride from Rhodia) | 0.20 |
| Polymer Ex. No.: Control 3*, Control 4**, Control 5***, 17, 28, 37, 38, 39, 40, 42, 43, 45 and 46 | 1.00 |
| Citric Acid (50% aqueous wt./wt.) | qs to pH 5.5 |
| *Polyacrylate-1 Crosspolymer<br>**Formulation does not contain test polymer; contains 0.5% NaCl in addition to the other components listed in Table 20<br>***Formulation does not contain test polymer and Jaguar C-13s; contains 0.5% NaCl in addition to the other components listed in Table 20 | |

**Procedure:**

Two-in-one conditioning shampoos are formulated by (1) Combining the ingredients in the order listed from the top to bottom of Table 20, excluding citric acid, and mixing until homogeneous shampoo formulation is obtained; and (2) adjusting the final pH of each shampoo formulation with citric acid. Viscosity at 24 hrs. is measured and reported in Table 21 below. Wet Combability values and silicone deposition values are measured for the compositions that include the polymers of Examples 42, 45, and 46 and recorded in Table 22.

Table 21: Two-in-one Conditioning Shampoo

| Example No. | Viscosity (24 Hrs, mPa·s) |
|---|---|
| Control 3 | 7,398 |
| Control 4 | 7,638 |
| 17 | 11,946 |

(continued)

| Example No. | Viscosity (24 Hrs, mPa·s) |
|---|---|
| 28 | 8,073 |
| 37 | 10,249 |
| 38 | 7,116 |
| 39 | 10,467 |
| 40 | 9,292 |
| 42 | 8,813 |
| 43 | 10,184 |
| 45 | 1,197 |
| 46 | 3,395 |

Table 22: Polymer Performance in 2-in-1 Shampoo

| (Wet Combing and Silicone Deposition) | | | |
|---|---|---|---|
| Example No. | % Reduction of Total Combing Work | % Reduction of Detangling Force | Silicone Deposition* (kcps) |
| Control-3** | 58 | 44 | 4.0 |
| Control-4*** | 68 | 69 | 12.2 |
| Control-5**** | 6 | 5 | 2.0 |
| 42 | 61 | 10 | 2.7 |
| 45 | 79 | 88 | 14.8 |
| 46 | 68 | 70 | 7.0 |

*Average of 3 test tresses.
**Polyacrylate-1 Crosspolymer
***Formulation does not contain test polymer; contains 0.5% NaCl in addition to the other components listed in Table 20
****Formulation does not contain test polymer and Jaguar C-13s; contains 0.5% NaCl in addition to the other components listed in Table 20

## Claims

**1.** A composition comprising:

A) a hydrolytically stable polymer polymerized from a monomer mixture selected from:

(i) a mixture comprising (a), (b) and (c);
(ii) a mixture comprising (a), (b) and (d);
(iii) a mixture comprising (a), (b) and (e);
(iv) a mixture comprising (a), (b), (c) and (d);
(v) a mixture comprising (a), (b), (c) and (e);
(vi) a mixture comprising (a), (b), (d) and (e); or
(vii) a mixture comprising (a), (b), (c), (d) and (e)

where monomer component (a) is at least one amino-substituted meth(acrylate) (ASMA) monomer or salt thereof; monomer component (b) is at least one nonionic vinyl (NIV) monomer; monomer component (c) is at least one vinyl associative (VA) monomer; monomer component (d) is at least one vinyl surfactant (VS) monomer; monomer component (e) is at least one polymerizable silicone macromer (PSM), and wherein any of the above mixtures further optionally contain: (f) at least one crosslinking (XL) monomer; (g) at least one chain transfer

agent (CTA); (h) at least one polymeric stabilizer; or any suitable combination of two or more of components (f), (g) and/or (h); and

B) a component selected from a surfactant, a monoquaternium compound, a polyquaternium compound, an acidic compound, and combinations thereof, wherein monomer component (a) is selected from at least one compound, or salt thereof, represented by Formulas (I) and (II):

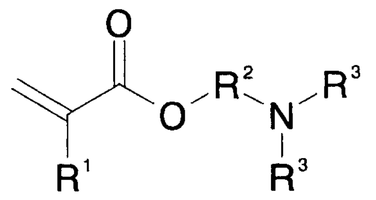

(I)

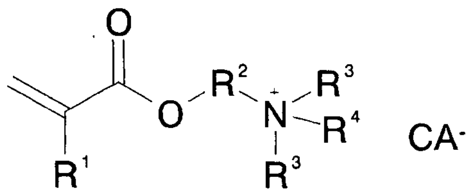

(II)

where $R^1$ is methyl; where $R^2$ is a substituted or unsubstituted, linear or branched $C_2$ to $C_8$ alkanediyl group, with the proviso that when $R^2$ has two carbons at least one of the two carbon atoms of the $R^2$ group is substituted with a linear or branched $C_1$ to $C_{30}$ alkyl group; where each $R^3$ is independently selected from hydrogen, linear or branched $C_1$ to $C_{30}$ alkyl groups, linear or branched $C_1$ to $C_{30}$ alkyl groups that contain one or more heteroatoms, linear or branched $C_2$ to $C_{30}$ alkenyl groups, linear or branched $C_2$ to $C_{30}$ alkenyl groups that contain one or more heteroatoms, linear or branched $C_2$ to $C_{30}$ alkynyl groups, linear or branched $C_2$ to $C_{30}$ alkynyl groups that contain one or more heteroatoms, $C_4$ to $C_{20}$ aryl groups, $C_4$ to $C_{20}$ aryl groups that contain one or more heteroatoms, $C_4$ to $C_{20}$ cycloalkyl groups, $C_4$ to $C_{20}$ cycloalkyl groups that contain one or more heteroatoms, $C_4$ to $C_{20}$ heterocyclic groups, or where both $R^3$ substituents and the nitrogen atom to which they are attached can form a saturated or unsaturated $C_2$ to $C_{20}$ heterocyclic group or a saturated or unsaturated $C_2$ to $C_{20}$ heterocyclic group having two or more heteroatoms, where the heteroatoms, if present, are selected from a carbonyl group, N, S, P or O; and where $R^4$ is a linear or branched $C_1$ to $C_{30}$ alkyl group; and where CA is a counter-anion suitable to balance the charge on the quaternary ammonium moiety;

wherein monomer component (b) is selected from at least one copolymerizable, nonionic, ethylenically unsaturated monomer represented by Formulas (III) and (IV):

$C(X)_2=C(X)Z$ (III)

$CH_2=CH-OC(O)R_1$ (IV)

wherein, in each of Formulas (III) and (IV), each X is independently hydrogen, methyl, $-CH_2C(O)OR_1$, $-C(O)OR_1$; and Z is $-C(O)OR_1$, $-C_6H_4R_1$, $-C_6H_4OR_1$, -CN, $-C(O)N(R_1)_2$, $-NHC(O)CH_3$, -NHC(O)H, $-C(O)OA'OR_{15}$ N-(2-pyrrolidonyl), N-caprolactamyl, $-C(O)NHCH_2CH_2$-N-ethyleneurea, or $-C(O)NHC(CH_3)_3$; A' is a divalent radical selected from $-CH_2CH(OH)CH_2-$ and $-CH_2CH(CH_2OH)-$, each $R_1$ is independently linear and branched $C_1$ to $C_{30}$ alkyl, hydroxy-substituted $C_2$ to $C_{30}$ alkyl, $C_5$ to $C_{30}$ cycloalkyl, and $C_1$ to $C_5$ alkyl-substituted $C_5$ to $C_{30}$ cycloalkyl; $R_{15}$ is an acyl residue of a linear or branched, saturated or unsaturated $C_6$ to $C_{22}$ fatty acid;

wherein monomer component (c) is selected from at least one monomer represented by Formula (V):

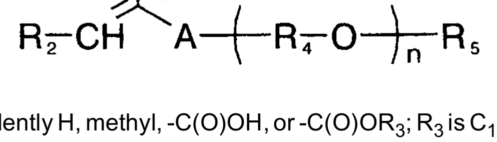

(V)

wherein, each $R_2$ is independently H, methyl, -C(O)OH, or $-C(O)OR_3$; $R_3$ is $C_1$ to $C_{30}$ alkyl; A is $-CH_2C(O)O-$, -C(O)O-,

-O-, or $-CH_2O-$; $(R_4-O)_n$ is a polyoxyalkylene, which is a homopolymer, a random copolymer, or a block copolymer of $C_2$ to $C_4$ oxyalkylene units, wherein each $R_4$ is independently $C_2H_4$, $C_3H_6$, $C_4H_8$, or a mixture thereof, and n is an integer in the range of about 5 to about 250; and wherein $R_5$ is a substituted or unsubstituted alkyl selected from linear or branched $C_8$ to $C_{40}$ alkyls, $C_8$ to $C_{40}$ carbocyclic alkyls, $C_2$ to $C_{40}$ alkyl-substituted phenyls, aryl-substituted $C_2$ to $C_{40}$ alkyls, and $C_8$ to $C_{80}$ complex esters, wherein the $R_5$ alkyl group optionally comprises one or more substituents selected from a hydroxyl group, an alkoxyl group, or a halogen group;
wherein monomer component (d) is selected from at least one monomer represented by Formula (VI):

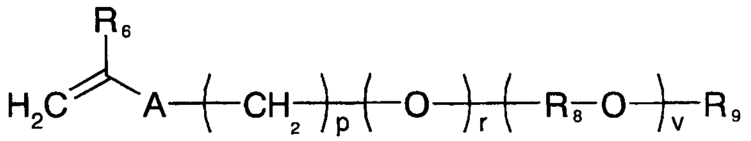

(VI)

where each $R_6$ is independently hydrogen or methyl, -C(O)OH, or $-C(O)OR_7$; $R_7$ is $C_1$ to $C_{30}$ alkyl; A is $-CH_2C(O)O-$, -C(O)O-, -O-, or $-CH_2O-$; p is an integer in the range of 0 to 30, and r is 0 or 1, with the proviso that when p is 0, r is 0, and when p is in the range of 1 to 30, r is 1; $(R_8-O)_v$ is a polyoxyalkylene, which is a homopolymer, a random copolymer or a block copolymer of $C_2$ to $C_4$ oxyalkylene units, wherein each $R_8$ is independently $C_2H_4$, $C_3H_6$, $C_4H_8$, or a mixture thereof, and v is an integer in the range of 5 to 250; and $R_9$ is hydrogen or $C_1$ to $C_4$ alkyl; and
wherein monomer component (e) is selected from at least one polymerizable silicone macromer represented by the following Formula:

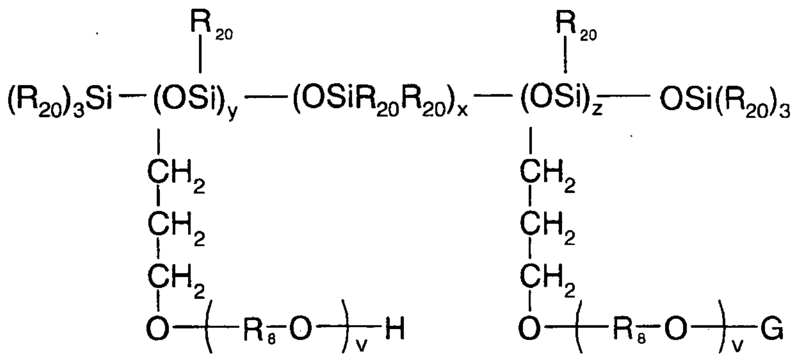

where each $R_{20}$ is independently selected from linear or branched $C_1$ to $C_{30}$ alkyl, $C_4$ to $C_{20}$ aryl, or $C_2$ to $C_{20}$ alkeneyl; where $(R_8-O)_v$ is a polyoxyalkylene moiety, which can be arranged as a homopolymer, a random copolymer or a block copolymer of $C_2$ to $C_4$ oxyalkylene units, wherein each $R_8$ is independently $C_2H_4$, $C_3H_6$, $C_4H_8$, or a mixture thereof, and v is an integer in the range of 5 to 250; x is an integer in the range of 0 to 200; y is an integer in the range of 0 to 200; and z is an integer in the range of 1 to 200; and where G is selected from any moiety that contains at least one free radically polymerizable carbon-carbon double bond.

2. The composition of claim 1, wherein CA is selected from a halogen, a sulfate, a sulfonate, methosulfate, a phosphate, or a phosphonate, acetate, formate, citrate, maleate glycolate, lactate, a fumarate, and nitrate.

3. The composition of claim 1, wherein monomer component (a) is one or more monomers selected from 3-(dimethylamino)propyl methacrylate, 2-(dimethylamino)propan-2-yl methacrylate, 3-(dimethylamino)-2,2-dimethylpropyl methacrylate, 2-(dimethylamine)-2-methylpropyl methacrylate, 4-(dimethylamine)butyl methacrylate, salts of one or more thereof, or mixtures of any one or more thereof.

4. The composition of claim 1, wherein one or more monomers according to Formula (I) are reacted with hydrogen peroxide ($H_2O_2$) to yield an amine oxide compound represented by the following Formula (Ia):

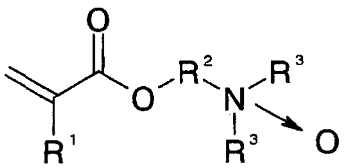

(Ia)

where $R^1$, $R^2$ and $R^3$ are as defined above.

5. The composition of claim 1, wherein monomer component (b) is selected from at least one $C_1$ to $C_{30}$ alkyl (meth)acrylate, at least one hydroxy $C_2$ to $C_{30}$ alkyl (meth)acrylate, at least one $C_1$ to $C_{30}$ alkyl (meth)acrylamide, at least one styrene; at least one substituted styrene, at least one vinyl ester, at least one unsaturated nitrite, the reaction product of glycidyl t-decanoate and acrylic acid, the reaction product of glycidyl t-decanoate and methacrylic acid, or any combination of two or more thereof.

6. The polymer of claim 1, wherein monomer component (b) is selected from methyl acrylate, ethyl acrylate, methyl methacrylate, n-butyl acrylate, 2-ethylhexyl acrylate, 3,3,5-trimethylcyclohexyl methacrylate, stearyl methacrylate, and suitable mixtures of any two or more thereof.

7. The composition of claim 1, wherein monomer component (c) is selected from one or more of cetyl polyethoxylated methacrylate, cetearyl polyethoxylated methacrylate, stearyl polyethoxylated (meth)acrylate, arachidyl polyethoxylated (meth)acrylate, behenyl polyethoxylated methacrylate, lauryl polyethoxylated methacrylate, cerotyl polyethoxylated (meth)acrylate, montanyl polyethoxylated (meth)acrylate, melissyl polyethoxylated (meth)acrylate, lacceryl polyethoxylated (meth)acrylate, tristyryl phenolpolyethoxylated methacrylate, hydrogenated castor oil polyethoxylated methacrylate, canola polyethoxylated (meth)acrylate, and cholesterol polyethoxylated methacrylate, where the polyethoxylated portion of the monomer comprises 5 to 100 ethylene oxide repeating units.

8. The composition of claim 1, wherein monomer component (d) is selected from at least one monomer having one of the following chemical Formulas:

$H_2C{=}CH{-}O(CH_2)_aO(C_3H_6O)_b(C_2H_4O)_cH$;

$H_2C{=}CHCH_2O(C_3H_6O)_d(C_2H_4O)_eH$;

$H_2C{=}C(Q){-}C(O){-}O(CH_2)_aO(C_3H_6O)_b(C_2H_4O)_cH$; or

$H_2C{=}C(Q){-}C(O){-}O(C_3H_6O)_d(C_2H_4O)_eH$

wherein Q is hydrogen or methyl; a is 2, 3, or 4; b is an integer in the range of 1 to 10; c is an integer in the range of 5 to 50; d is an integer in the range of 1 to 10; and e is an integer in the range of 5 to 50.

9. The composition of claim 1, wherein said monoquaternium compound is selected from acetamidopropyl trimonium chloride, behenamidopropyl dimethylamine, behenamidopropyl ethyldimonium ethosulfate, behentrimonium chloride, cetethyl morpholinium ethosulfate, cetrimonium chloride, cocoamidopropyl ethyldimonium ethosulfate, dicetyldimonium chloride, dimethicone hydroxypropyl trimonium chloride, hydroxyethyl behenamidopropyl dimonium chloride, quaternium-26, quaternium-27, quaternium-53, quaternium-63, quaternium-70, quaternium-72, quaternium-76 hydrolyzed collagen, stearalkonium chloride, stearamidopropyl ethyl dimonium ethosulfate, steardimonium hydroxypropyl hydrolyzed wheat protein, steardimonium hydroxypropyl hydrolyzed collagen, wheat germamidopropalkonium chloride, wheat germamidopropyl ethyldimonium ethosulfate and combinations thereof, or wherein said polyquaternium compound is selected from polyquaternium-4, polyquaternium-7, polyquaternium-10, polyquaternium-11, polyquaternium-15, polyquaternium-16, polyquaternium-22, polyquaternium-24, polyquaternium-28, polyquaternium-29, polyquaternium-32, polyquaternium-33, polyquaternium-34, polyquaternium-35, polyquaternium-37, polyquaternium-39, polyquaternium-43, polyquaternium-44, polyquaternium-46, polyquaternium-47, polyquaternium-55, polyquaternium-60, polyquaternium-66, polyquaternium-67, polyquaternium-68, polyquaternium-69, polyquaternium-72, polyquaternium-77, polyquaternium-85, polyquaternium-86, polyquaternium-87, and combinations thereof.

10. The composition of claim 1, wherein said acidic compound is selected from an organic acid, a mineral acid, and

mixtures thereof.

11. The composition of claim 10, wherein said acidic compound is selected from hydrochloric acid, nitric acid, sulfuric acid, phosphoric acid, sodium bisulfate, and mixtures thereof, or
wherein said acidic compound is selected from alpha-hydroxy acids (AHAs), beta-hydroxy acids (BHAs), alpha amino-acids, alpha-keto acids, and mixtures thereof, or
wherein said acidic compound is selected from lactic acid, acetic acid, fumaric acid, glycolic acid, malic acid, citric acid, tartaric acid, 2-hydroxyoctanoic acid, glyceric acid (dihydroxypropionic acid), tartronic acid, gluconic acid, mandelic acid, benzilic acid, alpha-lopioc acid, arginine glycolate, ammonium lactate, sodium lactate, alpha-hydroxybutyric acid, alpha-hydroxyisobutyric acid, alpha-hydroxyisocaproic acid, alpha-hydroxyisovaleric acid, atrolactic acid, 3-hydroxy propanoic acid, beta-hydroxybutyric acid, beta-phenyl lactic acid, beta-phenylpyruvic acid, salicylic acid, aspartic acid, glutamic acid, pyruvic acid, acetopyruvic acid, retinoic acid, trichloroacetic acid, phytic acid, lysophosphatidic acid, salicylic acid, 5-octanoylsalicylic acid.

12. The composition of claim 1, wherein said surfactant is selected from an anionic surfactant, a cationic surfactant, an amphoteric surfactant, a nonionic surfactant, and combinations thereof.

13. The composition of claim 1, further comprising a component selected from a conditioner, a moisturizer, antioxidants, particulates, keratolytic agents, botanicals, vitamins, anti-dandruff agents, anti-inflammatory agents, analgesics, antiperspirant agents, deodorant compounds, hair fixative polymers, auxiliary acidifying agents, auxiliary alkalizing agents, rheology modifiers, viscosity control agents, sheen enhancers, a emollient, a humectant, a lubricant, sunscreen agent, UV absorbing agent; oxidizing agent, reducing agent, polymer film modifying agent, chelating agent, opacifier, preservative; fragrance; fragrance solubilizer; colorant, propellant, buffering agent; fixative, formers; and combinations thereof.

14. The composition of claim 13, wherein said conditioner is a silicone.

15. The composition of claim 14, wherein said silicone is selected from a silicone fluid, a silicone gum, a silicone resin, and mixtures thereof.

16. The composition of any of claims 1 to 15, wherein said polymer is polymerized from a monomer mixture selected from (ii) comprising (a), (b) and (d), wherein monomer component (a) is selected from at least one of 3-(dimethylamino)propyl methacrylate; 2-(dimethylamino)propan-2-yl methacrylate; 3-(dimethylamino)-2,2-dimethylpropyl methacrylate; 2-(dimethylamino)-2-methylpropyl methacrylate; 4-(dimethylamino)butyl methacrylate, and mixtures thereof; monomer component (b) is selected from at least one of a $C_1$ to $C_{30}$ alkyl (meth)acrylate, hydroxyl-substituted $C_1$ to $C_{30}$ alkyl (meth)acrylate, and mixtures thereof; and monomer component (d) is selected from at least one monomer represented by the formula:

$$H_2C=CHCH_2O(C_3H_6O)_d(C_2H_4O)_eH$$

and mixtures thereof, wherein d ranges from 1 to 20, and e ranges from 5 to 40.

17. The composition of claim 16, wherein said polymer is polymerized from a monomer mixture comprising 3-(dimethylamino)-2,2-dimethylpropyl methacrylate, ethyl acrylate, methyl acrylate, and a monomer represented by the formula $H_2C=CHCH_2O(C_3H_6O)_{2\text{-}10}(C_2H_4O)_{10\text{-}25}H$.

18. The composition of any of claims 1 to 17, wherein said polymer is polymerized from a monomer mixture selected from (ii) comprising (a), (b) and (c), wherein monomer component (a) is selected from at least one of 3-(dimethylamino)propyl methacrylate; 2-(dimethylamino)propan-2-yl methacrylate; 3-(dimethylamino)-2,2-dimethylpropyl methacrylate; 2-(dimethylamino)-2-methylpropyl methacrylate; 4-(dimethylamino)butyl methacrylate, and mixtures thereof; monomer component (b) is selected from at least one of a $C_1$ to $C_{30}$ alkyl (meth)acrylate, and mixtures thereof; and monomer component (c) is selected from at least one monomer selected from one or more of cetyl polyethoxylated methacrylate, cetearyl polyethoxylated methacrylate, stearyl polyethoxylated (meth)acrylate, arachidyl polyethoxylated (meth)acrylate, behenyl polyethoxylated methacrylate, lauryl polyethoxylated methacrylate, cerotyl polyethoxylated (meth)acrylate, montanyl polyethoxylated (meth)acrylate, melissyl polyethoxylated (meth)acrylate, lacceryl polyethoxylated (meth)acrylate, tristyryl phenolpolyethoxylated methacrylate, hydrogenated castor oil polyethoxylated methacrylate, canola polyethoxylated (meth)acrylate, and cholesterol polyethoxylated methacrylate, where the polyethoxylated portion of the monomer comprises about 5 to about 100 ethylene oxide

repeating units.

**19.** A personal care product, a health care product, a household care product, an institutional and industrial care product, or an industrial application product comprising the composition of any of claims 1 to 18.

**Patentansprüche**

**1.** Zusammensetzung, umfassend:

A) ein hydrolysestabiles Polymer, das ausgehend von einem Monomergemisch polymerisiert ist, welches ausgewählt ist aus:

(i) einem Gemisch, das (a), (b) und (c) umfasst;
(ii) einem Gemisch, das (a), (b) und (d) umfasst;
(iii) einem Gemisch, das (a), (b) und (e) umfasst;
(iv) einem Gemisch, das (a), (b), (c) und (d) umfasst;
(v) einem Gemisch, das (a), (b), (c) und (e) umfasst;
(vi) einem Gemisch, das (a), (b), (d) und (e) umfasst; oder
(vii) einem Gemisch, das (a), (b), (c), (d) und (e) umfasst;

wobei Monomerkomponente (a) wenigstens ein aminosubstituiertes Meth(acrylat)-Monomer (ASMA) oder ein Salz davon ist; Monomerkomponente (b) wenigstens ein nichtionisches Vinyl-Monomer (NIV) ist; Monomerkomponente (c) wenigstens ein vinylassoziatives (VA) Monomer ist; Monomerkomponente (d) wenigstens ein Vinyl-Tensid-Monomer (VS) ist; Monomerkomponente (e) wenigstens ein polymerisierbares Silikonmakromer (PSM) ist; und wobei eines der obigen Gemische gegebenenfalls weiterhin enthält: (f) wenigstens ein vernetzendes (XL) Monomer; (g) wenigstens ein Kettenübertragungsmittel (CTA); (h) wenigstens einen polymeren Stabilisator; oder irgendeine geeignete Kombination von zwei oder mehr der Komponenten (f), (g) und/oder (h); und
B) eine Komponente, die aus einem Tensid, einer Monoquaterniumverbindung, einer Polyquaterniumverbindung, einer sauren Verbindung und Kombinationen davon ausgewählt ist;

wobei Monomerkomponente (a) aus wenigstens einer Verbindung, die durch die Formeln (I) und (II) dargestellt wird, oder einem Salz davon ausgewählt ist:

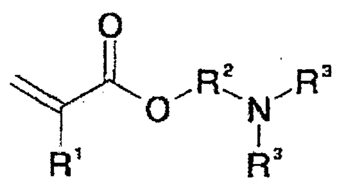

(I)

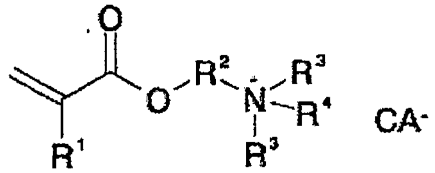

(II)

wobei $R^1$ Methyl ist; wobei $R^2$ eine substituierte oder unsubstituierte, lineare oder verzweigte $C_2$- bis $C_8$-Alkandiylgruppe ist, mit der Maßgabe, dass dann, wenn $R^2$ zwei Kohlenstoffatome aufweist, wenigstens eines der beiden Kohlenstoffatome der $R^2$-Gruppe mit einer linearen oder verzweigten $C_1$- bis $C_{30}$-Alkylgruppe substituiert ist; wobei jedes $R^3$ unabhängig aus Wasserstoff, linearen oder verzweigten $C_1$- bis $C_{30}$-Alkylgruppen, linearen oder verzweigten $C_1$- bis $C_{30}$-Alkylgruppen, die ein oder mehrere Heteroatome enthalten, linearen oder verzweigten $C_2$- bis $C_{30}$-Alkenylgruppen, linearen oder verzweigten $C_2$- bis $C_{30}$-Alkenylgruppen, die ein oder mehrere Heteroatome enthalten, linearen oder verzweigten $C_2$- bis $C_{30}$-Alkinylgruppen, linearen oder verzweigten $C_2$- bis $C_{30}$-Alkinylgruppen, die ein oder mehrere Heteroatome enthalten, $C_4$- bis $C_{20}$-Arylgruppen, $C_4$- bis $C_{20}$-Arylgruppen, die ein oder mehrere Heteroatome enthalten, $C_4$- bis $C_{20}$-Cycloalkylgruppen, $C_4$- bis $C_{20}$-Cycloalkylgruppen, die ein oder meh-

rere Heteroatome enthalten, heterocyclischen $C_4$- bis $C_{20}$-Gruppen ausgewählt ist, oder wobei die beiden $R^3$-Substituenten und das Stickstoffatom, an das sie gebunden sind, eine gesättigte oder ungesättigte heterocyclische $C_2$- bis $C_{20}$-Gruppe oder eine gesättigte oder ungesättigte heterocyclische $C_2$- bis $C_{20}$-Gruppe, die zwei oder mehr Heteroatome aufweist, bilden können, wobei die Heteroatome, falls vorhanden, aus einer Carbonylgruppe, N, S, P oder O ausgewählt sind; und wobei $R^4$ eine lineare oder verzweigte $C_1$- bis $C_{30}$-Alkylgruppe ist; und wobei $CA^-$ ein Gegenanion ist, das die Ladung auf der quartären Ammonium-Struktureinheit ausgleichen kann;
wobei Monomerkomponente (b) aus wenigstens einem copolymerisierbaren, nichtionischen, ethylenisch ungesättigten Monomer, das durch die Formeln (III) und (IV) dargestellt wird, ausgewählt ist:

$C(X)_2=C(X)Z$ (III)

$CH_2=CH-OC(O)R_1$ (IV),

wobei in jeder der Formeln (III) und (IV) X jeweils unabhängig Wasserstoff, Methyl, $-CH_2C(O)OR_1$, $-C(O)OR_1$ ist; und Z = $-C(O)OR_1$, $-C_6H_4R_1$, $-C_6H_4OR_1$, -CN, $-C(O)N(R_1)_2$, $-NHC(O)CH_3$, -NHC(O)H, $-C(O)OA'OR_{15}$, N-(2-Pyrrolidonyl), N-Caprolactamyl, $-C(O)NHCH_2CH_2$-N-ethylenharnstoff oder $-C(O)NHC(CH_3)_3$ ist; A' ein zweiwertiger Rest ist, der aus $-CH_2CH(OH)CH_2$- und $-CH_2CH(CH_2OH)$- ausgewählt ist, jedes $R_1$ unabhängig lineares und verzweigtes $C_1$- bis $C_{30}$-Alkyl, hydroxysubstituiertes $C_2$- bis $C_{30}$-Alkyl, $C_5$- bis $C_{30}$-Cycloalkyl und $C_1$-bis-$C_5$-alkylsubstituiertes $C_5$- bis $C_{30}$-Cycloalkyl ist; $R_{15}$ ein Acylrest einer linearen oder verzweigten, gesättigten oder ungesättigten $C_6$- bis $C_{22}$-Fettsäure ist;
wobei Monomerkomponente (c) aus wenigstens einem Monomer, das durch Formel (V) dargestellt wird, ausgewählt ist:

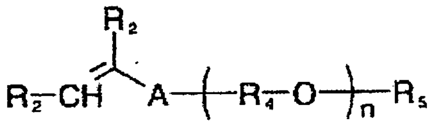
(V) ,

wobei jedes $R_2$ unabhängig H, Methyl, -C(O)OH oder $-C(O)OR_3$ ist; $R_3$ = $C_1$- bis $C_{30}$-Alkyl ist; A = $-CH_2C(O)O$-, -C(O)O-, -O- oder $-CH_2O$- ist; $(R_4\text{-}O)_n$ ein Polyoxyalkylen ist, bei dem es sich um ein Homopolymer, statistisches Copolymer oder Blockcopolymer von $C_2$- bis $C_4$-Oxyalkyleneinheiten handelt, wobei jedes $R_4$ unabhängig $C_2H_4$, $C_3H_6$, $C_4H_8$ oder ein Gemisch davon ist und n eine ganze Zahl im Bereich von etwa 5 bis etwa 250 ist; und wobei $R_5$ ein substituiertes oder unsubstituiertes Alkyl ist, das aus linearen oder verzweigten $C_8$- bis $C_{40}$-Alkylgruppen, $C_8$- bis $C_{40}$-carbocyclischen Alkylgruppen, $C_2$- bis $C_{40}$-alkylsubstituierten Phenylgruppen, arylsubstituierten $C_2$- bis $C_{40}$-Alkylgruppen und komplexen $C_8$- bis $C_{80}$-Estern ausgewählt ist, wobei die $R_5$-Alkylgruppe gegebenenfalls einen oder mehrere Substituenten umfasst, die aus einer Hydroxygruppe, einer Alkoxygruppe oder einer Halogengruppe ausgewählt sind;
wobei Monomerkomponente (d) aus wenigstens einem Monomer, das durch Formel (VI) dargestellt wird, ausgewählt ist:

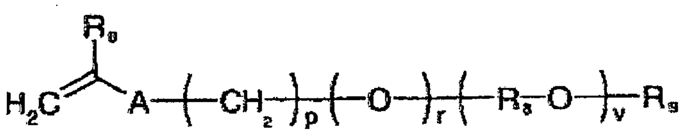
(VI)

wobei jedes $R_6$ unabhängig Wasserstoff oder Methyl, -C(O)OH oder $-C(O)OR_7$ ist; $R_7$ = $C_1$- bis $C_{30}$-Alkyl ist; A = $-CH_2C(O)O$-, -C(O)O-, -O- oder $-CH_2O$- ist; p eine ganze Zahl im Bereich von 0 bis 30 ist und r = 0 oder 1 ist, mit der Maßgabe, dass dann, wenn p = 0 ist, r = 0 ist und wenn p im Bereich von 1 bis 30 liegt, r = 1 ist; $(R_8\text{-}O)_v$ ein Polyoxyalkylen ist, bei dem es sich um ein Homopolymer, statistisches Copolymer oder Blockcopolymer von $C_2$- bis $C_4$-Oxyalkyleneinheiten handelt, wobei $R_8$ jeweils unabhängig $C_2H_4$, $C_3H_6$, $C_4H_8$ oder ein Gemisch davon ist und v eine ganze Zahl im Bereich von 5 bis 250 ist; und $R_9$ Wasserstoff oder $C_1$- bis $C_4$-Alkyl ist; und
wobei Monomerkomponente (e) aus wenigstens einem polymerisierbaren Silikonmakromer, das durch die folgende Formel dargestellt wird, ausgewählt ist:

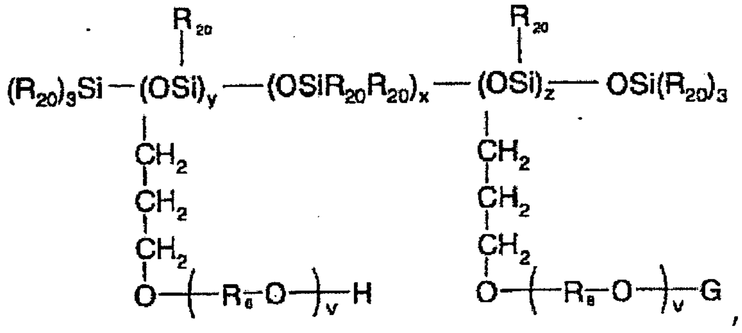

wobei $R_{20}$ jeweils unabhängig aus linearem oder verzweigtem $C_1$- bis $C_{30}$-Alkyl, $C_4$- bis $C_{20}$-Aryl oder $C_2$- bis $C_{20}$-Alkenyl ausgewählt ist; wobei $(R_8\text{-}O)_v$ eine Polyoxyalkylen-Struktureinheit ist, die als Homopolymer, statistisches Copolymer oder Blockcopolymer von $C_2$- bis $C_4$-Oxyalkyleneinheiten angeordnet sein kann, wobei $R_8$ jeweils unabhängig $C_2H_4$, $C_3H_6$, $C_4H_8$ oder ein Gemisch davon ist und v eine ganze Zahl im Bereich von 5 bis 250 ist; x eine ganze Zahl im Bereich von 0 bis 200 ist; y eine ganze Zahl im Bereich von 0 bis 200 ist; und z eine ganze Zahl im Bereich von 1 bis 200 ist; und wobei G aus irgendeiner Struktureinheit, die wenigstens eine radikalisch polymerisierbare Kohlenstoff-Kohlenstoff-Doppelbindung enthält, ausgewählt ist.

2. Zusammensetzung gemäß Anspruch 1, wobei CA aus einem Halogen, Sulfat, Sulfonat, Methosulfat, Phosphat oder Phosphonat, Acetat, Formiat, Citrat, Maleat, Glycolat, Lactat, Fumarat und Nitrat ausgewählt ist.

3. Zusammensetzung gemäß Anspruch 1, wobei es sich bei Monomerkomponente (a) um ein oder mehr Monomere handelt, die aus 3-(Dimethyl-amino)propylmethacrylat, 2-(Dimethylamino)propan-2-yl-methacrylat, 3-(Dimethylamino)-2,2-dimethylpropylmethacrylat, 2-(Dimethylamin)-2-methylpropylmethacrylat, 4-(Dimethylamin)butylmethacrylat, Salzen von einem oder mehreren davon oder Gemischen von einem oder mehreren davon ausgewählt sind.

4. Zusammensetzung gemäß Anspruch 1, wobei ein oder mehrere Monomere gemäß Formel (I) mit Wasserstoffperoxid ($H_2O_2$) umgesetzt werden, wobei eine Aminoxid-Verbindung entsteht, die durch die folgende Formel (Ia) dargestellt wird:

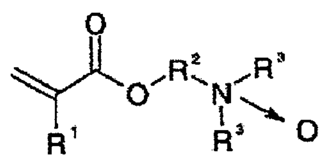 (Ia)

wobei $R^1$, $R^2$ und $R^3$ wie oben definiert sind.

5. Zusammensetzung gemäß Anspruch 1, wobei Monomerkomponente (b) aus wenigstens einem $C_1$- bis $C_{30}$-Alkyl(meth)acrylat, wenigstens einem Hydroxy-$C_2$-bis-$C_{30}$-alkyl(meth)acrylat, wenigstens einem $C_1$- bis $C_{30}$-Alkyl(meth)acrylamid, wenigstens einem Styrol, wenigstens einem substituierten Styrol, wenigstens einem Vinylester, wenigstens einem ungesättigten Nitrit, dem Reaktionsprodukt von Glycidyl-t-decanoat und Acrylsäure, dem Reaktionsprodukt von Glycidyl-t-decanoat und Methacrylsäure oder einer Kombination von zweien oder mehreren davon ausgewählt ist.

6. Polymer gemäß Anspruch 1, wobei Monomerkomponente (b) aus Methylacrylat, Ethylacrylat, Methylmethacrylat, n-Butylacrylat, 2-Ethylhexylacrylat, 3,3,5-Trimethylcyclohexylmethacrylat, Stearylmethacrylat und geeigneten Gemischen von zweien oder mehreren davon ausgewählt ist.

7. Zusammensetzung gemäß Anspruch 1, wobei Monomerkomponente (c) aus einem oder mehreren von Cetyl-polyethoxyliertem-Methacrylat, Cetearyl-polyethoxyliertem-Methacrylat, Stearyl-polyethoxyliertem-(Meth)acrylat, Arachidyl-polyethoxyliertem-(Meth)acrylat, Behenyl-poly-ethoxyliertem-Methacrylat, Lauryl-polyethoxyliertem-Methacrylat, Cerotylpolyethoxyliertem-(Meth)acrylat, Montanyl-polyethoxyliertem-(Meth)-acrylat, Melissyl-polyethoxyliertem-(Meth)acrylat, Lacceryl-polyethoxyliertem-(Meth)acrylat, Tristyryl-phenolpolyethoxyliertem-Methacrylat, hydriertem-Ricinusöl-polyethoxyliertem-Methacrylat, Canola-polyethoxyliertem-(Meth)acrylat und Cholesterin-polyethoxyliertem-Methacrylat ausgewählt ist, wobei der polyethoxylierte Teil des Monomers 5 bis 100 Ethylenoxid-Repetiereinheiten umfasst.

**8.** Zusammensetzung gemäß Anspruch 1, wobei Monomerkomponente (d) aus wenigstens einem Monomer, das eine der folgenden chemischen Formeln aufweist, ausgewählt ist:

$$H_2C{=}CH\text{-}O(CH_2)_aO(C_3H_6O)_b(C_2H_4O)_cH;$$

$$H_2C{=}CHCH_2O(C_3H_6O)_d(C_2H_4O)_eH;$$

$$H_2C{=}C(Q)\text{-}C(O)\text{-}O(CH_2)_aO(C_3H_6O)_b(C_2H_4O)_cH;\ \text{oder}$$

$$H_2C{=}C(Q)\text{-}C(O)\text{-}O(C_3H_6O)_d(C_2H_4O)_eH;$$

wobei Q Wasserstoff oder Methyl ist; a = 2, 3 oder 4 ist; b eine ganze Zahl im Bereich von 1 bis 10 ist; c eine ganze Zahl im Bereich von 5 bis 50 ist; d eine ganze Zahl im Bereich von 1 bis 10 ist; und e eine ganze Zahl im Bereich von 5 bis 50 ist.

**9.** Zusammensetzung gemäß Anspruch 1, wobei die Monoquaterniumverbindung aus Acetamidopropyltrimoniumchlorid, Behenamidopropyldimethylamin, Behenamidopropylethyldimoniumethosulfat, Behentrimoniumchlorid, Cetethylmorpholiniumethosulfat, Cetrimoniumchlorid, Kokosamidopropylethyldimoniumethosulfat, Dicetyldimoniumchlorid, Dimethiconhydroxypropyltrimoniumchlorid, Hydroxyethylbehenamidopropyldimoniumchlorid, Quaternium-26, Quaternium-27, Quaternium-53, Quaternium-63, Quaternium-70, Quaternium-72, Quaternium-76-hydrolysiertem-Collagen, PPG-9-Diethylmoniumchlorid, PPG-25-diethylmoniumchlorid, PPG-40-diethylmoniumchlorid, Stearalkoniumchlorid, Stearamidopropylethyldimoniumethosulfat, Steardimoniumhydroxypropyl-hydrolysiertem-Weizenprotein, Steardimoniumhydroxypropyl-hydrolysiertem-Collagen, Weizenkeimamidopropalkoniumchlorid, Weizenkeimamidopropylethyldimoniumethosulfat und Kombinationen davon ausgewählt ist oder wobei die Polyquaterniumverbindung aus Polyquaternium-4, Polyquaternium-7, Polyquaternium-10, Polyquaternium-11, Polyquaternium-15, Polyquaternium-16, Polyquaternium-22, Polyquaternium-24, Polyquaternium-28, Polyquaternium-29, Polyquaternium-32, Polyquaternium-33, Polyquaternium-34, Polyquaternium-35, Polyquaternium-37, Polyquaternium-39, Polyquaternium-43, Polyquaternium-44, Polyquaternium-46, Polyquaternium-47, Polyquaternium-55, Polyquaternium-60, Polyquaternium-66, Polyquaternium-67, Polyquaternium-68, Polyquaternium-69, Polyquaternium-72, Polyquaternium-77, Polyquaternium-85, Polyquaternium-86, Polyquaternium-87 und Kombinationen davon ausgewählt ist.

**10.** Zusammensetzung gemäß Anspruch 1, wobei die saure Verbindung aus einer organischen Säure, einer Mineralsäure und Gemischen davon ausgewählt ist.

**11.** Zusammensetzung gemäß Anspruch 10, wobei die saure Verbindung aus Chlorwasserstoffsäure, Salpetersäure, Schwefelsäure, Phosphorsäure, Natriumhydrogensulfat und Gemischen davon ausgewählt ist oder
wobei die saure Verbindung aus alpha-Hydroxysäuren (AHAs), beta-Hydroxysäuren (BHAs), alpha-Aminosäuren, alpha-Ketosäuren und Gemischen davon ausgewählt ist oder
wobei die saure Verbindung aus Milchsäure, Essigsäure, Fumarsäure, Glycolsäure, Äpfelsäure, Zitronensäure, Weinsäure, 2-Hydroxyoctansäure, Glycerinsäure (Dihydroxypropionsäure), Tartronsäure, Gluconsäure, Mandelsäure, Benzilsäure, alpha-Liponsäure, Argininglycolat, Ammoniumlactat, Natriumlactat, alpha-Hydroxybuttersäure, alpha-Hydroxyisobuttersäure, alpha-Hydroxyisocapronsäure, alpha-Hydroxyisovaleriansäure, Atrolactinsäure, 3-Hydroxypropansäure, beta-Hydroxybuttersäure, beta-Phenylmilchsäure, beta-Phenylbrenztraubensäure, Salicylsäure, Asparaginsäure, Glutaminsäure, Brenztraubensäure, Acetobrenztraubensäure, Retinsäure, Trichloressigsäure, Phytinsäure, Lysophosphatidinsäure, Salicylsäure, 5-Octanoylsalicylsäure ausgewählt ist.

**12.** Zusammensetzung gemäß Anspruch 1, wobei das Tensid aus einem anionischen Tensid, einem kationischen Tensid, einem amphoteren Tensid, einem nichtionischen Tensid und Kombinationen davon ausgewählt ist.

**13.** Zusammensetzung gemäß Anspruch 1, weiterhin umfassend eine Komponente, die aus einem Konditionierer, einem Feuchtigkeitsspender, Antioxidantien, Partikeln, keratolytischen Mitteln, sekundären Pflanzenstoffen, Vitaminen, Antischuppenmitteln, entzündungshemmenden Mitteln, Analgetika, Antitranspirantien, Deodorantien, haarfestigenden Polymeren, Hilfssäuerungsmitteln, Hilfsalkalisierungsmitteln, Rheologiemodifikatoren, Viskositätsmodifikatoren, Glanzverstärkern, einem Weichmacher, einem Feuchthaltemittel, einem Gleitmittel, Sonnenschutzmittel,

UV-Absorber, Oxidationsmittel, Reduktionsmittel, Polymerfilmmodifikator, Chelatisierungsmittel, Opaker, Konservierungsmittel, Duftstoff, Duftstoff-Löslichmacher, Farbstoff, Treibmittel, Puffermittel, Festiger, Bildnern und Kombinationen davon ausgewählt ist.

**14.** Zusammensetzung gemäß Anspruch 13, wobei der Konditionierer ein Silikon ist.

**15.** Zusammensetzung gemäß Anspruch 14, wobei das Silikon aus einem flüssigen Silikon, einem Silikonkautschuk, einem Silikonharz und Gemischen davon ausgewählt ist.

**16.** Zusammensetzung gemäß einem der Ansprüche 1 bis 15, wobei das Polymer ausgehend von einem Monomergemisch polymerisiert ist, welches ausgewählt ist aus (ii) einem Gemisch, das (a), (b) und (d) umfasst, wobei Monomerkomponente (a) aus wenigstens einem von 3-(Dimethyl-amino)propylmethacrylat; 2-(Dimethylamino)propan-2-yl-methacrylat; 3-(Dimethylamino)-2,2-dimethylpropylmethacrylat; 2-(Dimethylamino)-2-methylpropylmethacrylat; 4-(Dimethylamino)butylmethacrylat und Gemischen davon ausgewählt ist, Monomerkomponente (b) aus wenigstens einem von einem $C_1$- bis $C_{30}$-Alkyl(meth)acrylat, hydroxysubstituiertem $C_1$- bis $C_{30}$-Alkyl(meth)acrylat und Gemischen davon ausgewählt ist und Monomerkomponente (d) aus wenigstens einem Monomer, das durch die Formel

$$H_2C{=}CHCH_2O(C_3H_6O)_d(C_2H_4O)_eH$$

dargestellt wird, und Gemischen davon ausgewählt ist, wobei d im Bereich von 1 bis 20 liegt und e im Bereich von 5 bis 40 liegt.

**17.** Zusammensetzung gemäß Anspruch 16, wobei das Polymer ausgehend von einem Monomergemisch polymerisiert ist, welches 3-(Dimethylamino)-2,2-dimethylpropylmethacrylat, Ethylacrylat, Methylacrylat und ein Monomer, das durch die Formel $H_2C{=}CHCH_2O(C_3H_6O)_{2\text{-}10}(C_2H_4O)_{10\text{-}25}H$ dargestellt wird, umfasst.

**18.** Zusammensetzung gemäß einem der Ansprüche 1 bis 17, wobei das Polymer ausgehend von einem Monomergemisch polymerisiert ist, welches ausgewählt ist aus (ii) einem Gemisch, das (a), (b) und (c) umfasst, wobei Monomerkomponente (a) aus wenigstens einem von 3-(Dimethylamino)propylmethacrylat; 2-(Dimethylamino)propan-2-yl-methacrylat; 3-(Dimethylamino)-2,2-dimethylpropylmethacrylat; 2-(Dimethylamino)-2-methylpropylmethacrylat; 4-(Dimethylamino)butylmethacrylat und Gemischen davon ausgewählt ist, Monomerkomponente (b) aus wenigstens einem von einem $C_1$- bis $C_{30}$-Alkyl(meth)acrylat und Gemischen davon ausgewählt ist und Monomerkomponente (c) aus wenigstens einem Monomer ausgewählt ist, das aus einem oder mehreren von Cetyl-polyethoxyliertem-Methacrylat, Cetearyl-polyethoxyliertem-Methacrylat, Stearyl-polyethoxyliertem-(Meth)acrylat, Arachidyl-polyethoxyliertem-(Meth)-acrylat, Behenyl-polyethoxyliertem-Methacrylat, Lauryl-polyethoxyliertem-Methacrylat, Cerotyl-polyethoxyliertem-(Meth)acrylat, Montanyl-polyethoxyliertem-(Meth)acrylat, Melissyl-polyethoxyliertem-(Meth)acrylat, Lacceryl-polyethoxyliertem-(Meth)acrylat, Tristyryl-phenolpolyethoxyliertem-Methacrylat, hydriertem-Ricinusöl-polyethoxyliertem-Methacrylat, Canola-polyethoxyliertem-(Meth)acrylat und Cholesterin-polyethoxyliertem-Methacrylat ausgewählt ist, wobei der polyethoxylierte Teil des Monomers 5 bis 100 Ethylenoxid-Repetiereinheiten umfasst.

**19.** Körperpflegeprodukt, Gesundheitspflegeprodukt, Haushaltspflegeprodukt, Anstaltspflegeprodukt und industrielles Pflegeprodukt oder industrielles Anwendungsprodukt, das die Zusammensetzung gemäß einem der Ansprüche 1 bis 18 umfasst.

**Revendications**

**1.** Composition comprenant :

A) un polymère stable à l’hydrolyse polymérisé à partir d’un mélange de monomères choisi parmi :

(i) un mélange comprenant (a), (b) et (c) ;
(ii) un mélange comprenant (a), (b) et (d) ;
(iii) un mélange comprenant (a), (b) et (e) ;
(iv) un mélange comprenant (a), (b), (c) et (d) ;
(v) un mélange comprenant (a), (b), (c) et (e) ;

(vi) un mélange comprenant (a), (b), (d) et (e) ; ou
(vii) un mélange comprenant (a), (b), (c), (d) et (e)

où le composant de monomère (a) est au moins un monomère de méth(acrylate) substitué par un groupe amino (ASMA) ou un sel de celui-ci ; le composant de monomère (b) est au moins un monomère de vinyle non ionique (NIV) ; le composant de monomère (c) est au moins un monomère associatif de type vinyle (VA) ; le composant de monomère (d) est au moins un monomère de type tensioactif vinylique (VS) ; le composant de monomère (e) est au moins un macromère de silicone polymérisable (PSM), et dans laquelle l'un quelconque des mélanges indiqués ci-dessus contient en outre optionnellement : (f) au moins un monomère de réticulation (XL) ; (g) au moins un agent de transfert de chaîne (CTA) ; (h) au moins un stabilisant polymère ; ou toute combinaison adéquate de deux ou plusieurs des composants (f), (g) et/ou (h) ; et
B) un composant choisi parmi un tensioactif, un composé monoquatemium, un composé polyquaternium, un composé acide et leurs combinaisons,

dans laquelle le composant de monomère (a) est choisi parmi au moins un composé ou un sel de celui-ci, représenté par les Formules (I) et (II) :

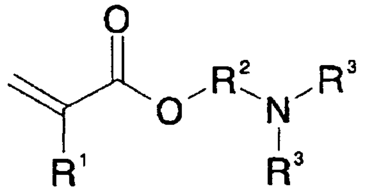
(I)

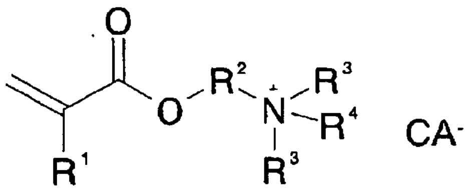
(II)

où $R^1$ représente un groupe méthyle ; où $R^2$ représente un groupe alcanediyle en $C_2$ à $C_8$ linéaire ou ramifié, substitué ou non substitué, à condition que lorsque $R^2$ comporte deux atomes de carbone au moins l'un des deux atomes de carbone du groupe $R^2$ soit substitué par un groupe alkyle en $C_1$ à $C_{30}$ linéaire ou ramifié ; où chaque $R^3$ est indépendamment choisi parmi l'atome d'hydrogène, les groupes alkyles en $C_1$ à $C_{30}$ linéaires ou ramifiés, les groupes alkyles en $C_1$ à $C_{30}$ linéaires ou ramifiés qui contiennent un ou plusieurs hétéroatomes, les groupes alcényles en $C_2$ à $C_{30}$ linéaires ou ramifiés, les groupes alcényles en $C_2$ à $C_{30}$ linéaires ou ramifiés qui contiennent un ou plusieurs hétéroatomes, les groupes alcynyles en $C_2$ à $C_{30}$ linéaires ou ramifiés, les groupes alcynyles en $C_2$ à $C_{30}$ linéaires ou ramifiés qui contiennent un ou plusieurs hétéroatomes ; les groupes aryles en $C_4$ à $C_{20}$, les groupes aryles en $C_4$ à $C_{20}$ qui contiennent un ou plusieurs hétéroatomes, les groupes cycloalkyles en $C_4$ à $C_{20}$, les groupes cycloalkyles en $C_4$ à $C_{20}$ qui contiennent un ou plusieurs hétéroatomes, les groupes hétérocycliques en $C_4$ à $C_{20}$, ou où les deux substituants $R^3$ et l'atome d'azote auquel ils sont liés peuvent former un groupe hétérocyclique en $C_2$ à $C_{20}$, saturé ou insaturé, ou un groupe hétérocyclique en $C_2$ à $C_{20}$, saturé ou insaturé, comportant deux ou plusieurs hétéroatomes, où les hétéroatomes, le cas échéant, sont choisis parmi un groupe carbonyle ou un atome de N, de S, de P ou d'O ; et où $R^4$ représente un groupe alkyle en $C_1$ à $C_{30}$, linéaire ou ramifié ; et où $CA^-$ représente un contre-anion adéquat pour équilibrer la charge sur le groupement ammonium quaternaire ;
dans laquelle le composant de monomère (b) est choisi parmi au moins un monomère éthyléniquement insaturé, copolymérisable, non ionique, représenté par les Formules (III) et (IV) :

$$C(X)_2=C(X)Z \qquad (III)$$

$$CH_2=CH\text{-}OC(O)R_1 \qquad (IV)$$

dans laquelle, dans chacune des Formules (III) et (IV), chaque X représente indépendamment un atome d'hydrogène, un groupe méthyle, $-CH_2C(O)OR_1$, $-C(O)OR_1$ ; et Z représente un groupe $-C(O)OR_1$, $-C_6H_4R_1$, $-C_6H_4OR_1$, -CN, $-C(O)N(R_1)_2$, $-NHC(O)CH_3$, -NHC(O)H, $-C(O)OA'OR_{15}$ N-(2-pyrrolidonyle), N-caprolactamyle,

-C(O)NHCH$_2$CH$_2$-N-éthylèneurée ou -C(O)NHC(CH$_3$)$_3$ ; A' représente un radical divalent choisi parmi les groupes -CH$_2$CH(OH)CH$_2$- et -CH$_2$CH(CH$_2$OH)-, chaque R$_1$ représente indépendamment un groupe alkyle en C$_1$ à C$_{30}$ linéaire et ramifié, alkyle en C$_2$ à C$_{30}$ substitué par un groupe hydroxy, cycloalkyle en C$_5$ à C$_{30}$ et cycloalkyle en C$_5$ à C$_{30}$ substitué par un groupe alkyle en C$_1$ à C$_5$ ; R$_{15}$ représente un résidu acyle d'un acide gras en C$_6$ à C$_{22}$ saturé ou insaturé, linéaire ou ramifié ;

dans laquelle le composant de monomère (c) est choisi parmi au moins un monomère représenté par la Formule (V) :

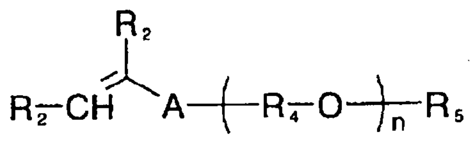

(V)

dans laquelle, chaque R$_2$ représente indépendamment l'atome d'H ou un groupe méthyle, -C(O)OH ou -C(O)OR$_3$ ; R$_3$ représente un groupe alkyle en C$_1$ à C$_{30}$; A représente un groupe -CH$_2$C(O)O-, -C(O)O-, -O- ou -CH$_2$O- ; (R$_4$-O)$_n$ représente un polyoxyalkylène, qui est un homopolymère, un copolymère aléatoire ou un copolymère séquencé d'unités oxyalkylène en C$_2$ à C$_4$, dans laquelle chaque R$_4$ représente indépendamment un groupe C$_2$H$_4$, C$_3$H$_6$, C$_4$H$_8$, ou un mélange de ceux-ci, et n représente un nombre entier dans la plage d'environ 5 à environ 250 ; et dans laquelle R$_5$ représente un groupe alkyle substitué ou non substitué choisi parmi les groupes alkyles en C$_8$ à C$_{40}$, linéaires ou ramifiés, les groupes alkyles carbocycliques en C$_8$ à C$_{40}$, les groupes phényles substitués par un groupe alkyle en C$_2$ à C$_{40}$, les groupes alkyles en C$_2$ à C$_{40}$ substitués par un groupe aryle, et les esters complexes en C$_8$ à C$_{80}$, dans laquelle le groupe alkyle R$_5$ comprend optionnellement un ou plusieurs substituants choisis parmi un groupe hydroxyle, un groupe alcoxyle ou un groupe halogéno ;

dans laquelle le composant de monomère (d) est choisi parmi au moins un monomère représenté par la Formule (VI) :

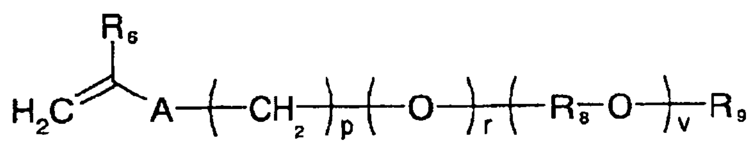

(VI)

où chaque R$_6$ représente indépendamment l'atome d'hydrogène ou un groupe méthyle, -C(O)OH ou -C(O)OR$_7$ ; R$_7$ représente un groupe alkyle en C$_1$ à C$_{30}$ ; A représente un groupe -CH$_2$C(O)O-, -C(O)O-, -O- ou -CH$_2$O- ; p représente un nombre entier dans la plage de 0 à 30, et r vaut 0 ou 1, à condition que lorsque p vaut 0, r vaille 0, et que lorsque p se situe dans la plage de 1 à 30, r vaille 1 ; (R$_8$-O)$_v$ représente un polyoxyalkylène, qui est un homopolymère, un copolymère aléatoire ou un copolymère séquencé d'unités oxyalkylène en C$_2$ à C$_4$, dans laquelle chaque R$_8$ représente indépendamment un groupe C$_2$H$_4$, C$_3$H$_6$, C$_4$H$_8$, ou un mélange de ceux-ci, et v représente un nombre entier dans la plage de 5 à 250 ; et R$_9$ représente l'atome d'hydrogène ou un groupe alkyle en C$_1$ à C$_4$ ; et dans laquelle le composant de monomère (e) est choisi parmi au moins un macromère de silicone polymérisable représenté par la Formule suivante :

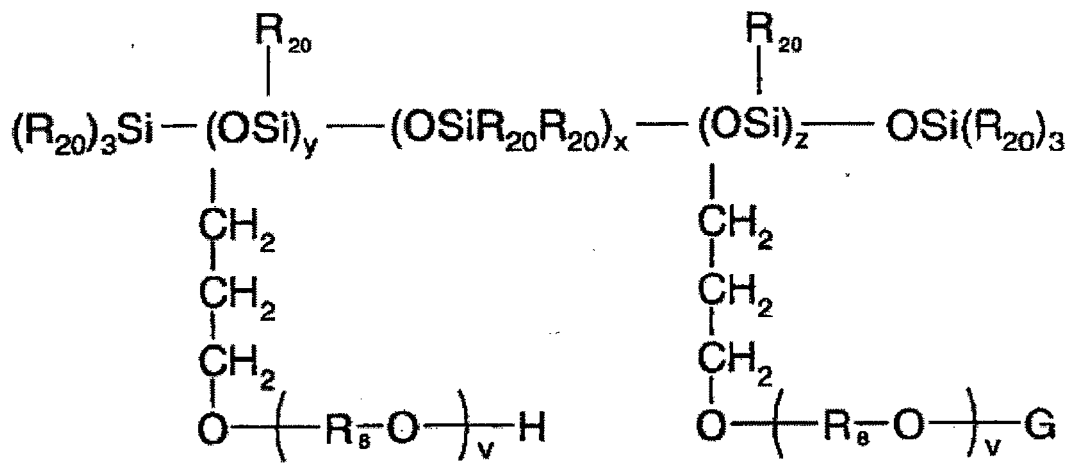

où chaque $R_{20}$ est indépendamment choisi parmi un groupe alkyle en $C_1$ à $C_{30}$ linéaire ou ramifié, un groupe aryle en $C_4$ à $C_{20}$ ou un groupe alcényle en $C_2$ à $C_{20}$ ; où $(R_8\text{-}O)_v$ représente un groupement polyoxyalkylène, qui peut se présenter sous la forme d'un homopolymère, d'un copolymère aléatoire ou d'un copolymère séquencé d'unités oxyalkylène en $C_2$ à $C_4$, dans laquelle chaque $R_8$ représente indépendamment un groupe $C_2H_4$, $C_3H_6$, $C_4H_8$, ou un mélange de ceux-ci, et v représente un nombre entier dans la plage de 5 à 250 ; x représente un nombre entier dans la plage de 0 à 200 ; y représente un nombre entier dans la plage de 0 à 200 ; et z représente un nombre entier dans la plage de 1 à 200 ; et où G est choisi parmi tout groupement qui contient au moins une double liaison carbone-carbone polymérisable par la voie des radicaux libres.

2. Composition selon la revendication 1, dans laquelle CA est choisi parmi un atome d'halogène, un sulfate, un sulfonate, un méthosulfate, un phosphate ou un phosphonate, un acétate, un formate, un citrate, un maléate, un glycolate, un lactate, un fumarate et un nitrate.

3. Composition selon la revendication 1, dans laquelle le composant de monomère (a) est un ou plusieurs monomères choisis parmi le méthacrylate de 3-(diméthylamino)propyle, le méthacrylate de 2-(diméthylamino)propan-2-yle, le méthacrylate de 3-(diméthylamino)-2,2-diméthylpropyle, le méthacrylate de 2-(diméthylamine)-2-méthylpropyle, le méthacrylate de 4-(diméthylamine)butyle, les sels d'un ou plusieurs de ceux-ci, ou les mélanges d'un ou plusieurs de ceux-ci.

4. Composition selon la revendication 1, dans laquelle un ou plusieurs monomères répondant à la Formule (I) sont mis à réagir avec du peroxyde d'hydrogène ($H_2O_2$) pour donner un composé oxyde d'amine représenté par la Formule suivante (Ia) :

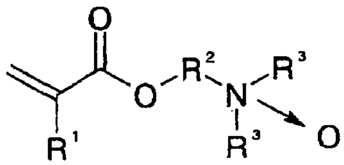

(Ia)

où $R^1$, $R^2$ et $R^3$ sont tels que définis ci-dessus.

5. Composition selon la revendication 1, dans laquelle le composant de monomère (b) est choisi parmi au moins un (méth)acrylate d'alkyle en $C_1$ à $C_{30}$, au moins un (méth)acrylate d' hydroxy-alkyle en $C_2$ à $C_{30}$, au moins un (méth)acrylamide d'alkyle en $C_1$ à $C_{30}$, au moins un styrène ; au moins un styrène substitué, au moins un ester vinylique, au moins un nitrite insaturé, le produit réactionnel de t-décanoate de glycidyle et d'acide acrylique, le produit réactionnel de t-décanoate de glycidyle et d'acide méthacrylique, ou toute combinaison de deux ou plusieurs de ceux-ci.

6. Polymère selon la revendication 1, dans lequel le composant de monomère (b) est choisi parmi l'acrylate de méthyle, l'acrylate d'éthyle, le méthacrylate de méthyle, l'acrylate de n-butyle, l'acrylate de 2-éthylhexyle, le méthacrylate de 3,3,5-triméthylcyclohexyle, le méthacrylate de stéaryle, et les mélanges adéquats de deux ou plusieurs quelconques de ceux-ci.

7. Composition selon la revendication 1, dans laquelle le composant de monomère (c) est choisi parmi un ou plusieurs du méthacrylate polyéthoxylé de cétyle, du méthacrylate polyéthoxylé de cétéaryle, du (méth)acrylate polyéthoxylé de stéaryle, du (méth)acrylate polyéthoxylé d'arachidyle, du méthacrylate polyéthoxylé de béhényle, du méthacrylate polyéthoxylé de lauryle, du (méth)acrylate polyéthoxylé de cérotyle, du (méth)acrylate polyéthoxylé de montanyle, du (méth)acrylate polyéthoxylé de mélissyle, du (méth)acrylate polyéthoxylé de laccéryle, du méthacrylate phénolpolyéthoxylé de tristyryle, du méthacrylate polyéthoxylé d'huile de ricin hydrogénée, du (méth)acrylate polyéthoxylé de canola et du méthacrylate polyéthoxylé de cholestérol, où la partie polyéthoxylée du monomère comprend 5 à 100 unités répétitives d'oxyde d'éthylène.

8. Composition selon la revendication 1, dans laquelle le composant de monomère (d) est choisi parmi au moins un monomère ayant l'une des Formules chimiques suivantes :

$$H_2C{=}CH\text{-}O(CH_2)_aO(C_3H_6O)_b(C_2H_4O)_cH ;$$

$H_2C{=}CHCH_2O(C_3H_6O)_d(C_2H_4O)_eH$ ;

$H_2C{=}C(Q)\text{-}C(O)\text{-}O(CH_2)_aO(C_3H_6O)_b(C_2H_4O)_cH$ ; ou

$H_2C{=}C(Q)\text{-}C(O)\text{-}O(C_3H_6O)_d(C_2H_4O)_eH$

formules dans lesquelles Q représente l'atome d'hydrogène ou un groupe méthyle ; a vaut 2, 3 ou 4 ; b représente un nombre entier dans la plage de 1 à 10 ; c représente un nombre entier dans la plage de 5 à 50 ; d représente un nombre entier dans la plage de 1 à 10 ; et e représente un nombre entier dans la plage de 5 à 50.

**9.** Composition selon la revendication 1, dans laquelle ledit composé monoquaternium est choisi parmi le chlorure d'acétamidopropyltrimonium, la béhénamidopropyldiméthylamine, l'éthosulfate de béhénamidopropyléthyldimonium, le chlorure de béhéntrimonium, l'éthosulfate de cététhylmorpholinium, le chlorure de cétrimonium, l'éthosulfate de cocoamidopropyléthyldimonium, le chlorure de dicétyldimonium, le chlorure de diméthicone-hydroxypropyltrimonium, le chlorure d'hydroxyéthylbéhénamidopropyldimonium, le quaternium-26, le quaternium-27, le quaternium-53, le quaternium-63, le quaternium-70, le quaternium-72, le collagène hydrolysé de quaternium-76, le chlorure de stéaralkonium, l'éthosulfate de stéaramidopropyléthyldimonium, la protéine de blé hydrolysée d'hydroxypropyle de stéardimonium, le collagène hydrolysé d'hydroxypropyle de stéardimonium, le chlorure de germamidopropalkonium de blé, l'éthosulfate de germamidopropyléthyldimonium de blé et leurs combinaisons, ou
dans laquelle ledit composé polyquaternium est choisi parmi le polyquaternium-4, le polyquaternium-7, le polyquaternium-10, le polyquaternium-11, le polyquaternium-15, le polyquaternium-16, le polyquaternium-22, le polyquaternium-24, le polyquaternium-28, le polyquatemium-29, le polyquaternium-32, le polyquatemium-33, le polyquaternium-34, le polyquaternium-35, le polyquaternium-37, le polyquaternium-39, le polyquaternium-43, le polyquatemium-44, le polyquaternium-46, le polyquatemium-47, le polyquaternium-55, le polyquaternium-60, le polyquaternium-66, le polyquaternium-67, le polyquaternium-68, le polyquatemium-69, le polyquatemium-72, le polyquaternium-77, le polyquaternium-85, le polyquaternium-86, le polyquaternium-87 et leurs combinaisons.

**10.** Composition selon la revendication 1, dans laquelle ledit composé acide est choisi parmi un acide organique, un acide minéral et les mélanges de ceux-ci.

**11.** Composition selon la revendication 10, dans laquelle ledit composé acide est choisi parmi l'acide chlorhydrique, l'acide nitrique, l'acide sulfurique, l'acide phosphorique, le bisulfate de sodium et les mélanges de ceux-ci, ou
dans laquelle ledit composé acide est choisi parmi les alpha-hydroxy acides (AHA), les bêta-hydroxy acides (BHA), les alpha amino-acides, les alpha-céto acides, et les mélanges de ceux-ci, ou
dans laquelle ledit composé acide est choisi parmi l'acide lactique, l'acide acétique, l'acide fumarique, l'acide glycolique, l'acide malique, l'acide citrique, l'acide tartrique, l'acide 2-hydroxyoctanoïque, l'acide glycérique (acide dihydroxypropionique), l'acide tartronique, l'acide gluconique, l'acide mandélique, l'acide benzilique, l'acide alpha-lipoïque, le glycolate d'arginine, le lactate d'ammonium, le lactate de sodium, l'acide alpha-hydroxybutyrique, l'acide alpha-hydroxyisobutyrique, l'acide alpha-hydroxyisocaproïque, l'acide alpha-hydroxyisovalérique, l'acide atrolactique, l'acide 3-hydroxypropanoïque, l'acide bêta-hydroxybutyrique, l'acide bêta-phényllactique, l'acide bêta-phénylpyruvique, l'acide salicylique, l'acide aspartique, l'acide glutamique, l'acide pyruvique, l'acide acétopyruvique, l'acide rétinoïque, l'acide trichloroacétique, l'acide phytique, l'acide lysophosphatidique, l'acide salicylique, l'acide 5-octanoylsalicylique.

**12.** Composition selon la revendication 1, dans laquelle ledit tensioactif est choisi parmi un tensioactif anionique, un tensioactif cationique, un tensioactif amphotère, un tensioactif non ionique et leurs combinaisons.

**13.** Composition selon la revendication 1, comprenant en outre un composant choisi parmi un conditionneur, un hydratant, les antioxydants, les particules, les agents kératolytiques, les produits d'origine végétale, les vitamines, les agents antipelliculaires, les agents anti-inflammatoires, les analgésiques, les agents anti-transpirants, les composés déodorants, les polymères de fixation capillaire, les agents acidifiants auxiliaires, les agents alcalinisants auxiliaires, les modificateurs de rhéologie, les agents de régulation de la viscosité, les agents augmentant le brillant, un émollient, un humectant, un lubrifiant, un agent de protection solaire, un agent absorbant les UV ; un agent oxydant, un agent réducteur, un agent modifiant les films polymères, un agent chélatant, un opacifiant, un conservateur ; un parfum ; un solubilisant de fragrance ; un colorant, un propulseur, un agent de tamponnage ; un fixateur, des agents de mise en forme ; et leurs combinaisons.

**14.** Composition selon la revendication 13, dans laquelle ledit conditionneur est une silicone.

**15.** Composition selon la revendication 14, dans laquelle ladite silicone est choisie parmi un fluide de silicone, une gomme de silicone, une résine de silicone et les mélanges de ceux-ci.

**16.** Composition selon l'une quelconque des revendications 1 à 15, dans laquelle ledit polymère est polymérisé à partir d'un mélange de monomères choisi parmi (ii) comprenant (a), (b) et (d), dans laquelle le composant de monomère (a) est choisi parmi au moins l'un du méthacrylate de 3-(diméthylamino)propyle ; du méthacrylate de 2-(diméthylamino)propan-2-yle ; du méthacrylate de 3-(diméthylamino)-2,2-diméthylpropyle ; du méthacrylate de 2-(diméthylamino)-2-méthylpropyle ; du méthacrylate de 4-(diméthylamino)butyle, et les mélanges de ceux-ci ; le composant de monomère (b) est choisi parmi au moins l'un d'un (méth)acrylate d'alkyle en $C_1$ à $C_{30}$, d'un (méth)acrylate d'alkyle en $C_1$ à $C_{30}$ substitué par un groupe hydroxyle, et les mélanges de ceux-ci ; et le composant de monomère (d) est choisi parmi au moins un monomère représenté par la formule :

$$H_2C=CHCH_2O(C_3H_6O)_d(C_2H_4)_eH$$

et les mélanges de ceux-ci, dans laquelle d est situé dans la plage de 1 à 20, et e est situé dans la plage de 5 à 40.

**17.** Composition selon la revendication 16, dans laquelle ledit polymère est polymérisé à partir d'un mélange de monomères comprenant le méthacrylate de 3-(diméthylamino)-2,2-diméthylpropyle, l'acrylate d'éthyle, l'acrylate de méthyle, et un monomère représenté par la formule $H_2C=CHCH_2O(C_3H_6O)_{2-10}(C_2H_4O)_{10-25}H$.

**18.** Composition selon l'une quelconque des revendications 1 à 17, dans laquelle ledit polymère est polymérisé à partir d'un mélange de monomères choisi parmi (ii) comprenant (a), (b) et (c), dans laquelle le composant de monomère (a) est choisi parmi au moins l'un du méthacrylate de 3-(diméthylamino)propyle ; du méthacrylate de 2-(diméthylamino)propan-2-yle ; du méthacrylate de 3-(diméthylamino)-2,2-diméthylpropyle ; du méthacrylate de 2-(diméthylamino)-2-méthylpropyle ; du méthacrylate de 4-(diméthylamino)butyle, et les mélanges de ceux-ci ; le composant de monomère (b) est choisi parmi au moins l'un d'un (méth)acrylate d'alkyle en $C_1$ à $C_{30}$, et les mélanges de ceux-ci ; et le composant de monomère (c) est choisi parmi au moins un monomère choisi parmi un ou plusieurs du méthacrylate polyéthoxylé de cétyle, du méthacrylate polyéthoxylé de cétéaryle, du (méth)acrylate polyéthoxylé de stéaryle, du (méth)acrylate polyéthoxylé d'arachidyle, du méthacrylate polyéthoxylé de béhényle, du méthacrylate polyéthoxylé de lauryle, du (méth)acrylate polyéthoxylé de cérotyle, du (méth)acrylate polyéthoxylé de montanyle, du (méth)acrylate polyéthoxylé de mélissyle, du (méth)acrylate polyéthoxylé de laccéryle, du méthacrylate phénolpolyéthoxylé de tristyryle, du méthacrylate polyéthoxylé d'huile de ricin hydrogénée, du (méth)acrylate polyéthoxylé de canola et du méthacrylate polyéthoxylé de cholestérol, où la partie polyéthoxylée du monomère comprend d'environ 5 à environ 100 unités répétitives d'oxyde d'éthylène.

**19.** Produit d'hygiène personnelle, produit de soins de santé, produit d'entretien ménager, produit d'entretien institutionnel et industriel ou produit d'application industrielle comprenant la composition selon l'une quelconque des revendications 1 à 18.

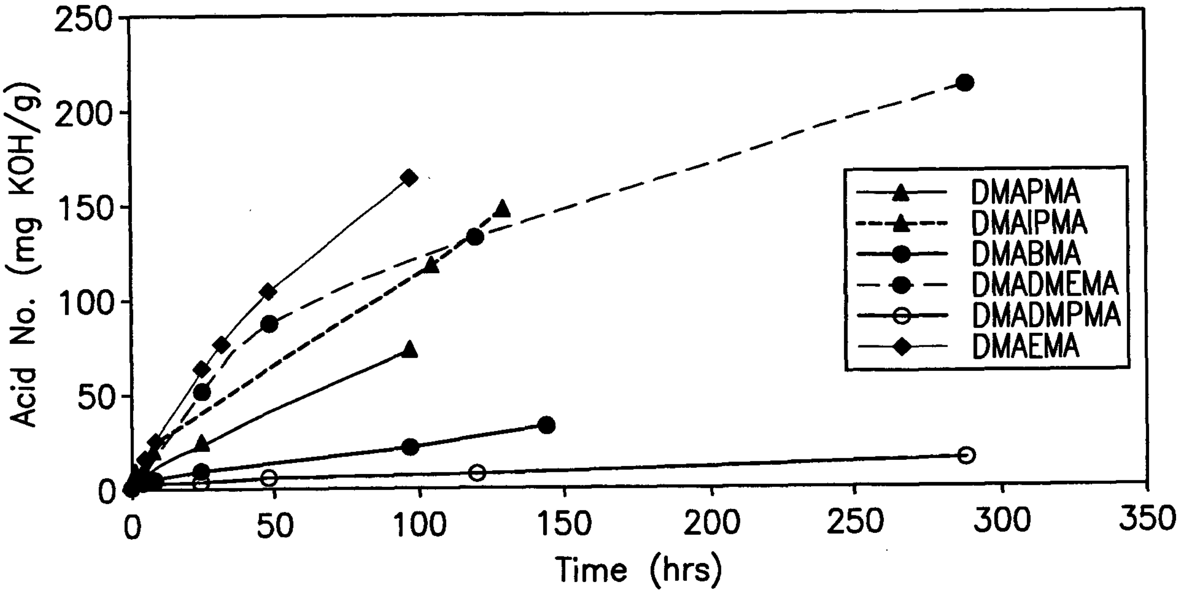
250
200
150
100
50
0
Acid No. (mg KOH/g)
0
50
100
150
200
250
300
350
Time (hrs)
DMAPMA
DMAIPMA
DMABMA
DMADMEMA
DMADMPMA
DMAEMA

FIG. 1

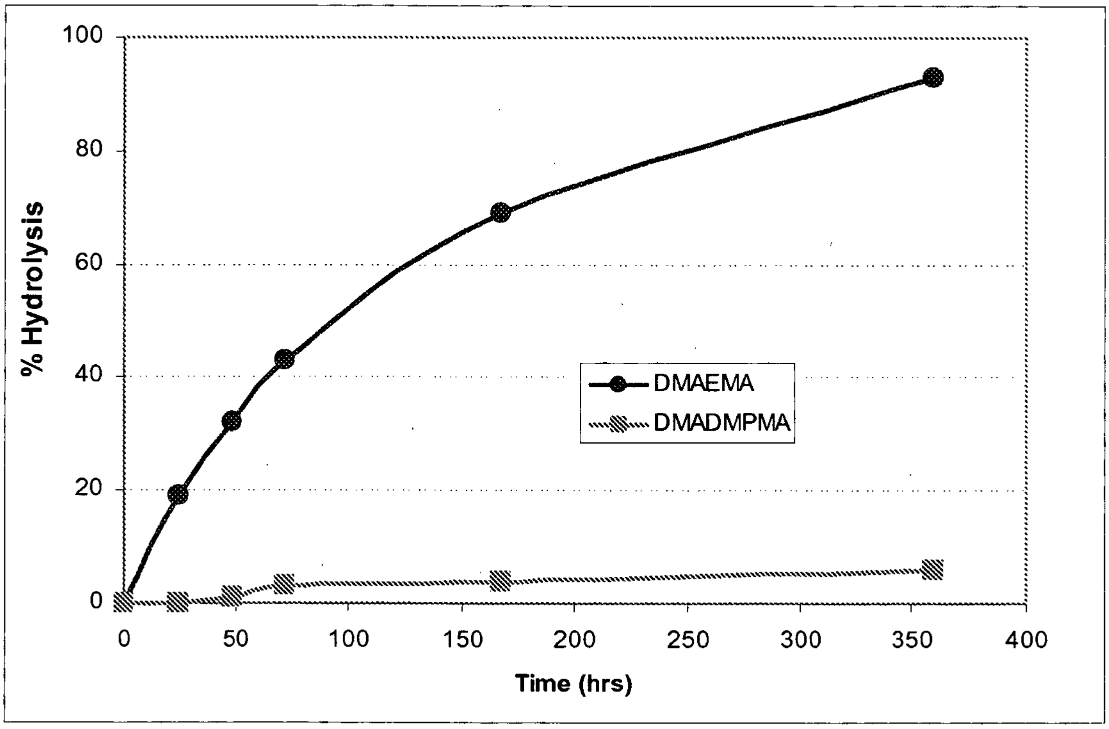
100
80
60
40
20
0
% Hydrolysis
0
50
100
150
200
250
300
350
400
Time (hrs)
DMAEMA
DMADMPMA

FIG. 2

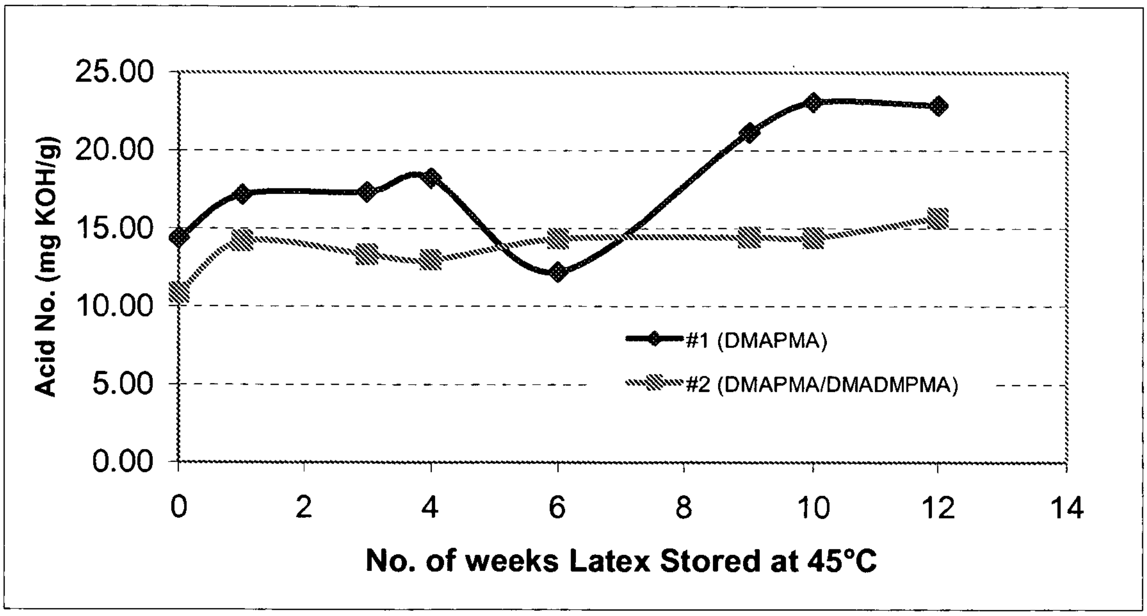

25.00
20.00
15.00
10.00
5.00
0.00
Acid No. (mg KOH/g)
0
2
4
6
8
10
12
14
No. of weeks Latex Stored at 45°C
#1 (DMAPMA)
#2 (DMAPMA/DMADMPMA)


FIG. 3

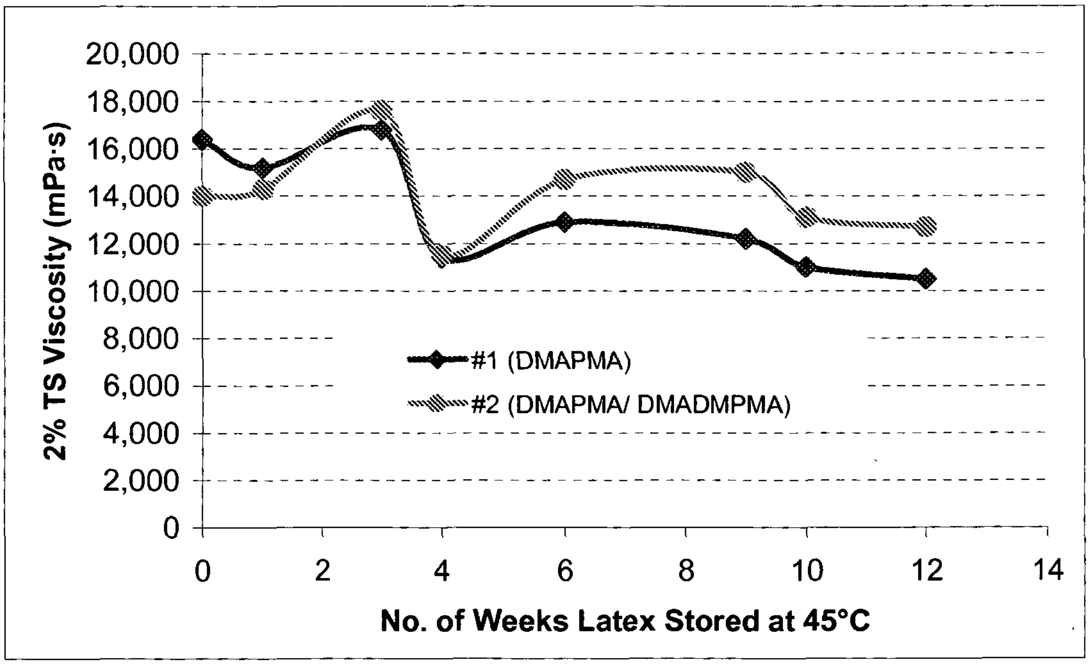
2% TS Viscosity (mPa·s)
20,000
18,000
16,000
14,000
12,000
10,000
8,000
6,000
4,000
2,000
0
0
2
4
6
8
10
12
14
No. of Weeks Latex Stored at 45°C
#1 (DMAPMA)
#2 (DMAPMA/ DMADMPMA)

FIG. 4

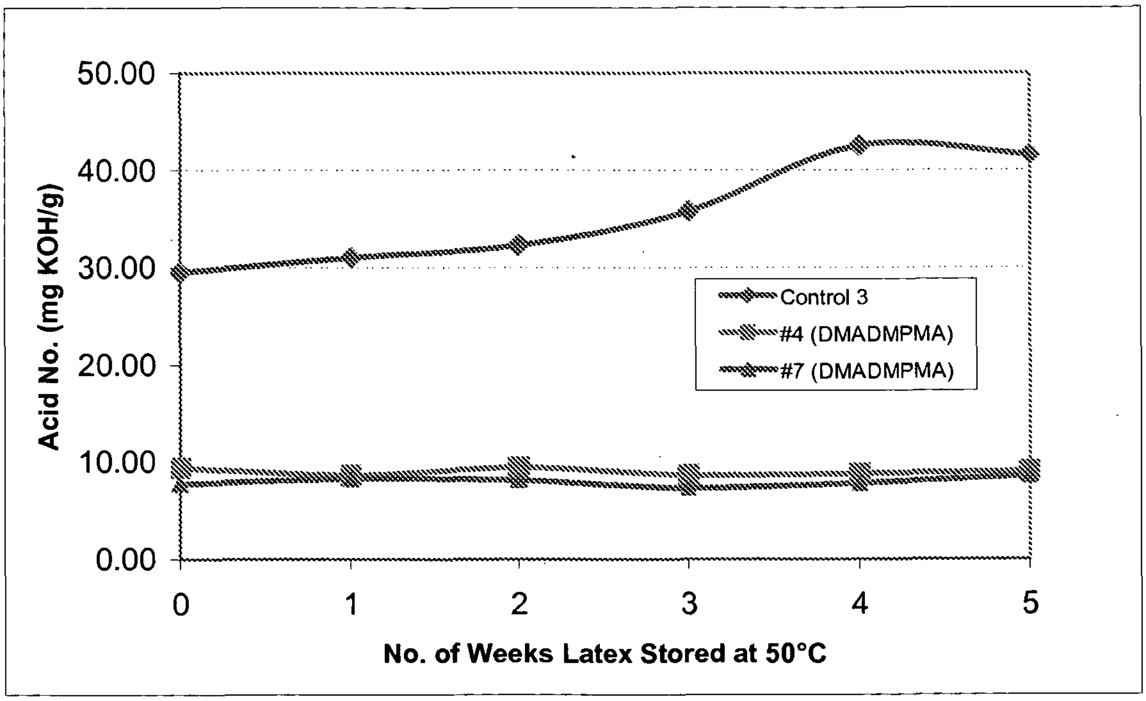
50.00
40.00
30.00
20.00
10.00
0.00
Acid No. (mg KOH/g)
0
1
2
3
4
5
No. of Weeks Latex Stored at 50°C
Control 3
#4 (DMADMPMA)
#7 (DMADMPMA)

FIG. 5

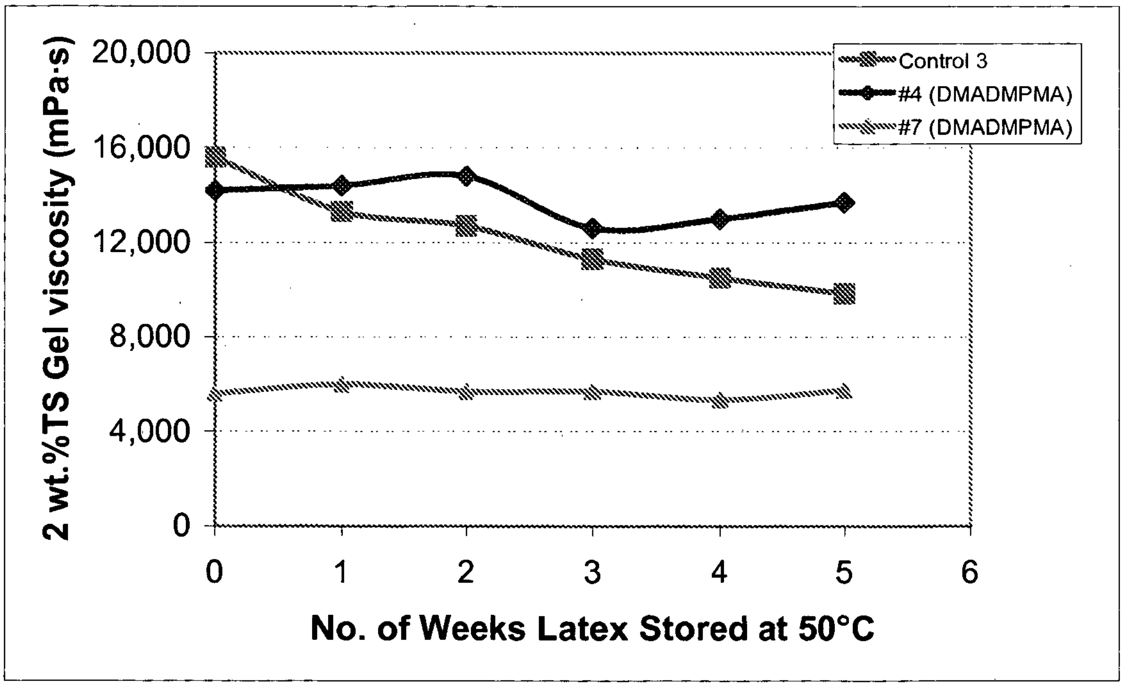
2 wt.%TS Gel viscosity (mPa·s)
20,000
16,000
12,000
8,000
4,000
0
0
1
2
3
4
5
6
No. of Weeks Latex Stored at 50°C
Control 3
#4 (DMADMPMA)
#7 (DMADMPMA)

FIG. 6

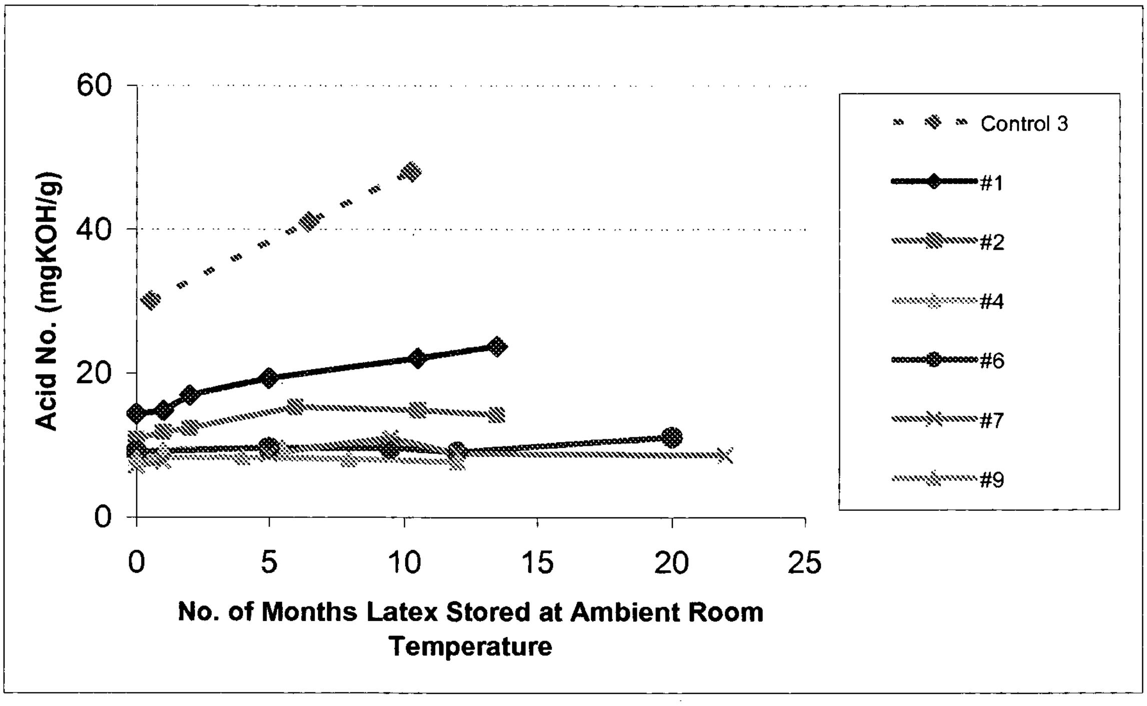

60
40
20
0
Acid No. (mgKOH/g)
0
5
10
15
20
25
No. of Months Latex Stored at Ambient Room Temperature
Control 3
#1
#2
#4
#6
#7
#9


FIG. 7

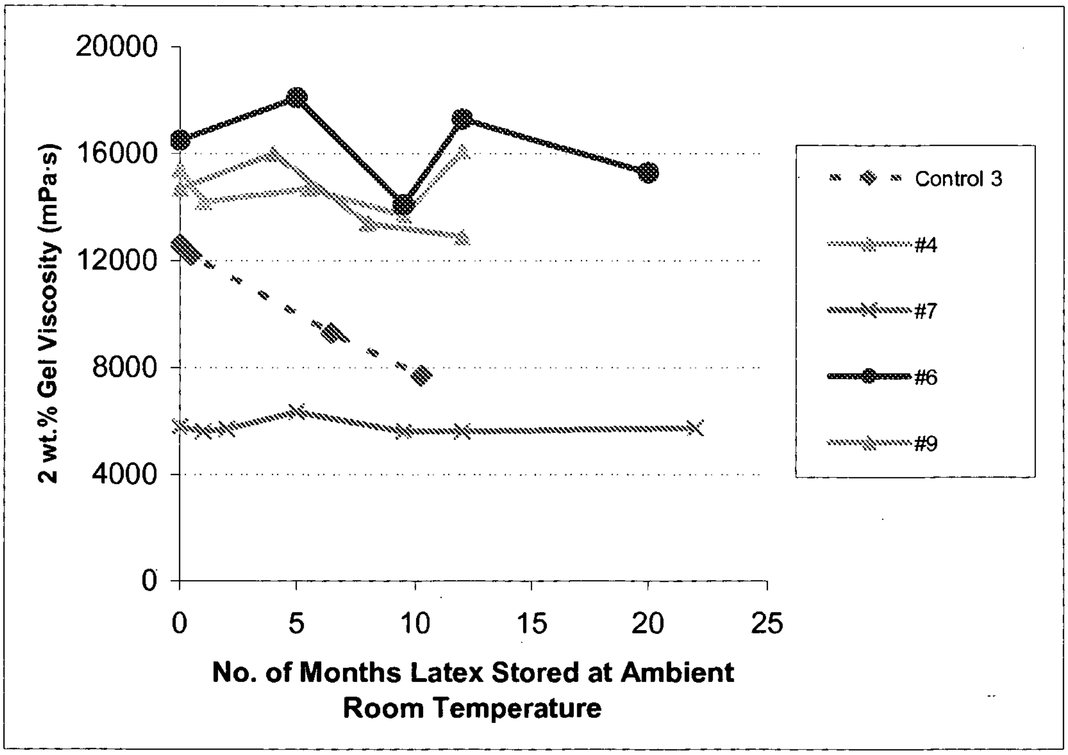
20000
16000
12000
8000
4000
0
2 wt.% Gel Viscosity (mPa·s)
0
5
10
15
20
25
No. of Months Latex Stored at Ambient Room Temperature
Control 3
#4
#7
#6
#9

FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5639841 A
- US 5179157 A
- US 33156 A
- US 5294692 A
- US 5011978 A
- US 4600761 A
- US 4421902 A
- US 4384096 A
- US 4514552 A
- US 4616074 A
- US 5292843 A
- US 5770760 A
- US 5412142 A
- WO 2007101048 A
- US 6140435 A
- US 20090029928 A
- US 20090232873 A
- US 20060079417 A
- US 20060079418 A
- US 20060079419 A
- US 20060079420 A
- US 20060079421 A
- US 20060079422 A
- US 20070009463 A
- US 20070072781 A
- US 20070280976 A
- US 20080317698 A
- US 6197317 B
- US 7205271 B
- US 4902499 A
- US 4152416 A
- US 5136063 A
- US 5180843 A
- EP 0486135 A
- WO 9311103 A
- US 6573375 B
- US 6727357 B
- US 61231780 B
- US 20040241130 A


### Non-patent literature cited in the description


- **WHALLEY.** Fabric Conditioning Agents. *HAPPI,* February 1995, 55-58
- Cosmetics Science and Technology. 1957
- The Chemistry and Manufacture of Cosmetics. 1975
- Harry's Cosmeticology. Chemical Publishing, Co., Inc, 2000
- Remington's Pharmaceutical Sciences. Mack Publishing Company, 1970
- Encyclopedia of Polymer Science and Engineering. John Wiley & Sons, Inc, 1989, vol. 15, 204-308
- **NOLL ; WALTER.** Chemistry and Technology of Silicones. Academic Press, 1968
- **TODD ; BYERS.** *Volatile Silicone Fluids for Cosmetics, Cosmetics and Toiletries,* 1976, vol. 91 (1), 27-32
- **KASPRZAK.** *Volatile Silicones, Soap/Cosmetics/Chemical Specialties,* December 1986, 40-43
- **TODD ; BYERS.** Volatile Silicone Fluids for Cosmetic Formulations. *Cosmetics and Toiletries,* January 1976, vol. 91, 29-32
- **KASPRZAK.** Volatile Silicones. *Soap/Cosmetics/Chemical Specialties,* December 1986, 40-43